(19) Europäisches Patentamt
European Patent Office
Office européen des brevets

(11) EP 1 400 245 B1

(12) **EUROPEAN PATENT SPECIFICATION**

(45) Date of publication and mention of the grant of the patent:
**03.05.2006 Bulletin 2006/18**

(51) Int Cl.:
*A61K 31/69* (2006.01) *C07F 5/02* (2006.01)
*C07D 207/08* (2006.01)

(21) Application number: **03255590.6**

(22) Date of filing: **09.09.2003**

(54) **Boronic acid salts useful in parenteral formulations for selective thrombin inhibition**

In parenteralen Formulierungen für die selektive Thrombininhibierung verwendbare Borsäuresalze

Sels d'acide boronique utiles dans les formulations parentérales pour l'inhibition sélective de la thrombine

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority:
**09.09.2002 GB 0220764**
**09.09.2002 GB 0220822**
**04.04.2003 GB 0307817**
**16.05.2003 GB 0311237**
**04.07.2003 GB 0315691**
**08.07.2003 US 485786 P**

(43) Date of publication of application:
**24.03.2004 Bulletin 2004/13**

(60) Divisional application:
**04076510.9 / 1 466 916**
**04076521.6 / 1 466 917**
**04076548.9 / 1 561 466**

(73) Proprietor: **TRIGEN LIMITED**
**London SW1A 1ES (GB)**

(72) Inventors:
- **Madge, David Jonathan**
  **Gower Street,**
  **London WC1E 6AU (GB)**
- **Dolman, Mark**
  **Gower Street,**
  **London WC1E 6AU (GB)**
- **Combe-Marzelle, Sophie-Marie**
  **26/27 Oxendon Street,**
  **London SW1Y 4EL (GB)**
- **Kennedy, Anthony James**
  **26/27 Oxendon Street,**
  **London SW1Y 4EL (GB)**
- **Kakkar, Sanjay Kumar**
  **26/27 Oxendon Street,**
  **London SW1Y 4EL (GB)**
- **Deadman, John Joseph**
  **576 Swann Street,**
  **Richmond,Victoria 3121 (AU)**
- **Walter, Armin**
  **33777 Halle/Westf. (DE)**
- **Olbrich, Alfred**
  **33777 Halle/Westf. (DE)**
- **Krimmer, Dieter**
  **6330 Cham-Steinhausen (CH)**
- **Weiland-Weibel, Andrea Maria Theresia**
  **6330 Cham-Steinhausen (CH)**

(74) Representative: **Harrison Goddard Foote**
**Belgrave Hall**
**Belgrave Street**
**Leeds LS2 8DD (GB)**

(56) References cited:
**WO-A-95/09634** **WO-A-95/09859**
**WO-A-02/059130** **US-A- 5 444 049**
**US-A- 5 462 964** **US-A- 5 563 127**
**US-A- 5 681 978**

- **SKORDALAKES EMMANUEL ET AL: "Bifunctional peptide boronate inhibitors of thrombin: Crystallographic analysis of inhibition enhanced by linkage to an exosite 1 binding peptide" BIOCHEMISTRY, vol. 37, no. 41, 13 October 1998 (1998-10-13), pages 14420-14427, XP002262060 ISSN: 0006-2960**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 400 245 B1

- KATZ B A ET AL: "Episelection: novel Ki approximately nanomolar inhibitors of serine proteases selected by binding or chemistry on an enzyme surface." BIOCHEMISTRY, vol. 34, no. 26, 4 July 1995 (1995-07-04), pages 8264-8280, XP002262061 ISSN: 0006-2960
- ELGENDY S ET AL: "New peptide boronic acid inhibitors of thrombin" TETRAHEDRON LETTERS, vol. 33, no. 29, 14 July 1992 (1992-07-14), pages 4209-4212, XP000609104 ISSN: 0040-4039
- SPENCER J ET AL: "Synthesis of ortho-modified mercapto- and piperazino-methyl-phenylbo ronic acid derivatives" TETRAHEDRON, ELSEVIER SCIENCE PUBLISHERS, AMSTERDAM, NL, vol. 58, no. 8, 18 February 2002 (2002-02-18), pages 1551-1556, XP004336392 ISSN: 0040-4020
- JOURNAL OF PHARMACEUTICAL SCIENCES, vol. 89, no. 6, 6 June 2000 (2000-06-06), pages 758-765,
- JOURNAL OF THE AMERICAN CHEMICAL SOCIETY, vol. 118, no. 5, 1996, pages 950-958,

## Description

BACKGROUND

The present disclosure relates to pharmaceutically useful products obtainable from organoboronic acids. The disdosure also relates to the use of members of the aforesaid dass of products, to their formulation, their preparation, their synthetic intermediates and to other subject matter.
The disdosure further relates to parenteral pharmaceutical formulations containing the described products.

### *Boronic Acid Compounds*

It has been known for some years that boronic acid compounds and their derivatives, e.g. esters, have biological activities, notably as inhibitors or substrates of proteases. For example, Koehler et al. *Biochemistry* 10:2477, 1971 report that 2-phenylethane boronic acid Inhibits the serine protease chymotrypsin at millimolar levels. The inhibition of chymotrypsin and subtilisin by arylboronic acids (phenylboronic acid, m-nitro-phenylboronic acid, m-aminophenylboronic acid, m-bromophenylboronic acid) is reported by Phillip et al, *Proc. Nat. Acad. Sci. USA* 68:478-480, 1971. A study of the inhibition of subtilisin Carlsberg by a variety of boronic acids, especially phenyl boronic acids substituted by Cl, Br, $CH_3$, $H_2N$, MeO and others, is described by Seufer-Wasserthal et al, *Biorg. Med. Chem.* 2(1):35-48, 1994.
In describing inhibitors or substrates of proteases, P1, P2, P3, etc. designate substrate or inhibitor residues which are amino-terminal to the scissile peptide bond, and S1, S2, S3, etc., designate the corresponding subsites of the cognate protease in accordance with: Schechter, I. and Berger, A. On the Size of the Active Site in Proteases, *Biochem.Biophys.Res.Comm.,* 27:157-162, 1967. In thrombin, the S1 binding site or "specificity pocket" is a well defined slit in the enzyme, whilst the S2 and S3 binding subsites (also respectively called the proximal and distal hydrophobic pockets) are hydrophobic and interact strongly with, respectively, Pro and (R)-Phe, amongst others.
Pharmaceutical research into serine protease inhibitors has moved from the simple arylboronic acids to boropeptides, i.e. peptides containing a boronic acid analogue of an $\alpha$-amino carboxylic acid. The boronic acid may be derivatised, often to form an ester. Shenvi (EP-A-145441 and US 4499082) disclosed that peptides containing an $\alpha$-aminoboronic acid with a neutral side chain were effective inhibitors of elastase and has been followed by numerous patent publications relating to boropeptide inhibitors of serine proteases. Specific, tight binding boronic acid inhibitors have been reported for elastase ($K_i$, 0.25nM), chymotrypsin ($K_i$, 0.25nM), cathepsin G ($K_i$, 21nM), $\alpha$-lytic protease ($K_i$, 0.25nM), dipeptidyl aminopeptidase type IV ($K_i$, 16pM) and more recently thrombin (Ac-D-Phe-Pro-boroArg-OH (DuP 714 initial $K_i$, 1.2nM).
Claeson et al (US 5574014 and others) and Kakkar et al (WO 92/07869 and family members including US 5648338) disclose thrombin inhibitors having a neutral C-terminal side chain, for example an alkyl or alkoxyalkyl side chain.
Modifications of the compounds described by Kakkar et al are included in WO 96/25427, directed to peptidyl serine protease inhibitors in which the P2-P1 natural peptide linkage is replaced by another linkage. As examples of non-natural peptide linkages may be mentioned $-CO_2-$, $-CH_2O-$, -NHCO-, $-CHYCH_2-$, -CH=CH-, $-CO(CH_2)_pCO-$ where p is 1, 2 or 3, -COCHY-, $-CO_2-CH_2NH-$, -CHY-NX-, $-N(X)CH_2-N(X)CO-$, -CH=C(CN)CO-, -CH(OH)-NH-, -CH(CN)-NH-, $-CH(OH)-CH_2-$ or -NH-CHOH-, where X is H or an amino protecting group and Y is H or halogen, especially F. Particular non-natural peptide linkages are $-CO_2-$ or $-CH_2O-$.
Mettemich (EP 471651 and US 5288707, the latter being assigned to Trigen Limited) discloses variants of Phe-Pro-BoroArg boropeptides in which the P3 Phe is replaced by an unnatural hydrophobic amino acid such as trimethylsilylalanine, p-tert.butyl-diphenyt-silyloxymethylphenylalanine or p-hydroxymethylphenylalanine and the P1 side chain may be neutral (alkoxyalkyl, alkylthioalkyl or trimethylsilylalkyl).
The replacement of the P2 Pro residue of borotripeptide thrombin inhibitors by an N-substituted glycine is described in Fevig J M et al *Bioorg. Med. Chem.* 8: 301-306 and Rupin A et al *Thromb. Haemost.* 78(4):1221-1227, 1997. See also US 5,585,360 (de Nanteuil et al).
Amparo (WO 96/20698 and family members including US 5698538) discloses peptidomimetics of the structure Aryl-linker-Boro(Aa), where Boro(Aa) may be an aminoboronate residue with a non-basic side chain, for example BoroMpg. The linker is of the formula $-(CH_2)_mCONR-$ (where m is 0 to 8 and R is H or certain organic groups) or analogues thereof in which the peptide linkage -CONR- is replaced by -CSNR-, $-SO_2NR-$, $-CO_2-$, -C(S)O- or $-SO_2O-$. Aryl is phenyl, naphthyl or biphenyl substituted by one, two or three moieties selected from a specified group. Most typically these compounds are of the structure Aryl-$(CH_2)_n$-CONH-$CHR^2$-$BY^1Y^2$, where $R^2$ is for example a neutral side chain as described above and n is 0 or 1.
Other aminoboronate or peptidoboronate inhibitors or substrates of serine proteases are described in:

- US 4935493
- EP 341661

- WO 94/25049
- WO 95/09859
- WO 96/12499
- WO 96/20689
- Lee S-L et al, *Biochemistry* 36:13180-13186, 1997
- Dominguez C et al, *Bioorg. Med. Chem. Lett.* 7:79-84, 1997
- EP 471651
- WO 94/20526
- WO 95/20603
- WO97/05161
- US 4450105
- US 5106948
- US 5169841.

Unfortunately, organoboronic acids can be relatively difficult to obtain in analytically pure form. Thus, alkylboronic acids and their boroxines are often air-sensitive. Korcek et al, *J. Chem. Soc. Perkin Trans.* 2:242, 1972, teaches that butylboronic acid is readily oxidized by air to generate 1-butanol and boric acid.

It is known that derivatisation of boronic acids as cyclic esters provides oxidation resistance. For example, Martichonok V et al *J. Am. Chem. Soc.* 118: 950-958, 1996 state that diethanolamine derivatisation provides protection against possible boronic acid oxidation. US Patent No 5,681,978 (Matteson DS et al) teaches that 1,2-diols and 1,3 diols, for example pinacol, form stable cydic boronic esters that are not easily oxidised.

Wu et al, *J. Pharm. Sci.,* 89:758-765, 2000, discuss the stability of the compound N-(2-pyrazine) carbonyl-phenylalanine-leucine boronic acid (LDP-341, also known as bortezomib), an anti-cancer agent. It is described how "during an effort to formulate [LDP-341] for parenteral administration, the compound showed erratic stability behaviour". The degradation pathways were investigated and it was concluded that the degradation was oxidative, the initial oxidation being attributed to peroxides or molecular oxygen and its radicals.

WO 02/059131 discloses boronic acid products which are described as stable. In particular, these products are certain boropeptides and/or boropeptidomimetics in which the boronic acid group has been derivatised with a sugar. Some of the disclosed compounds are sugar derivatives of LDP-341.

Many drugs comprise an active moiety which is a carboxylic acid. There are a number of differences between carboxylic acids and boronic acids, whose effects on drug delivery, stability and transport (amongst others) have not been Investigated. One feature of trivalent boron compounds is that the boron atom is $sp^2$ hybridised, which leaves an empty $2p_z$, orbital on the boron atom. A molecule of the type $BX_3$ can therefore act as an electron-pair acceptor, or Lewis acid. It can use the empty $2p_z$ orbital to pick up a pair of nonbonding electrons from a Lewis base to form a covalent bond. $BF_3$ therefore reacts with Lewis bases such as $NH_3$ to form acid-base complexes in which all of the atoms have a filled shell of valence electrons.

Boric acid, accordingly, can act as a Lewis acid, accepting $OH^-$:

$$B(OH)_3 + H_2O \rightarrow B(OH)_4^- + H^+$$

Further, boronic acids of the type $RB(OH)_2$ are dibasic and have two pKa's. Another point of distinction about boron compounds is the unusually short length of bonds to boron, for which three factors may be responsible:

1. Formation of pπ-pπ bonds;
2. Ionic-covalent resonance;
3. Reduced repulsions between non-bonding electrons.

The presumed equilibria of boronic and carboxylic acids in aqueous KOH are shown below (exduding formation of $RBO_2^{2-}$):

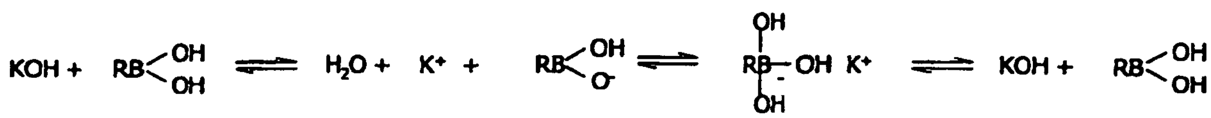

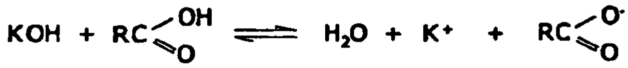

***Aminoboronate Synthesis***

It is known in the prior art to synthesise TRI 50c esters (see below) via the following process:

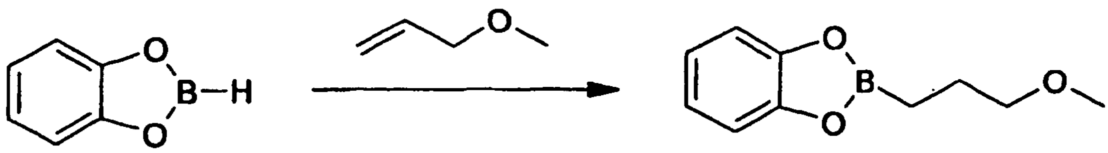

The product of the above step is then converted by known methods to, for example, TRI 50b (see below). See for example Deadman J et al, *J. Medicinal Chemistry* **1995**, *38*, 1511-1522.

***Thrombosis***

Hemostasis is the normal physiological condition of blood in which its components exist in dynamic equilibrium. When the equilibrium is disturbed, for instance following injury to a blood vessel, certain biochemical pathways are triggered leading, in this example, to arrest of bleeding via clot formation (coagulation). Coagulation is a dynamic and complex process in which proteolytic enzymes such as thrombin play a key role. Blood coagulation may occur through either of two cascades of zymogen activations, the extrinsic and intrinsic pathways of the coagulation cascade. The last protease in each pathway is thrombin, which acts to hydrolyze four small peptides (two FpA and two FpB) from each molecule of fibrinogen, thus deprotecting its polymerization sites. Once formed, the linear fibrin polymers may be cross-linked by factor XIIIa, which is itself activated by thrombin. In addition, thrombin is a potent activator of platelets, upon which it acts at specific receptors. Thrombin activation of platelets leads to aggregation of the cells and secretion of additional factors that further accelerate the creation of a hemostatic plug. Thrombin also potentiates its own production by the activation of factors V and VIII.

As indicated above, platelets play two important roles in normal hemostasis. First, by aggregating, they constitute the initial hemostatic plug which immediately curtails bleeding from broken blood vessels. Secondly, the platelet surface can become activated and potentiate blood clotting, a property referred to as platelet procoagulant activity. This may be observed as an increase in the rate of activation of prothrombin by factor Xa in the presence of factor Va and $Ca^{2+}$, referred to as the prothrombinase reaction. Normally, there are few (if any) clotting factors on the surface of unstimulated platelets but, when platelets are activated, negatively charged phospholipids (phosphatidylserine and phospatidylinositol) that are normally on the cytoplasmic side of the membrane become available and provide a surface on which two steps of the coagulation sequence occur. The phospholipid on the surface of activated platelets profoundly accelerates the reactions leading to the formation of thrombin, so that thrombin can be generated at a rate faster than its neutralisation by antithrombin III.

A thrombus can be considered as an abnormal product of a normal mechanism and can be defined as a mass or deposit formed from blood constituents on a surface of the cardiovascular system, for example of the heart or a blood vessel. Thrombosis can be regarded as the pathological condition wherein improper activity of the hemostatic mechanism results in intravascular thrombus formation. Three basic types of thrombi are recognised:

- the white thrombus which is usually seen in arteries and consists chiefly of platelets;
- the red thrombus which is found in veins and is composed predominantly of fibrin and red cells;
- the mixed thrombus which is composed of components of both white and red thrombi.

The composition of thrombi is influenced by the velocity of blood flow at their sites of formation. In general white platelet-rich thrombi form in high flow systems, while red coagulation thrombi form in regions of stasis. The high shear rate in arteries prevents the accumulation of coagulation intermediates on the arterial side of the circulation: only platelets have the capacity to form thrombi binding to the area of damage via von Willebrand factor. Such thrombi composed only of platelets are not stable and disperse. If the stimulus is strong then the thrombi will form again and then disperse continually until the stimulus has diminished. For the thrombus to stabilise, fibrin must form. In this respect, small amounts

of thrombin can accumulate within the platelet thrombus and activate factor Va and stimulate the platelet procoagulant activity. These two events lead to an overall increase in the rate of activation of prothrombin by factor Xa of 300,000 fold. Fibrin deposition stabilises the platelet thrombus. Indirect thrombin inhibitors, for example heparin, are not clinically effective at inhibiting stimulation of platelet procoagulant activity. Accordingly, a therapeutic agent which inhibits platelet procoagulant activity would be useful for treating or preventing arterial thrombotic conditions.

On the venous side of circulation, the thrombus is comprised of fibrin: thrombin can accumulate because of the slower flow on the venous side and platelets play only a minor role.

Thrombosis is thus not considered to be a single indication but, rather, is a dass of indications embracing distinct sub-classes for which differing therapeutic agents and/or protocols may be appropriate. Thus, regulatory authorities treat disorders such as, for example, deep vein thrombosis, cerebrovascular arterial thrombosis and pulmonary embolism as distinct indications for the purposes of licensing medicines. Two main sub-classes of thrombosis are arterial thrombosis and venous thrombosis. Arterial thrombosis includes such specific disorders as acute coronary syndromes [for example acute myocardial infarction (heart attack, caused by thrombosis in a coronary artery)], cerebrovascular arterial thrombosis (stroke, caused by thrombosis in the cerebrovascular arterial system) and peripheral arterial thrombosis. Examples of conditions caused by venous thrombosis are deep vein thrombosis and pulmonary embolism.

The management of thrombosis commonly involves the use of antiplatelet drugs (inhibitors of platelet aggregation) to control future thrombogenesis and thrombolytic agents to lyse the newly formed dot, either or both such agents being used in conjunction or combination with anticoagulants. Anticoagulants are used also preventatively (prophylactically) in the treatment of patients thought susceptible to thrombosis.

Currently, two of the most effective classes of drugs in clinical use as anticoagulants are the heparins and the vitamin K antagonists. The heparins are ill-defined mixtures of sulfated polysaccharides that bind to, and thus potentiate, the action of antithrombin III. Antithrombin III is a naturally occurring inhibitor of the activated dotting factors IXa, Xa, XIa, thrombin and probably XIIa (see Jaques, *Pharmacol Rev.* 31:99-166, 1980). The vitamin K antagonists, of which warfarin is the most well-known example, act indirectly by inhibiting the post-ribosomal carboxylations of the vitamin K dependent coagulation factors II, VII, IX and X (see Hirsch, *Semin. Thromb. Hemostasis* 12:1-11, 1986). While effective therapies for the treatment of thrombosis, heparins and vitamin K antagonists have the unfortunate side effects of bleeding, heparin-induced thrombocytopenia (in the case of heparin) and marked interpatient variability, resulting in a small and unpredictable therapeutic safety margin.

The use of direct acting inhibitors of thrombin and other serine protease enzymes of the coagulation system is expected to alleviate these problems. To that end, a wide variety of serine protease inhibitors have been tested, including boropeptides, i.e. peptides containing a boronic acid analogue of an $\alpha$-amino acid. Whilst direct acting boronic acid thrombin inhibitors have been discussed earlier in this specification, they are further described in the following section.

### *Neutral P1 Residue Boropeptide Thrombin Inhibitors*

Claeson et al (US 5574014 and others) and Kakkar et al (WO 92/07869 and family members including US 5648338) disclose lipophilic thrombin inhibitors having a neutral (uncharged) C-terminal (P1) side chain, for example an alkoxyalkyl side chain.

The Claeson et al and Kakkar et al patent families disclose boronate esters containing the amino acid sequence D-Phe-Pro-BoroMpg [(R)-Phe-Pro-BoroMpg], which are highly specific inhibitors of thrombin. Of these compounds may be mentioned in particular Cbz-(R)-Phe-Pro-BoroMpg-OPinacol (also known as TRI 50b). The corresponding free boronic acid is known as TRI 50c. For further information relating to TRI 50b and related compounds, the reader is referred to the following documents:

- Elgendy S et al., in *The Design of Synthetic Inhibitors of Thrombin,* Claeson G et al Eds, *Advances in Experimental Medicine,* 340:173-178, 1993
- Claeson G et al, *Biochem J.* 290:309-312, 1993
- Tapparelli C et al, *J Biol Chem,* 268:4734-4741, 1993
- Claeson G, in *The Design of Synthetic Inhibitors of Thrombin,* Claeson G et al Eds, *Advances in Experimental Medicine,* 340:83-91, 1993
- Phillip et al, in *The Design of Synthetic Inhibitors of Thrombin,* Claeson G et al Eds, *Advances in Experimental Medicine,* 340:67-77, 1993
- Tapparelli C et al, *Trends Pharmacol. Sci.* 14:366-376, 1993
- Claeson G, *Blood Coagulation and Fibrinolysis* 5:411-436, 1994
- Elgendy et al, *Tetrahedron* 50:3803-3812, 1994
- Deadman J et al, *J. Enzyme Inhibition* 9:29-41, 1995
- Deadman J et al, *J. Medicinal Chemistry* 38:1511-1522, 1995.

The tripeptide sequence of TRI 50b has three chiral centres. The Phe residue is considered to be of (R)-configuration and the Pro residue of natural (S)-configuration, at least in compounds with commercially useful inhibitor activity; the Mpg residue is believed to be of (R)-configuration in isomers with commercially useful inhibitor activity. Thus, the active, or most active, TRI 50b stereoisomer is considered to be of R,S,R configuration and may be represented as:

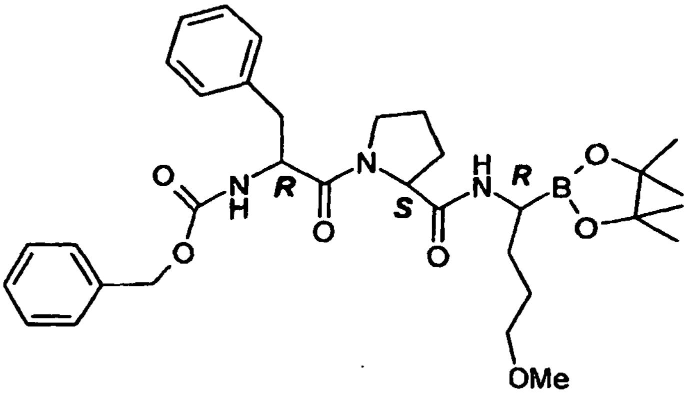

**(RSR)-TRI 50b: Cbz-(R)-Phe-(S)-Pro-(R)-boroMpg Pinacol**

Whilst indirect acting thrombin inhibitors have been found useful parenterally for the treatment of patients susceptible to or suffering from venous thrombosis, the same is not true of arterial thrombosis, because it would be necessary to raise the dosage used in the treatment of venous thrombosis by many times in order to treat (prevent) arterial thrombosis. Such raised dosages typically cause bleeding, which makes indirect acting thrombin inhibitors unsuitable or less preferable for treating arterial thrombosis. Heparin and its low molecular weight derivatives are indirect thrombin inhibitors, and so are unsuitable to treat arterial thrombosis. Oral direct thrombin inhibitors are in development for arterial indications but may have lower than desirable therapeutic indices, i.e. may have higher than desirable levels of bleeding at therapeutic doses.

Many organoboronic acid compounds may be classified as lipophilic or hydrophobic. Typically, such compounds include amongst others:

- boropeptides of which all or a majority of the amino acids are hydrophobic
- boropeptides of which at least half of the amino acids are hydrophobic and which have a hydrophobic N-terminal substituent (amino protecting group)
- non-peptides based on hydrophobic moieties.

GUIDE TO THE SPECIFICATION

This specification, as described in more detail below, concerns in particular pharmaceutical formulations comprising novel compounds. For convenience, the term **"Novel Products"** is sometimes (but not always) used in the description to refer to products comprising these novel compounds and compositions; for example the term is used in headings.

There are described synthetic methods devised in an earlier part of the research and development programme concerning the Novel Products, which methods generated one or more impurities and were otherwise not usable as such on an industrial scale. The term **"Synthetic Methods I"** is sometimes (but not always) used in the description to refer to such earlier methods; for example the term is used in headings. The subject matters relating to the Novel Products also include various aspects of subsequently devised synthetic techniques for making the novel compounds (or intermediates therefor) and relatively high purity products obtainable using these techniques; the term **"Synthetic Methods II"** is sometimes (but not always) used in the description to refer to such subsequent methods; for example the term is used in headings. At least in certain aspects, Synthetic Methods II represent a sub-set of Synthetic Methods I. The specific products of Synthetic Methods II are for convenience sometimes referred to as **"High Purity Products"**. The High Purity Products are a sub-set of the Novel Products.

The phrases Novel Products, Synthetic Methods I, Synthetic Methods II and High Purity Products are used solely for convenience and are not to be understood as limiting the scope of the invention, which includes the entire subject matter of the disclosure, including all materials, species, processes and uses thereof.

BRIEF SUMMARY OF THE DISCLOSURE

It has been discovered that TRI 50b tends to hydrolyse. Thus in acid conditions, for example of an HPLC assay, TRI 50b is converted to the acid form with a short half life, which implies potential hydrolysis in parenteral preparations containing water into species, comprising the free acid and its corresponding boronate anions in equilibrium therewith, taught in the literature to be unstable to degradation via de-boronation (carbon-boron bond cleavage), by an oxidative pathway (see e.g. Wu et al, discussed above).

The instability of TRI 50b to hydrolysis also presents potential disadvantages in preparation of the compound and its formulation, as well as in the storage of pharmaceutical formulations containing it

TRI 50c suffers further from instability, in that there is a problematic tendency for the boropeptide moiety itself to degrade via de-boronation (carbon-boron bond cleavage), such deboronation being taught by the literature to be oxidative (e.g. Wu et al, discussed above). The level of degradation can be remarkably high.

The properties discussed above of TRI 50b and TRI 50c will not be restricted to such compounds but will be shared by other boropeptide esters and acids, even if the properties of such other boropeptides differ quantitatively.

The present disclosure provides a solution to the problem of boronate diol ester and especially TRI50b instability which also provides the corresponding boronic acid with resistance to deboronation.

The present disclosure is predicated on, amongst other things, the finding that certain organoboronic acid products are indicated to be of enhanced stability.

The benefits of the present disclosure Include a solution to the problem of boronate diol ester and especially TRI 50b instability, that is to say the presently disclosed products provide *inter alia* pharmacologically active compounds which are more stable than TRI 50b and other comparable esters in the sense of stability to hydrolysis. The disclosure further includes a solution to the problem of organoboronic acid instability, that is to say the presently disclosed products provide *Inter alia* pharmacologically active compounds which are more stable to deboronation than TRI 50c. The stability provided within the framework of the disclosure is not absolute but is improved relative to the comparator compounds. The benefits offered by the disdosure further include the provision of products which have an unexpected usefulness in parenteral formulations.

There is disclosed an amino boronic acid derivative which avoids the disadvantages of pinacol esters. The disclosure further includes a peptide boronic acid derivative which is Indicated to be of enhanced stability. In particular, the disclosure includes amongst other subject matter boronic acid derivatives which are of relative stability to hydrolysis and deboronation and are useful In parenteral formulations for inhibiting thrombin.

The disclosure concerns a pharmaceutically acceptable base addition salt of certain organoboronic acid drugs, namely hydrophobic boropeptides (e.g. di- or tri-peptides) which are thrombin inhibitors having a non-basic P1 group. As a class, such salts are not only contrary to the direction of the prior art but additionally have an improved level of stability which cannot be explained or predicted on the basis of known chemistry.

The disclosure relates to base addition salts of boronic acids which have a neutral aminoboronic acid residue capable of binding to the thrombin S1 subsite linked through a peptide linkage to a hydrophobic moiety capable of binding to the thrombin S2 and S3 subsites. In a first aspect, the invention provides a parenteral pharmaceutical formulation that includes a pharmaceutically acceptable base addition salt of a boronic acid of formula (I):

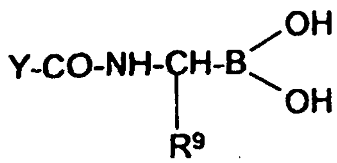

(I)

wherein

Y comprises a hydrophobic moiety which, together with the aminoboronic acid residue $-NHCH(R^9)-B(OH)_2$, has affinity for the substrate binding site of thrombin; and

$R^9$ is a straight chain alkyl group Interrupted by one or more ether linkages (e.g. 1 or 2) and in which the total number of oxygen and carbon atoms is 3, 4, 5 or 6 (e.g. 5) or $R^9$ is $-(CH_2)_m-W$ where m is 2, 3, 4 or 5 (e.g. 4) and W is -OH or halogen (F, Cl, Br or I). $R^9$ is an alkoxyalkyl group in one subset of compounds, e.g. alkoxyalkyl containing 4 carbon atoms.

The boronic acids (I) are hydrophobic boronic acid inhibitors of thrombin. Such inhibitors may contain hydrophobic amino acids in Y, and this class of amino acids includes those whose side chain is hydrocarbyl, hydrocarbyl containing an in-chain oxygen and/or linked to the remainder of the molecule by an in-chain oxygen or heteroaryl, or any of the aforesaid groups when substituted by hydroxy, halogen or trifluoromethyl. Representative hydrophobic side chains include alkyl, alkoxyalkyl, either of the aforesaid when substituted by at least one aryl or heteroaryl, aryl, heteroaryl, aryl substituted by at least one alkyl and heteroaryl substituted by at least one alkyl. Proline and other imino acids which

are ring-substituted by nothing or by one of the moieties listed in the previous sentence are also hydrophobic.

Some hydrophobic side chains contain from 1 to 20 carbon atoms, e.g. non-cydic moieties having 1, 2, 3 or 4 carbon atoms. Side chains comprising a cyclic group typically but not necessarily contain from 5 to 13 ring members and in many cases are phenyl or alkyl substituted by one or two phenyls.

Included are inhibitors which contain hydrophobic non-peptide moieties, which are typically based on moieties which may form a side chain of a hydrophobic amino acid, as described above.

Hydrophobic compounds may contain, for example, one amino group and/or one acid group (e.g. -COOH, -B$(OH)_2$). Generally, they do not contain multiple polar groups of any one type.

The disclosure comprises formulations comprising base addition salts of hydrophobic boronic acid inhibitors of thrombin, and therefore indudes such salts of peptide boronic acids which have a partition coefficient between 1-n-octanol and water expressed as log P of greater than 1.0 at physiological pH and 25°C. Some peptide boronic acids useful in the invention have a partition coefficient of at least 1.5. A dass of hydrophobic peptide boronic acids useful in the invention has a partition coefficient of no more than 5.

Some sub-dasses of hydrophobic organoboronic acids are those described by Formula (III) under the subsequent heading "Detailed Description of Several Examples".

Included in the disclosure are parenteral formulations containing a pharmaceutically acceptable base addition salt of a peptide boronic acid of formula (II):

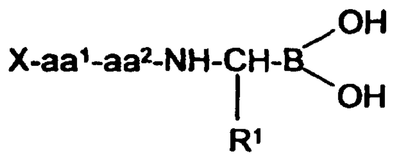

(II)

where:

X is a moiety bonded to the N-terminal amino group and may be H to form $NH_2$. The identity of X is not critical but may be a particular X moiety described above. In one example there may be mentioned benzyloxycarbonyl.

$aa^1$ is an amino acid having a hydrocarbyl side chain containing no more than 20 carbon atoms (e.g. up to 15 and optionally up to 13 C atoms) and comprising at least one cyclic group having up to 13 carbon atoms. In certain examples, the cyclic group(s) of $aa^1$ have/has 5 or 6 ring members. For instance, the cyclic group(s) of $aa^1$ may be aryl groups, particularly phenyl. Typically, there are one or two cyclic groups in the $aa^1$ side chain. Certain side chains comprise, or consist of, methyl substituted by one or two 5- or 6- membered rings.

More particularly, $aa^1$ is Phe, Dpa or a wholly or partially hydrogenated analogue thereof. The wholly hydrogenated analogues are Cha and Dcha.

$aa^2$ is an imino acid having from 4 to 6 ring members. Alternatively, $aa^2$ is Gly N-substituted by a $C_3$-$C_{13}$ hydrocarbyl group, e.g. a $C_3$-$C_8$ hydrocarbyl group comprising a $C_3$-$C_6$ hydrocarbyl ring; the hydrocarbyl group may be saturated, for example exemplary N-substituents are cydopropyl, cydobutyl, cyclopentyl and cydohexyl. As a hydrocarbyl group containing one or more unsaturated bonds may be mentioned phenyl and methyl or ethyl substituted by phenyl, e.g. 2-phenylethyl, as well as β,β-dialkylphenylethyl.

There is a debate in the literature as to whether boronates In aqueous solution form the 'trigonal' $B(OH)_2$ or 'tetrahedral' $B(OH)_3^-$ boron species, but NMR evidence seems to indicate that at a pH below the first pKa of the boronic acid the main boron species is the neutral $B(OH)_2$. In the duodenum the pH is likely to be between 6 and 7, so the trigonal species is likely to be predominant here. In any event, the symbol $-B(OH)_2$ includes tetrahedral as well as trigonal boron species, and throughout this specification symbols indicating trigonal boron species embrace also tetrahedral species. The symbol may further include boronic groups in anhydride form.

The salts may be in the form of solvates, particularly hydrates.

The salts may comprise, or consist essentially of, acid salts in which the boronic acid is singly deprotonated. The disclosure therefore includes products having a metal/boronate stoichiometry consistent with the boronate groups in the product predominantly (more than 50 mol %) carrying a single negative charge.

In particular, the parenteral formulations may comprise the salts in the solid phase, for example particulate salts for reconstitution as aqueous solutions prior to administration by injection or infusion. Such reconstituted solutions are also included in the disdosure.

As described further hereinafter, there are provided also haemodialysis solutions comprising a salt of the disclosure.

TRI 50c salts are obtained via TRI 50c esters. However, published synthetic routes to TRI 50c esters and thus to TRI 50c give rise to one or more impurities. Synthetic Methods I (unpublished as of filing this application) for making the salts give rise to one or more impurities and very high purity salts were not obtained. Further, the salts have proved most challenging to obtain in high purity. Thus, purification techniques which were applied failed to produce very high purity salts. HPLC will not be usable on an industrial scale to purify salts made via published TRI 50c ester syntheses and the salt preparation techniques of Synthetic Methods I. In other words, in order for the therapeutic benefits of TRI 50c salts to be provided to those in need thereof, the salts must be obtainable industrially in adequately pure form and the pure form must be attainable without the use of excessively expensive purification techniques.

The disclosure provides techniques for purifying organoboronic compounds and techniques for helping to maintain the purity of organoboronic compounds, and the products of such techniques. The present disclosure further provides techniques for use in making such high purity salts and the high purity salts themselves.

Embodiments of the formulations of the invention include boronic acid salts of specified purity.

The salts described herein include products obtainable by (having the characteristics of a product obtained by) reaction of the boronic acid with a strong base and the term "salt" herein is to be understood accordingly. The term "salt" in relation to the disclosed products, therefore, does not necessarily imply that the products contain discrete cations and anions and is to be understood as embracing products which are obtainable using a reaction of a boronic acid and a base. The disclosure embraces products which, to a greater or lesser extent, are in the form of a coordination compound. The disclosure thus provides also products obtainable by (having the characteristics of a product obtained by) reaction of a boronic acid (I) with a strong base a well as the therapeutic, including prophylactic, use of such products.

The present disclosure is not limited as to the method of preparation of the salts, provided that they contain a boronate species derived from boronic acid (I) and a counter-ion. Such boronate species may be boronate anions in any equilibrium form thereof. The term "equilibrium form" refers to differing forms of the same compounds which may be represented in an equilibrium equation (e.g. boronic acid in equilibrium with a boronic anhydride and in equilibrium with different boronate ions). Boronates in the solid phase may form anhydrides and the disclosed boronate salts when in the solid phase may comprise boronate anhydrides, as a boronic equilibrium species. It is not required that the salts be prepared by reaction of a base containing the counter-ion and the boronic acid (I). Further, the disclosure includes salt products which might be regarded as indirectly prepared by such an acid/base reaction as well as salts obtainable by (having the characteristics of products obtained by) such indirect preparation. As examples of possibly indirect preparation may be mentioned processes in which, after initial recovery of the salt, it is purified and/or treated to modify its physicochemical properties, for example to modify solid form or hydrate form, or both.

In some embodiments, the cations of the salts are monovalent.

In some embodiments the salts comprise anhydride species; in others they are essentially free of anhydride species.

Further aspects and embodiments of the disclosure are set forth in the following description and claims.

Throughout the description and claims of this specification, the words "comprise" and "contain" and variations of the words, for example "comprising" and "comprises", mean "including but not limited to", and are not intended to (and do not) exclude other moieties, additives, components, integers or steps.

This patent contains data indicating that the stability (resistance to deboronation) of organoboronic acids may be increased by providing them in the form of salts, e.g. metal salts. In single experiments, the ammonium salt of TRI 50c appeared to decompose on drying to yield ammonia, whilst the choline salt demonstrated rapid decomposition to a deboronated impurity. Although experiments have not been conducted to reproduce these unrepeated observations, there is provided a sub-class in which the ammonium and choline salts are excluded. The salt may be an acid salt. In any event, this stabilisation technique forms part of the disclosure and is applicable, *inter alia,* to organoboronic acids described under the heading "BACKGROUND" and to organoboronic acids described in publications mentioned under that heading.

BRIEF DESCRIPTION OF THE DRAWINGS

Figure 1 is a chart referred to in Example 35, showing the results of a thrombin amidolytic assay of TRI 1405 (TRI 50c magnesium salt) and TRI 50b, where Vmax is the maximum rate of reaction measured by amidolytic assay.

Figure 2 is a plot referred to in Example 39, showing intravenous phase dearance and kinetics following a single dose of TRI 50b or TRI 50c.

DETAILED DESCRIPTION OF SEVERAL EXAMPLES

**Glossary**

The following terms and abbreviations are used in this specification:

The expression "acid salt" as applied to a salt of a boronic acid refers to salts of which a single -OH group of the trigonally-represented acid group $-B(OH)_2$ is deprotonated. Thus salts wherein the boronate group carries a single negative charge and may be represented as $-B(OH)(O^-)$ or as $[-B(OH)_3]^-$ are acid salts. The expression encompasses salts of a cation having a valency n wherein the molar ratio of boronic acid to cation is approximately n to 1. In practical terms, the observed stoichiometry is unlikely to be exactly n:1 but will be consistent with a notional n:1 stoichiometry. For example, the observed mass of the cation might vary from the calculated mass for a n:1 stoichiometry by no more than about 10%, e.g. no more than about 7.5%; in some cases an observed mass of a cation might vary from the calculated mass by no more than about 1%. Calculated masses are suitably based on the trigonal form of the boronate. (At an atomic level, a salt stoichiometrically consistent with being an acid salt might contain boronates in a mix of protonation states, whose average approximates to single deprotonation and such "mixed" salts are included in the term "acid salt"). Examples of acid salts are monosodium salts and hemicaldum salts.

α-Aminoboronic acid or Boro(aa) refers to an amino acid in which the $CO_2$ group has been replaced by $BO_2$.

The term "amino-group protecting moiety" refers to any group used to derivatise an amino group, especially an N-terminal amino group of a peptide or amino acid. Such groups include, without limitation, alkyl, acyl, alkoxycarbonyl, aminocarbonyl, and sulfonyl moieties. However, the term "amino-group protecting moiety" is not intended to be limited to those particular protecting groups that are commonly employed in organic synthesis, nor is it intended to be limited to groups that are readily cleavable.

The phrase "pharmaceutically acceptable" is employed herein to refer to those compounds, materials, compositions, and/or dosage forms which are, within the scope of sound medical judgment, suitable for use in contact with the tissues of human beings or animals without excessive toxicity, irritation, allergic response, or other problem or complication, commensurate with a reasonable benefit/risk ratio.

The expression "thrombin inhibitor" refers to a product which, within the scope of sound pharmacological judgement, is potentially or actually pharmaceutically useful as an inhibitor of thrombin, and includes reference to substance which comprises a pharmaceutically active species and is described, promoted or authorised as a thrombin inhibitor. Such thrombin inhibitors may be selective, that is they are regarded, within the scope of sound pharmacological judgement, as selective towards thrombin in contrast to other proteases; the term "selective thrombin inhibitor" includes reference to substance which comprises a pharmaceutically active species and is described, promoted or authorised as a selective thrombin inhibitor.

The term "heteroaryl" refers to a ring system which has at least one (e.g. 1, 2 or 3) in-ring heteroatoms and has a conjugated in-ring double bond system. The term "heteroatom" includes oxygen, sulfur and nitrogen, of which sulfur is sometimes less preferred.

"Natural amino acid" means an L-amino acid (or residue thereof) selected from the following group of neutral (hydrophobic or polar), positively charged and negatively charged amino acids:

Hydrophobic amino acids

A = Ala = alanine

V = Val = valine

I = Ile = isoleucine

L = Leu = leucine

M = Met = methionine

F = Phe = phenylalanine

P = Pro = proline

W = Trp = tryptophan

Polar (neutral or uncharged) amino acids

N = Asn = asparagine
C = Cys = cysteine
Q = Gln = glutamine
G = Gly = glycine
S = Ser = serine
T = Thr = threonine
Y = Tyr = tyrosine

Positively charged (basic) amino acids

R = Arg = arginine
H = His = histidine
K = Lys = lysine

Negatively charged amino acids

D = Asp = aspartic acid
E = Glu = glutamic acid.

ACN = acetonitrile
Amino acid = $\alpha$-amino acid
Base addition salt = a salt which is prepared from addition of an inorganic base or an organic base to a free acid (in this case the boronic acid).
Cbz = benzyloxycarbonyl
Cha = cydohexylalanine (a hydrophobic unnatural amino acid)
Charged (as applied to drugs or fragments of drug molecules, e.g. amino acid residues) = carrying a charge at physiological pH, as in the case of an amino, amidino or carboxy group
Dcha = dicyclohexylalanine (a hydrophobic unnatural amino acid)
Dpa = diphenylalanine (a hydrophobic unnatural amino acid)
Drug = a pharmaceutically useful substance, whether the active in vivo principle or a prodrug
i.v. = intravenous
Mpg = 3-methoxypropylglycine (a hydrophobic unnatural amino acid)
Multivalent = valency of at least two, for example two or three
Neutral (as applied to drugs or fragments of drug molecules, e.g. amino acid residues) = uncharged = not carrying a charge at physiological pH
Pinac = Pinacol = 2,3-dimethyl-2,3-butanediol
Pinanediol = 2,3-pinanediol = 2,6,6-trimethylbicydo [3.1.1] heptane-2,3-diol
Pip = pipecolinic acid
Room temperature = 25°C $\pm$ 2°C
s.c. = subcutaneous
Strong base = a base having a sufficiently high pKb to react with a boronic acid. Suitably such bases have a pKb of 7 or more, e.g. 7.5 or more, for example about 8 or more
THF = tetrahydrofuran
Thr = thrombin

***Novel Products - The Compounds***

The invention provides the products and uses set forth in the claims. This section of the patent describes the compounds used in the daimed products and uses.

The products of the disclosure comprise base addition salts of boronic acids which are members of a dass having a neutral aminoboronic acid residue capable of binding to the thrombin S1 subsite linked through a peptide linkage to a hydrophobic moiety capable of binding to the thrombin S2 and S3 subsites, or are members of a sub-class thereof. One aspect of the invention resides in parenteral medicaments comprising base addition salts of selective thrombin inhibitors which are acids of this type, the salts having a cation of valency n and an observed stoichiometry consistent with a notional acid:cation stoichiometry of n:1.

The disclosure includes parenteral formulations, products, uses and methods as set forth in the claims which involve base addition salts of acids of formula (I):

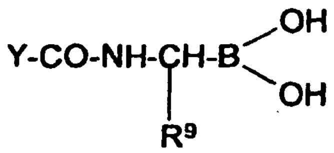

(I)

wherein

Y comprises a hydrophobic moiety which, together with the aminoboronic acid residue $-NHCH(R^9)-B(OH)_2$, has affinity for the substrate binding site of thrombin; and

$R^9$ is a straight chain alkyl group interrupted by one or more ether linkages and in which the total number of oxygen and carbon atoms is 3, 4, 5 or 6 (e.g. 5) or $R^9$ is $-(CH_2)_m-W$ where m is from 2, 3, 4 or 5 (e.g. 4) and W is -OH or halogen (F, Cl, Br or I). As examples of straight chain alkyl interrupted by one or more ether linkages (-O-) may be mentioned alkoxyalkyl (one interruption) and alkoxyalkoxyalkyl (two interruptions). $R^9$ is an alkoxyalkyl group in one subset of compounds, e.g. alkoxyalkyl containing 4 carbon atoms.

Typically, YCO- comprises an amino acid residue (whether natural or unnatural) which binds to the S2 subsite of thrombin, the amino acid residue being N-terminally linked to a moiety which binds the S3 subsite of thrombin.

In one class of Formula (I) acids, YCO- is an optionally N-terminally protected dipeptide residue which binds to the S3 and S2 binding sites of thrombin and the peptide linkages in the acid are optionally and independently N-substituted by a $C_1$-$C_{13}$ hydrocarbyl group optionally containing in-chain and/or in-ring nitrogen, oxygen or sulfur and optionally substituted by a substituent selected from halo, hydroxy and trifluoromethyl. The N-terminai protecting group, when present, may be a group X as defined above (other than hydrogen). Normally, the acid contains no N-substituted peptide linkages; where there is an N-substituted peptide linkage, the substituent is often 1C to 6C hydrocarbyl, e.g. saturated hydrocarbyl; the N-substituent comprises a ring in some embodiments, e.g. cycloalkyl, and may be cyclopentyl, for example. One class of acids has an N-terminal protecting group (e.g. an X group) and unsubstituted peptide linkages.

Where YCO- is a dipeptide residue (whether or not N-terminally protected), the S3-binding amino acid residue may be of R configuration and/or the S2-binding residue may of S configuration. The fragment $-NHCH(R^9)-B(OH)$ may of R configuration. The disclosure is not restricted to chiral centres of these conformations, however.

In one dass of compounds, the side chain of the P3 (S3-binding) amino acid and/or the P2 (S2-binding) amino acid is a moiety other than hydrogen selected from a group of formula A or B:

$$-(CO)_a-(CH_2)_b-D_c-(CH_2)_d-E \qquad (A)$$

$$-(CO)_a-(CH_2)_b-D_c-C_e(E^1)(E^2)(E^3) \qquad (B)$$

wherein

a is 0 or 1;

e is 1;

b and d are independently 0 or an integer such that (b+d) is from 0 to 4 or, as the case may be, (b+e) is from 1 to 4;

c is 0 or 1;

D is O or S;

E is H, $C_1$-$C_6$ alkyl, or a saturated or unsaturated cydic group which normally contains up to 14 members and particularly is a 5-6 membered ring (e.g. phenyl) or an 8-14 membered fused ring system (e.g. naphthyl), which alkyl or cyclic group is optionally substituted by up to 3 groups (e.g. 1 group) independently selected from $C_1$-$C_6$ trialkylsilyl, -CN, $-R^{13}$, $-R^{12}OR^{13}$, $-R^{12}COR^{13}$, $-R^{12}CO_2R^{13}$ and $-R^{12}O_2CR^{13}$, wherein $-R^{12}$ is $-(CH_2)_f-$ and $-R^{13}$ is $-(CH_2)_gH$, or by a moiety whose non-hydrogen atoms consist of carbon atoms and in-ring heteroatoms and number from 5 to 14 and which contains a ring system (e.g. an aryl group) and optionally an alkyl and/or alkylene group, wherein f and g are each independently from 0 to 10, g particularly being at least 1 (although -OH may also be mentioned as a substituent), provided that (f+g) does not exceed 10, more particularly does not exceed 6 and most particularly is 1, 2, 3 or 4, and provided that there is only a single substituent if the substituent is a said moiety containing a ring system, or E is $C_1$-$C_6$ trialkylsilyl; and $E^1$, $E^2$ and $E^3$ are each independently selected from $-R^{15}$ and $-J-R^{15}$, where J is a 5-6 membered ring and $-R^{15}$ is selected from $C_1$-$C_6$ trialkylsilyl, -CN, $-R^{13}$, $-R^{12}OR^{13}$, $-R^{12}COR^{13}$, $-R^{12}CO_2R^{13}$, $-R^{12}O_2CR^{13}$, and one or two halogens (e.g. in the latter case to form a $-J-R^{15}$ moiety which is dichlorophenyl), where $R^{12}$ and $R^{13}$ are, respectively, an $R^{12}$ moiety and an $R^{13}$ moiety as defined above (in some acids where $E^1$, $E^2$ and $E^3$ contain an $R^{13}$ group, g is 0 or 1);

in which moiety of Formula (A) or (B) any ring is carbocyclic or aromatic, or both, and any one or more hydrogen atoms bonded to a carbon atom is optionally replaced by halogen, especially F.

In certain examples, a is 0. If a is 1, c may be 0. In particular examples, (a+b+c+d) and (a+b+c+e) are no more than 4 and are more especially 1, 2 or 3. (a+b+c+d) may be 0.

Exemplary groups for E, $E^1$, $E^2$ and $E^3$ include aromatic rings such as phenyl, naphthyl, pyridyl, quinolinyl and furanyl, for example; non-aromatic unsaturated rings, for example cydohexenyl; saturated rings such as cyclohexyl, for example. E may be a fused ring system containing both aromatic and non-aromatic rings, for example fluorenyl. One class of E, $E^1$, $E^2$ and $E^3$ groups are aromatic (induding heteroaromatic) rings, especially 6-membered aromatic rings. In some compounds, $E^1$ is H whilst $E^2$ and $E^3$ are not H; in those oompounds, examples of $E^2$ and $E^3$ groups are phenyl (substituted or unsubstituted) and $C_1$-$C_4$ alkyl, e.g. methyl.

In one class of embodiments, E contains a substituent which is $C_1$-$C_6$ alkyl, ($C_1$-$C_5$ alkyl)carbonyl, carboxy $C_1$-$C_5$ alkyl, aryl (including heteroaryl), especially 5-membered or preferably 6-membered aryl (e.g. phenyl or pyridyl), or arylalkyl (e.g. arylmethyl or arylethyl where aryl may be heterocyclic and is preferably 6-membered).

In another dass of embodiments, E contains a substituent which is $OR^{13}$, wherein $R^{13}$ can be a 6-membered ring, which may be aromatic (e.g. phenyl) or is alkyl (e.g. methyl or ethyl) substituted by such a 6-membered ring.

A class of moieties of formula A or B are those in which E is a 6-membered aromatic ring optionally substituted, particularly at the 2-position or 4-position, by $-R^{13}$ or $-OR^{13}$.

The disclosure includes salts in which the P3 and/or P2 side chain comprises a cyclic group in which 1 or 2 hydrogens have been replaced by halogen, e.g. F or Cl.

The disclosure includes a class of salts in which the side chains of formula (A) or (B) are of the following formulae (C), (D) or (E):

$$C_qH_{2q}CHT_2 \qquad (C)$$

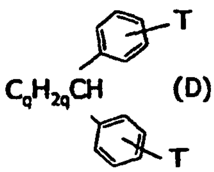
(D)

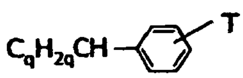
(E)

wherein q is from 0 to 5, e.g. is 0, 1 or 2, and each T is independently hydrogen, one or two halogens (e.g. F or Cl), $-SiMe_3$, -CN, $-R^{13}$, $-OR^{13}$, $-COR^{13}$, $-CO_2R^{13}$ or $-O_2CR^{13}$. In some embodiments of structures (D) and (E), T is at the 4-position of the phenyl group(s) and is $-R^{13}$, $-OR^{13}$, $-COR^{13}$, $-CO_2R^{13}$ or $-O_2CR^{13}$, and $R^{13}$ is $C_1$-$C_{10}$ alkyl and more particularly $C_1$-$C_6$ alkyl. In one sub-dass, T is $-R^{13}$ or $-OR^{13}$, for example in which f and g are each independently 0, 1, 2 or 3; in some side chains groups of this sub-class, T is $-R^{12}OR^{13}$ and $R^{13}$ is H.

In one class of the moieties, the side chain is of formula (C) and each T is Independently $R^{13}$ or $OR^{13}$ and $R^{13}$ is $C_1$-$C_4$ alkyl. In some of these compounds, $R^{13}$ is branched alkyl and in others it is straight chain. In some moieties, the number of carbon atoms is from 1 to 4.

In many dipeptide fragments YCO- (which dipeptides may be N-terminally protected or not), the P3 amino acid has a side chain of formula (A) or (B) as described above and the P2 residue is of an imino acid.

The disclosure therefore includes medicaments comprising salts, e.g. metal salts, of organoboronic acids which are thrombin inhibitors, particularly selective thrombin inhibitors, having a neutral $P_1$ (S1-binding) moiety. For more information about moieties which bind to the S3, S2 and S1 sites of thrombin, see for example Tapparelli C et al, *Trends Pharmacol. Sci.* 14: 366-376, 1993; Sanderson P et at, *Current Medicinal Chemistry,* 5: 289-304, 1998; Rewinkel J et al, *Current Pharmaceutical Design,* 5:1043-1075, 1999; and Cobum C *Exp. Opin. Ther. Patents* 11(5): 721-738, 2001. The thrombin inhibitory salts of the disclosure are not limited to those having S3, S2 and S1 affinity groups described in the publications listed in the preceding sentence.

The boronic acids may have a Ki for thrombin of about 100 nM or less, e.g. about 20 nM or less.

A subset of the Formula (I) acids comprises the acids of Formula (III):

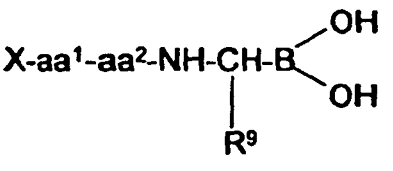
(III)

X is a moiety bonded to the N-terminal amino group and may be H to form $NH_2$. The identity of X is not critical but may be a particular X moiety described above. In one example there may be mentioned benzyloxycarbonyl.

In certain examples X is $R^6$-$(CH_2)_p$-C(O)-, $R^6$-$(CH_2)_p$ $S(O)_2$-, $R^6$-$(CH_2)_p$-NH-(O)- or $R^6$-$(CH_2)_p$-O-C(O)- wherein p is 0, 1, 2, 3, 4, 5 or 6 (of which 0 and 1 are preferred) and $R^6$ is H or a 5 to 13-membered cyclic group optionally substituted by 1, 2 or 3 substituents selected from halogen, amino, nitro, hydroxy, a $C_5$-$C_6$ cyclic group, $C_1$-$C_4$ alkyl and $C_1$-$C_4$ alkyl containing, and/or linked to the 5 to 13-membered cyclic group through, an in-chain 0, the aforesaid alkyl groups optionally being substituted by a substituent selected from halogen, amino, nitro, hydroxy and a $C_5$-$C_6$ cyclic group. More particularly X is $R^6$-$(CH_2)_p$-C(O)- or $R^6$-$(CH_2)_p$-O-C(O)- and p is 0 or 1. Said 5 to 13-membered cyclic group is often aromatic or heteroaromatic, for example is a 6-membered aromatic or heteroaromatic group. In many cases, the group is not substituted.

Exemplary X groups are (2-pyrazine) carbonyl, (2-pyrazine) sulfonyl and particularly benzyloxycarbonyl.

$aa^1$ is an amino acid residue having a hydrocarbyl side chain containing no more than 20 carbon atoms (e.g. up to 15 and optionally up to 13 C atoms) and comprising at least one cydic group having up to 13 carbon atoms. In certain examples, the cyclic group(s) of $aa^1$ have/has 5 or 6 ring members. For instance, the cyclic group(s) of $aa^1$ may be aryl groups, particularly phenyl. Typically, there are one or two cyclic groups in the $aa^1$ side chain. Certain side chains comprise, or consist of, methyl substituted by one or two 5- or 6- membered rings.

More particularly, $aa^1$ is Phe, Dpa or a wholly or partially hydrogenated analogue thereof. The wholly hydrogenated analogues are Cha and Dcha.

$aa^2$ is an imino acid residue having from 4 to 6 ring members. Alternatively, $aa^2$ is Gly N-substituted by a $C_3$-$C_{13}$ hydrocarbyl group, e.g. a $C_3$-$C_8$ hydrocarbyl group comprising a $C_3$-$C_6$ hydrocarbyl ring; the hydrocarbyl group may be saturated, for example exemplary N-substituents are cydopropyl, cydobutyl, cydopentyl and cyclohexyl. As a hydrocarbyl group containing one or more unsaturated bonds may be mentioned phenyl and methyl or ethyl substituted by phenyl, e.g. 2-phenylethyl, as well as β,β-dialkylphenylethyl.

An exemplary dass of products comprises those in which $aa^2$ is a residue of an imino acid of formula (IV)

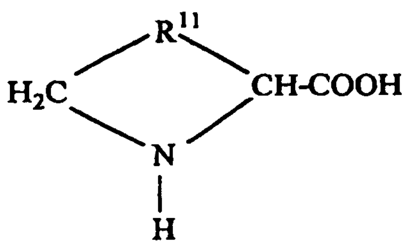

(IV),

where $R^{11}$ is $-CH_2-$,$-CH_2-CH_2-$,$-S-CH_2-$or $-CH_2-CH_2-CH_2-$, which group when the ring is 5 or 6-membered is optionally substituted at one or more $-CH_2-$ groups by from 1 to 3 $C_1$-$C_3$ alkyl groups, for example to form the $R^{11}$ group $-S-C(CH_3)_2-$. Of these imino acids, azetidine-2-carboxylic acid, especially (s)-azetidine-2-carboxylic acid, and more particularly proline are illustrative.

It will be appreciated from the above that a very preferred class of products consists of those in which $aa^1$-$aa^2$ is Phe-Pro. In another preferred dass, $aa^1$-$aa^2$ is Dpa-Pro. In other products, $aa^1$-$aa^2$ is Cha-Pro or Dcha-Pro. Of course, also included are corresponding product dasses in which Pro is replaced by (s)-azetidine-2-carboxylic acid.

$R^9$ is as defined previously and may be a moiety $R^1$ of the formula $-(CH_2)_S-Z$. Integer s is 2, 3 or 4 and W is -OH, -OMe, -OEt or halogen (F, Cl, I or, preferably, Br). Particularly illustrative Z groups are -OMe and -OEt, especially -OMe. In certain examples s is 3 for all Z groups and, indeed, for all compounds of the disdosure. Particular $R^1$ groups are 2-bromoethyl, 2-chloroethyl, 2-methoxyethyl, 4-bromobutyl, 4-chlorobutyl, 4-methoxybutyl and, especially, 3-bromopropyl, 3-chloropropyl and 3-methoxypropyl. Most preferably, $R^1$ is 3-methoxypropyl. 2-Ethoxyethyl is another preferred $R^1$ group.

Accordingly, a specific class of salts consists of those of acids of the formula X-Phe-Pro-Mpg-$B(OH)_2$, especially Cbz-Phe-Pro-Mpg-$B(OH)_2$; also included are analogues of these compounds in which Mpg is replaced by a residue with another of the $R^1$ groups and/or Phe is replaced by Dpa or another $aa^1$ residue.

The $aa^1$ moiety of the salt is preferably of R configuration. The $aa^2$ moiety is preferably of (S)-configuration. Particularly preferred salts have $aa^1$ of (R)-configuration and $aa^2$ of (S)-configuration. The chiral centre -NH-CH($R^1$)-B- is preferably of (R)-configuration. It is considered that commercial formulations will have the chiral centres in (R,S,R) arrangement, as for example in the case of salts of Cbz-Phe-Pro-BoroMpg-OH:

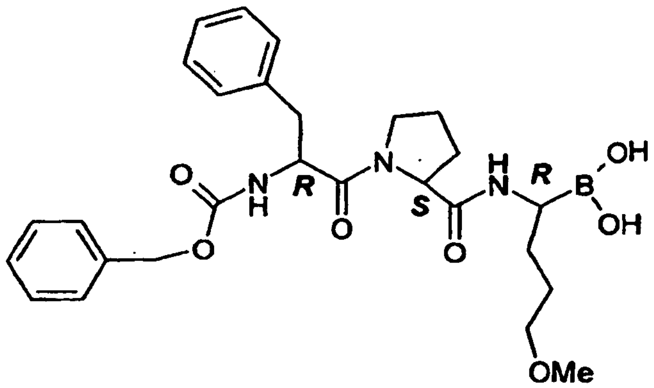

**Cbz-(R)-Phe-(S)-Pro-(R)-boroMpg-OH**

The disclosure includes salts of Cbz-(R)-Phe-(S)-Pro-(R)-boroMpg-OH (and of other compounds of the formula X-(R)-Phe-(5)-Pro-(R)-boroMpg-OH) which are at least 90% pure, e.g. at least 95% pure.

In broad terms, the salts described herein may be considered to correspond to reaction products of an organoboronic acid as described above with a strong base, e.g. a basic metal compound; the salts are however not limited to products resulting from such a reaction and may be obtained by alternative routes.

The salts are therefore obtainable by contacting an acid of formula (I) with a strong base. The disclosure thus contemplates products (compositions of matter) having the characteristics of a reaction product of an acid of formula (I) and a strong base. The base is pharmaceutically acceptable.

As suitable salts may be mentioned salts of metals, e.g. of monovalent or divalent metals, and stronger organic bases, for example:

1. Alkali metal salts;

2. Divalent, e.g. alkaline earth metal, salts;

3. Group III metals;

4. Salts of strongly basic organic nitrogen-containing compounds, induding:

    4A. Salts of guanidines and their analogues;

    4B. Salts of strongly basic amine, examples of which include (i) aminosugars and (ii) other amines.

Of the above salts, particularly illustrative are alkali metals, especially Na and U. Also illustrative are aminosugars.

Specific salts are of the acid boronate though in practice the acid salts may contain a very small proportion of the doubly deprotonated boronate. The term "acid boronate" refers to trigonal $-B(OH)_2$ groups in which one of the B-OH groups is deprotonated as well as to corresponding tetrahedral groups in equilibrium therewith. Acid boronates have a stoichiometry consistent with single deprotonation.

The disclosure includes therefore products (compositions of matter) which comprise salts which may be represented by formula (V):

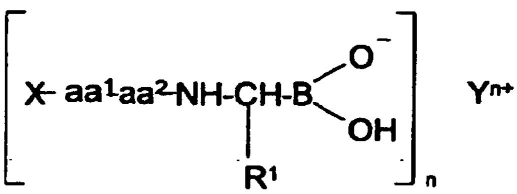

(V)

where $Y^{n+}$ is a pharmaceutically acceptable cation obtainable from a strong base, and $aa^1$, $aa^2$, X and $R^1$ are as defined above. Also included are products in which $R^1$ is replaced by another $R^9$ group.

One dass of salts have a solubility of about 10 mM or more, e.g. of at least about 20mM, when their solubility is determined as described in the examples at a dissolution of 25mg/ml. More particularly yet they have a solubility of least 50mM when their solubility is determined as described in the examples at a dissolution of 50mg/ml.

The disclosure includes salts of boronic acids (I) having an observed stoichiometry consistent with the salt being of (being representable by) the formula "$(boronate^-)_n$ $cation^{n+}$". One class of such salts are represented by the formula:

$$[Cbz\text{-}(R)\text{-}Phe\text{-}(S)\text{-}Pro\text{-}(R)\text{-}Mpg\text{-}B(OH)(O^-)]M^+$$

where $M^+$ represents a monovalent cation, especially an alkali metal cation. It will be understood that the above representation is a notional representation of a product whose observed stoichiometry is unlikely to be literally and exactly 1: 1. In any event, a particular salt is Cbz-(R)-Phe-(S)-Pro-(R)-Mpg-$B(OH)_2$ monosodium salt (TGN 255). In the above formula, the trigonally-represented boronate represents, as always, boronates which are trigonal, tetrahedral or mixed trigonal/tetrahedral.

Particularly exemplary are products which comprise:

(i) species selected from (a) acids of formula (VIII): X-(R)-Phe-(S)-Pro-(R)-Mpg-$B(OH)_2$ where X is H or an amino-protecting group, especially Cbz, (b) boronate anions thereof, and (c) any equilibrium form of the aforegoing (e.g. an anhydride); and

(ii) ions having a valency n in combination with said species, the species and said ions having an observed stoichiometry consistent with a notional species:ion stoichiometry of n:1. In one dass of salts, n is 1.

Considering the counter-ions in turn:

1. Monovalent metal, especially alkali metal salts

Suitable alkali metals include lithium, sodium and potassium. All of these are remarkably soluble. Lithium and sodium are illustrative because of their high solubility. The lithium and particularly sodium salts are of surprisingly high solubility in relation to potassium amongst others. Sodium is most used in many instances. Salts containing mixtures of alkali metals are contemplated by the disclosure.

The disclosure indudes products comprising salts of the formula (VI)

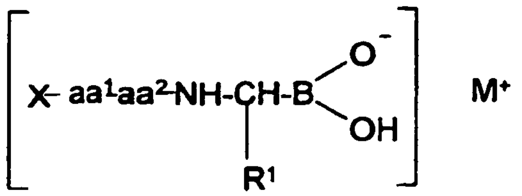

(VI)

where $M^+$ is an alkali metal ion and $aa^1$, $aa^2$, X and $R^1$ are as defined above, as well as salts in which both hydroxy groups of the boronate group are in salt form (preferably with another identical $M^+$ group) and mixtures of such salts. Included also are products wherein $R^1$ is replaced by another $R^9$ group.

2 Divalent, e.g. alkaline earth metal (Group II metal) salts

One example of a divalent metal is calcium. Another suitable divalent metal is magnesium. Also contemplated is zinc. The divalent metals are usually used in a boronic acid:metal ratio of substantially 2:1, in order to achieve the preferred monovalent boronate moiety. Salts containing mixtures of divalent metals, e.g. mixtures of alkaline earth metals, are also contemplated.

Further disclosed are products (compositions of matter) which comprise salts which may be represented by the formula (VII):

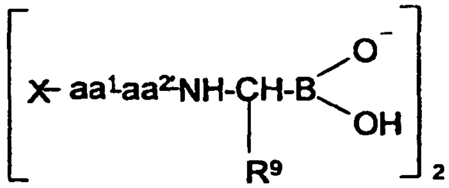 $M^{2+}$ (VII)

where $M^{2+}$ is a divalent metal cation, e.g. an alkaline earth metal or zinc cation, and $aa^1$, $aa^{2'}$, X and $R^9$ are as defined above, as well as salts in which both hydroxy groups of the boronate group are deprotonated and mixtures of such salts. As previously indicated, the boronate may comprise a tetrahedral species.

3. Group III metals

Suitable Group III metals include aluminium and gallium. Salts containing mixtures of Group III metals are also contemplated..

The disclosure includes products comprising salts of the formula (VIII):

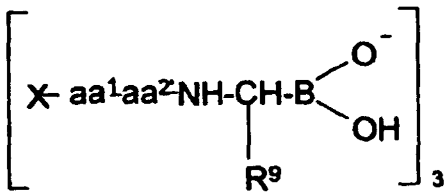 $M^{3+}$ (VIII)

where $M^{3+}$ is a Group III metal ion and $aa^1$, $aa^{2'}$, X and $R^9$ are as defined above, as well as salts in which both hydroxy groups of the boronate group are in salt form and mixtures of such salts. As previously indicated, the boronate may comprise a tetrahedral species.

4. Strongly basic organic nitrogen-containing compounds

The disclosure includes products obtainable by (having the characteristics of a product obtained by) reaction of a peptide boronic acid as defined above and a strong organic base. Two illustrative classes of organic base are described in sections 4A and 4B below. Particularly preferred are acid salts (in which one of the two boronic -OH groups is deprotonated). Most commonly, the salts contain a single type of organic counter-ion (disregarding trace contaminants) but the disclosure contemplates salts containing mixtures of organic counter-ions: in one sub-class, the different counter-ions all fall within the section 4A family described below or, as the case may be, in the section 2B family below; in another subclass, the salts comprise a mixture of organic counter-ions which are not all from the same family (4A or 4B).

Suitable organic bases include those with a pKb of 7 or more, e.g. 7.5 or more, for example in the region of 8 or more. Bases which are less lipophilic [e.g. have at least one polar functional group (e.g. 1, 2 or 3 such groups) for example hydroxy] are favoured; thus aminosugars are one favoured class of base.

*4A. Guanidines and their analogues*

The guanidino compound (guanidine) may in principle be any soluble and pharmaceutically acceptable compound having a guanidino or a substituted guanidino group, or a substituted or unsubstituted guanidine analogue. Suitable substituents include aryl (e.g. phenyl), alkyl or alkyl interrupted by an ether or thioether linkage and, in any event, typically contain from 1 to 6 and especially 1, 2, 3, or 4 carbon atoms, as in the case of methyl or ethyl. The guanidino group may have 1, 2, 3 or 4 substituent groups but more usually has 1 or 2 substituent groups, for instance on a terminal nitrogen. One class of guanidines is monoalkylated; another class is dialkylated. As guanidine analogues may be mentioned thioguanidines and 2-amino pyridines. Compounds having unsubstituted guanidino groups, for example guanidine and arginine, form one particular dass.

Salts containing mixtures of guanidines are contemplated by the disclosure.

A particular guanidino compound is L-arginine or an L-arginine analogue, for example D-arginine, or the D- or, preferably, L- isomers of homoarginine or agmatine [(4-aminobutyl) guanidine]. Less preferred arginine analogues are

NG-nitro-L-arginine methyl ester, for example, and constrained guanidine analogues, particularly 2-amino pyrimidines, for example 2,6-quinazolinediamines such as 5,6,7,8-tetrahydro-2,6-quinazolinediamine, for example. The guanidino compound may also be a peptide, for example a dipeptide, containing arginine; one such dipeptide is L-tyrosyl-L-arginine.

Some particular guanidino compounds are compounds of formula (VII):

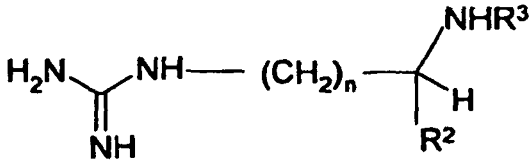

(VII)

where n is from 1 to 6 and for example at least 2, e.g. 3 or more, and in many instances no more than 5. Most particularly, n is 3, 4 or 5. $R^2$ is H or carboxylate or derivatised carboxylate, for example to form an ester (e.g. a $C_1$-$C_4$ alkyl ester) or amide. $R^3$ is H, $C_1$-$C_4$ alkyl or a residue of a natural or unnatural amino acid (e.g. tyrosine). The compounds of formula (IV) are usually of L-configuration. The compounds of formula (IV) are arginine (n=3; $R^2$=carboxyl; $R^3$=H) and arginine derivatives or analogues.

The disclosure includes products comprising salts of the formula (IX)

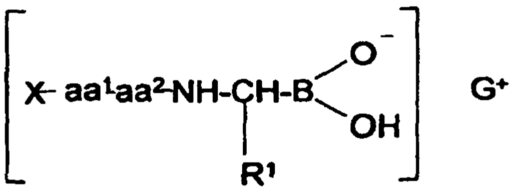

(IX)

where $aa^1$, $aa^2$, X and $R^1$ are as defined previously and $G^+$ is the protonated form of a pharmaceutically acceptable organic compound comprising a guanidino group or an analogue thereof, as well as salts in which both hydroxy groups of the boronate group are in salt form (preferably with another identical $G^+$ group) and mixtures of such salts. Also included are products wherein $R^1$ is replaced by another $R^9$ group.

### *4B. Strongly basic amines*

The disclosure includes products obtainable by (having the characteristics of a product obtained by) reaction of a peptide boronic acid as defined above and a strong organic base which is an amine. The amine may in principle be any soluble and pharmaceutically acceptable amine.

It is envisaged that a desirable class of amine includes those having polar functional groups in addition to a single amine group, as such compounds will be more hydrophilic and thus more soluble than others. In certain salts, the or each additional functional group is hydroxy. Some amines have 1, 2, 3, 4, 5 or 6 additional functional groups, espedally hydroxy groups. In one illustrative class of amines the ratio of (amino plus hydroxy groups):carbon atoms is from 1:2 to 1:1, the latter ratio being particularly preferred. These amines with one or more additional polar functional groups may be a hydrocarbon, especially an alkane, substituted by the amino group and the additional polar group(s). The amino group may be substituted or unsubstituted and, excluding amino substituents, the polar base may contain, for example, up to 10 carbon atoms; usually there are no less than three such carbon atoms, e.g. 4, 5 or 6. Aminosugars are included in this category of polar bases.

The disclosure includes products comprising salts of the formula (X)

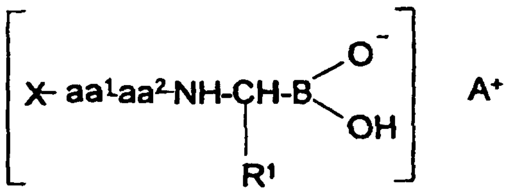

(X)

where $aa^1$, $aa^2$, X and $R^1$ are as defined previously and $A^+$ is the protonated form of a pharmaceutically acceptable amine, as well as salts in which both hydroxy groups of the boronate group are in salt form (preferably with another identical $A^+$ group) and mixtures of such salts. In one dass of such products, $A^+$ is the protonated form of an amine described in section 2B(i) below; in another class $A^+$ is the protonated form of an amine described in 2B(ii) below. Also included are products in which $R^1$ is replaced by another $R^9$ group.

Two illustrative dasses of amine base are described in sections 4B(i) and 4B(ii) below. Particularly preferred are acid salts (in which one of the two boronic -OH groups is deprotonated). Most commonly, the salts contain a single type of amine counter-ion (disregarding trace contaminants) but the disclosure contemplates salts containing mixtures of amine counter-ions; in one sub-class, the different counter-ions all fall within the sub-section 4B(i) family described below or, as the case may be, in the sub-section 4B(ii) family below; in another subclass, the salts comprise a mixture of organic counter-ions which are not all from the same family (4B(i) or 4B(ii)).

**4B(i) Aminosugars**

The identity of the aminosugar is not critical. Preferred aminosugars include ring-opened sugars, especially glucamines. Cyclic aminosugars are also envisaged as useful. One dass of the aminosugars is N-unsubstituted and another, preferred, dass is N-substituted by one or two N-substituents (e.g. one). Suitable substituents are hydrocarbyl groups, for example and without limitation containing from 1 to 12 carbon atoms; the substituents may comprise alkyl or aryl moieties or both. Exemplary substituents are $C_1$, $C_2$, $C_3$, $C_4$, $C_5$, $C_6$, $C_7$ and $C_8$ alkyl groups, in particular methyl and ethyl, of which methyl is illustrative. Data indicate that aminosugars, especially N-methyl-D-glucamine, are of surprisingly high solubility.

A most preferred aminosugar is N-methyl-D-glucamine:

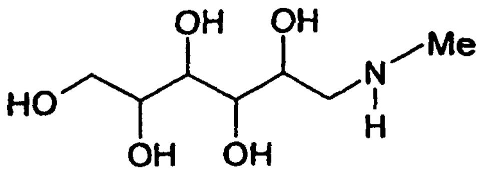

**4B(ii) Other amines**

Other suitable amines include amino acids (whether natural occurring or not) whose side chain is substituted by an amino group, especially lysine.

Some amines are compounds of formula (XI):

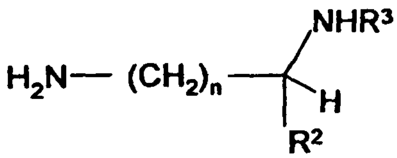

(XI)

where n, $R^2$ and $R^3$ are as defined in relation to formula (IV). The compounds of formula (VI) are usually of L-configuration. The compounds of formula (VI) are lysine (n=4; $R^2$=carboxyl; $R^3$=H) and lysine derivatives or analogues. A most preferred amine is L-tysine.

Other suitable amines are nitrogen-containing heterocycles. At least usually, such heterocyclic compounds are alicyclic; one class of the heterocyclic Compounds is N-substituted and another, preferred, class is N-unsubstituted. The heterocycles may contain 6 ring-forming atoms, as in the cases of piperidine, piperazine and morpholine. One class of amines includes N-containing heterocycles substituted by polar substituents, especially hydroxy, e.g. 1, 2 or 3 times.

The disclosure therefore includes amines other than aminosugars which have one or more (e.g. 1, 2, 3, 4, 5 or 6) polar substituents, especially hydroxy, in addition to one amine group. Such compounds may have a ratio of (amino plus hydroxy groups):carbon atoms of 1:2 to 1:1, the latter ratio being particularly preferred.

The disclosure indudes mixed salts, i.e. salts containing a mixture of boropeptide moieties and/or counterions but single salts are preferred.

The salts in solid form may contain a solvent, e.g. water. There are included a class of products in which the salts are essentially anhydrous. Also included is a class in which the salts are hydrates.

***Synthetic Methods I***

1. Peptide/Peptidomimetic Synthesis

The synthesis of boropeptides, including, for example, Cbz-D-Phe-Pro-BoroMpg-OPinacol is familiar to those skilled in the art and described in the prior art mentioned above, including Claeson et al (US 5574014 and others) and Kakkar et al (WO 92/07869 and family members including US 5648338). It is described also by Elgendy et al *Adv. Exp. Med. Biol. (USA)* 340:173-178, 1993; Claeson, G. et al *Biochem. J.* 290:309-312, 1993; Deadman et al *J. Enzyme Inhibition* 9:29-41, 1995, and by Deadman et al *J. Med. Chem.* 38:1511-1522, 1995.

Stereoselective synthesis with S or R configuration at the chiral B-terminal carbon may be conducted using established methodology (Elgendy et al *Tetrahedron. Lett.* 33:4209-4212, 1992; WO 92/07869 and family members induding US 5648338) using (+) or (-)- pinanediol as the chiral director (Matteson et al *J. Am. Chem. Soc.* 108:810-819, 1986; Matteson et al *Organometallics.* 3:1284-1288, 1984). Another approach is to resolve the requisite aminoboronate intermediate (e.g. Mpg-BOPinacol) to selectively obtain the desired (R)-isomer and couple it to the dipeptide moiety (e.g. Cbz-(R)-Phe-(S)-Pro, which is the same as Cbz-D-Phe-L-Pro) which will form the remainder of the molecule.

The boropeptides may be synthesised initially in the form of boronic acid esters, particularly esters with diols. Such diol esters may be converted to the peptide boronic acid as described next.

2. Ester to Acid Conversion

A peptide boronate ester such as Cbz-(R)-Phe-Pro-BomMpg-OPinacol may be hydrolysed to form the corresponding acid.

A novel technique for converting a diol ester of a peptide boronic acid of formula (I) into the acid comprises dissolving the diol ester in an ether and particularly a dialkyl ether, reacting the thus-dissolved diol with a diolamine, for example a dialkanolamine, to form a product precipitate, recovering the precipitate, dissolving it in a polar organic solvent and reacting the thus-dissolved product with an aqueous medium, e.g. an aqueous acid, to form the peptide boronic acid. The boronic acid may be recovered from the organic layer of the mixture resulting from the reaction, for example by removing the solvent, e.g. by evaporation under vacuum or distillation. The reaction between the diol ester and the diolamine may be carried out under reflux, for example.

The identity of the diol is not critical. As suitable diols may be mentioned aliphatic and aromatic compounds having hydroxy groups that are substituted on adjacent carbon atoms or on carbon atoms substituted by another carbon. That is to say, suitable diols include compounds having at least two hydroxy groups separated by at least two connecting carbon atoms in a chain or ring. One class of diols comprises hydrocarbons substituted by exactly two hydroxy groups. One such diol is pinacol and another is pinanediol; there may also be mentioned neopentylglycol, 1,2-ethanediol, 1,2-propanediol, 1,3-propanediol, 2,3-butanediol, 1,2-diisopropylethanediol, 5,6-decanediol and 1,2-dicydohexylethanediol.

The alkyl groups of the dialkyl ether preferably have 1, 2, 3 or 4 carbon atoms and the alkyl groups may be the same or different. An exemplary ether is diethyl ether.

The alkyl groups of the dialkanolamine preferably have 1, 2, 3 or 4 carbon atoms and the alkyl groups may be the same or different. An exemplary dialkanolamine is diethanolamine. The diethanolamine/boronic acid reaction product hydrolyses in water at room temperature and the rate of hydrolysis may be accelerated by adding acid or base.

The polar organic solvent is preferably $CHCl_3$. Other examples are polyhalogenated alkanes generally and ethyl acetate. In principle, any polar organic solvent is acceptable other than alcohols.

The aqueous acid is suitably a strong inorganic acid at a pH in the region of 1 such as hydrochloric acid, for example.

After reaction with the acid, the reaction mixture is suitably washed with, for example, $NH_4Cl$ or another mild base.

An example of a specific procedure is as follows

1. The pinacol or pinanediol ester of the selected peptide boronic acid is dissolved in diethylether.
2. Diethanolamine is added and the mixture is refluxed at 40 °C.
3. The precipitated product is removed (filtered), washed (usually several times) with diethyl ether or another polar organic solvent other than an alcohol, and dried (e.g. by evaporation under vacuum).
4. The dry product is dissolved in a polar organic solvent other than an alcohol, e.g. $CHCl_3$. Aqueous acid or base is added, e.g. hydrochloric acid (pH 1), and the mixture is stirred for e.g. approximately 1h at room temperature.
5. The organic layer is removed and washed with $NH_4Cl$ solution.
6. The organic solvent is distilled off and the residual solid product is dried.

The acid resulting from the above procedure may be converted to a salt of the acid with a pharmaceutically acceptable base, which salt may in turn be formulated into a pharmaceutical composition in parenteral dosage form.

3 Salt Synthesis

In general, the salts may be prepared by contacting the relevant peptide boronic acid with a strong base appropriate to form the desired salt. In the case of metal salts, the metal hydroxides are suitable bases (alternatively, metal carbonates might be used, for example), whilst sometimes it is more convenient to contact the acid with a relevant metal alkoxide (e.g. methoxide), for which purpose the corresponding alkanol is a suitable solvent. Salts with organic bases may be prepared by contacting the peptide boronic acid with the organic base itself. Illustrative salts are acid salts (one -BOH proton replaced) and, to make acid salts with a monovalent cation, the acid and the base are suitably reacted in substantially equimolar quantities. Generally stated, therefore, the usual acid:base molar ratio is substantially n:1, where n is the valency of the cation of the base.

In one procedure, a solution of the peptide boronic acid in a water-miscible organic solvent, for example acetonitrile or an alcohol (e.g. ethanol, methanol, a propanol, for example iso-propanol, or another alkanol), is combined with an aqueous solution of the base. The acid and the base are allowed to react and the salt is recovered. The reaction is typically carried out at ambient temperature (e.g. at a temperature of from 15 to 30°C, e.g. 15 to 25°C), but an elevated temperature may be used, for example up to the boiling point of the reaction mixture but more usually lower, e.g. a temperature of up to 40°C or 50°C. The reaction mixture may be allowed to stand or be agitated (usually stirred).

The time during which the acid and the base are allowed to react is not critical but it has been found desirable to maintain the reaction mixture for at least one hour. A period of from one to two hours is usually suitable but longer reaction times may be employed.

The salt may be recovered from the reaction mixture by any suitable method, for example evaporation or precipitation. Precipitation may be carried out by adding an excess of a miscible solvent in which the salt has limited solubility. In one preferred technique, the salt is recovered by evacuating the reaction mixture to dryness. The salt is preferably thereafter purified, for example by redissolving the salt before filtering the resulting solution and drying it, for example by evacuating it to dryness. The redissolution may be performed using water, e.g. distilled water. The salt may then be further purified, for example in order to remove residual water by further redissolution in a suitable solvent, which is advantageously ethyl acetate or THF followed by evaporating to dryness. The purification procedure may be carried out at ambient temperature (say, 15 to 30°C, e.g. 15 to 25°C), or at a modestly elevated temperature, such as e.g. a temperature not exceeding 40°C or 50°C; for example the salt may be dissolved in water and/or solvent by agitating with or without warming to, for example, 37°C.

Generally, preferred solvents for use in purifying the salts are ethyl acetate or THF, or perhaps another organic solvent

A general procedure for synthesising salts of Cbz-Phe-Pro-BoroMpg-OH is as follows:

Cbz-Phe-Pro-BoroMpg-OH (20.00g, 38.1mM) is dissolved in acetonitrile (200ml) with stirring at room temperature. To this solution is added the requisite base in solution in distilled water (190ml); the base is added as a 0.2M solution for a monovalent cation. The resultant dear solution is allowed to react for example by being left to stand or being agitated, for a usual period, in either case, of from one to two hours. The reaction is typically carried out at ambient temperature (e.g. 15-30°C, e.g. 15 to 25°C) but alternatively the temperature may be elevated (e.g. up to 30°C, 40°C or 50°C). The reaction mixture is then evacuated to dryness under vacuum with its temperature not exceeding 37°C, typically to yield a white brittle solid or an oil/tacky liquid. The oil/tacky liquid is redissolved in the minimum amount of distilled water necessary (200ml to 4L), typically with warming (e.g. to 30-40°C), usually for up to 2 hours. The solution is filtered, suitably through filter paper, and evacuated to dryness, again with the temperature of the solution not exceeding 37°C, or freeze dried. The resultant product is dried under vacuum overnight to normally yield a white bridte solid. If the product is present as an oil or tacky solid then it is dissolved in ethyl acetate and evacuated to dryness to produce the product as a white solid. The white solid is typically a coarse, amorphous powder.

In variations of the aforegoing general procedure, the acetonitrile is replaced by another water-miscible organic solvent, notably an alcohol, as discussed above, especially ethanol, methanol, iso-propanol or another propanol.

Where a boronic acid salt is less soluble in a selected reaction medium for salt formation such that its direct preparation from the corresponding acid and base is inconvenient, the less soluble salt may be prepared from a salt more soluble in the reaction medium.

4. Separation of Stereoisomers

The stereoisomers of a peptide boronic ester or a synthetic intermediate aminoboronate may be resolved in, for example, any known way. In particular, stereoisomers of boronic esters may be resolved by HPLC.

***Synthetic Methods II - Stability* and *Purity of the Compounds***

Existing publications teach that organoboronic acids are degraded by oxidation of the C-B bond. See for example Wu et al (see above). Earlier work on the salts of TRI 50c confirmed that these salts and/or intermediates in their preparation are slightly unstable, to the extent that the salts were found to contain a boron-free impurity, designated impurity I, which was evidently generated by C-B bond deavage. The salts as a class are significantly more stable to such degradation than the free acid.

These earlier TRI 50c salts were made via the general methods described in Examples 5 and 9 of this specification. Impurity I has the following structure:

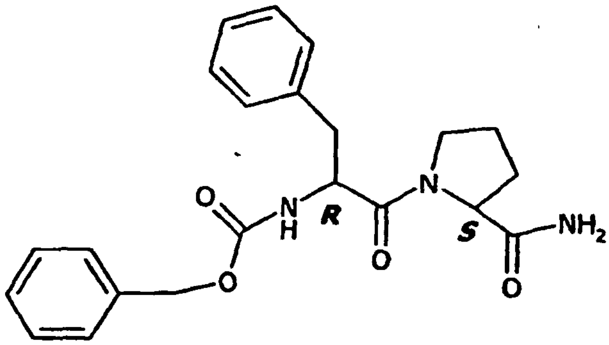

For example, an HPLC chromatogram, prepared using a reverse phase method more particularly described in Example 34, produced the following data for the monosodium salt of TRI 50c, made by following the procedures of Examples 5 and 9 herein:

| | Name | RT (min) | Area | Height | Amount | Units | % Area |
|---|---|---|---|---|---|---|---|
| 1 | Benzaldehyde | 6.145 | 2487 | 224 | | | 0.39 |
| 2 | Impurity I | 11.022 | 6379 | 539 | | | 1.00 |
| 3 | TRI50c | 11.679 | 628872 | 51108 | 946,063 | ug/mL | 98.61 |

Attempts to purify salts contaminated with Impurity I were not successful and it appeared that, for example, Impurity I was generated from the salts in HPLC columns.

Relative chiral purity of salts made following the general procedure of Examples 5 and 9 was achieved by resolving by HPLC the pinacol ester of TRI 50c, designated TRI 50b, and converting the thus-resolved TRI 50b into the salts. Such an HPLC procedure is not acceptable for normal commercial drug production.

It has further been found that the prior art synthesis summarised earlier under the heading "Aminoboronate Procedure" results, when applied to the synthesis of TRI 50c or an ester thereof, in formation of an impurity designated Impurity IV:

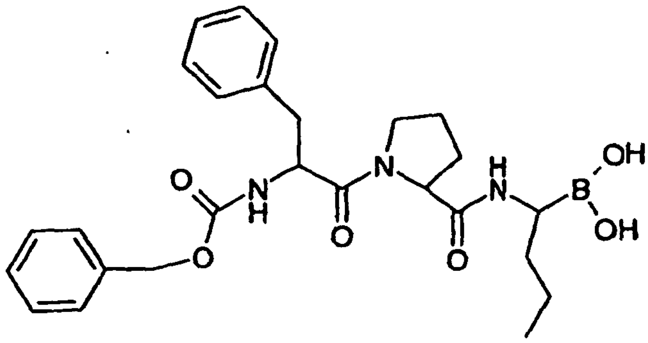

Attempts to separate Impurity IV from TRI 50c have not succeeded. The same applies to TRI 50c salts and esters and the corresponding salts and esters of Impurity IV. No purification technique which has been tried can prevent the presence of Impurity IV if said prior art synthesis is used.

***Synthetic Method II - The Methods***

Amongst other things, the present disclosure addresses the problems of controlling C-B bond cleavage in organoboronic compounds as well as providing chirally purified salts of TRI 50c and other organoboronic acids on a commercial scale. In this regard, it has been found that C-B bonds seem to be cleaved by a non-oxidative mechanism which occurs in the presence of many solvents, induding water and e.g. aqueous acids and bases, amongst others.

It has also been found that chirally-selective precipitation can be used to recover organoboronic acids in high purity.

Thus C-B bond cleavage (and hence in particular generation of Impurity I) may be controlled by:

- Selection of acetonitrile as a solvent, where a solvent is required in processing and acetonitrile has the necessary solvation power; in particular acetonitrile is selected in process where a polar solvent is desirable or necessary.
- Avoiding excessive contact with water.

In terms of TRI 50c salt production, therefore, the disclosure indudes processes comprising one, two or three of the following features:

(i) resolution of the (R,S,S) and (R,S,R) epimers of TRI 50c by chirally selective precipitation using diethanolamine and conveniently, but not necessarily, using as starting material TRI 50c in the form of an ester, for example the pinacol ester;

(ii) control of the duration and/or conditions of hydrolysis of TRI 50c diethanolamine ester, for example as obtained by such predpitation, to control C-B bond breakage;

(iii) use of acetonitrile as solvent for TRI 50c, for example as obtained by such hydrolysis, for the purposes of reacting the TRI 50c with a base to form the salt. Another favourable solvent can be tetrahydrofuran.

As an optional, or even stand-alone, fourth feature, TRI 50c salts may be dried by azeodrying using acetonitrile.

It is considered that C-B bond cleavage may occur by a nudeophilic mechanism, and the disclosure therefore includes methods in which opportunities for nudeophilic attack are minimised.

The above four features, or any one, two or three of them, may be applied to the manufacture and processing of other boronic compounds, particularly acids of formula (I) and their derivatives (e.g. esters and salts).

Diethanolamine may be used therefore to resolve by selective precipitation the diastereomers of boronic acids of formula (Ia)

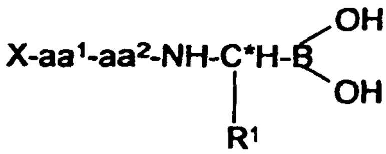

(Ia)

The starting material may be an acid (Ia) or a derivative thereof capable of forming a diethanolamine ester of the boronic acid. The precipitation selects acids having a chiral centre C* of (R) configuration as precipitate. The precipitate is recovered and converted to the corresponding boronic acid or a salt thereof. The salt is made into a parenteral pharmaceutical formulation.

For optimised chiral purity and yield, the diethanolamine may be used in an amount of about 1.25 ± 0.1 equivalents based on initial equivalents of boronic acid having a chiral centre C* of (R) configuration.

The initial boronic acid or acid derivative may for example comprise from 50% to 60% molecules having chiral centre C* of (R)-configuration and from 40% to 50% molecules having chiral centre C* of (S)-configuration.

The method opens the way to commercialisation of the boronic acids (I) and their derivatives, particularly salts, as pharmaceuticals. Commercial scale products and activities using the boronic acids (I) and their derivatives are therefore provided.

Useful for the manufacture of products of the invention, therefore, is a process for separating diastereomers of a boronic acid of formula (I), comprising:

combining in diethylether solution (A) a boronic species selected from the boronic acid (I) and its esters, the boronic species including molecules having a chiral centre C* of (R) configuration and molecules having a chiral centre C* of (S) configuration, and (B) diethanolamine, the diethanolamine being in an amount of about 1.25 ± 0.1 equivalents based on the boronic species in which the chiral centre C* is of (R) configuration, and mixing to form a mixture;
causing or allowing the boronic species and the diethanolamine to react until a precipitate forms; and
recovering the precipitate.

When the starting material is an ester, it may be an ester of the boronic acid with an alcohol selected from the group consisting of alcohols whose sole potential electron donor heteroatoms are oxygens which, in the boronic ester, correspond to the oxygens of the ester functional group;

In some methods, the diethanolamine is in an amount of from 1.2 to 1.3 equivalents based on the boronic species in which chiral centre C* is of (R) configuration.

There are included processes in which the boronate species is an ester of the boronic acid and a diol, in particular a diol which is not sterically hindered. As exemplary diols may be mentioned pinacol, neopentylglycol, 1,2-ethanediol, 1,2-propanediol, 1,3-propanediol, 2,3-butanediol, 1,2-diisopropylethanediol, or 5,6-decanediol. A particular diol is pinacol.

The boronic species and the diethanolamine may be caused to react by heating the mixture to an elevated temperature, for example the mixture may be refluxed, e.g. for at least 10 hours.

The precipitate may be recovered by filtration. The recovered precipitate may be washed with diethylether. The recovered precipitate, after washing if such takes places, may be dissolved in a solvent selected from $CH_2Cl_2$ and $CHCl_3$ and reprecipitated by combining the resulting solution with diethylether. A particular solvent is $CH_2Cl_2$.

The recovered precipitate may be converted to the acid of formula (I), suitably by hydrolysis, for example by dissolving the precipitate in an organic solvent selected from e.g. halohydrocarbons and combinations thereof, agitating the resulting solution with an aqueous liquid, e.g. an aqueous acid having a pH of below 3, whereby the dissolved precipitate is converted to the formula (I) acid, and recovering the formula (I) acid by evaporation. The organic solvent may be $CH_2Cl_2$ or $CHCl_3$. A particular solvent is $CH_2Cl_2$. In some processes, organic solvent is further evaporated from the recovered formula (I) acid.

Also useful for the manufacture of products of the invention are methods in which an ester of a boronic acid (I), particularly a diethanolamine ester, is hydrolysed in a manner which controls C-B bond cleavage. In particular, this involves limiting the period of hydrolysis at the selected temperature. In the case of diethanolamine ester hydrolysis, the hydrolysis is suitably carried out at room temperature, or less, for a period not exceeding about 30 minutes, e.g. not exceeding about 20 minutes, and optimally of about 20 minutes.

Thus the recovered precipitate referred to in the last paragraph but one may be hydrolysed using an aqueous acid, particularly 2% hydrochloric acid or another mineral acid of similar pH, for no more than about 30 minutes at about room temperature, or less. Suitably, the precipitate is dissolved in a non-nucleophilic organic solvent (e.g. a halohydrocarbon or halohydrocarbon mixture for example $CH_2Cl_2$) and the resulting solution is contacted with the aqueous acid

for a period as previously described. The precipitate is thereby hydrolysed to form the free acid of formula (I), which remains in the organic solvent. The organic solvent may be separated from the aqueous medium and then evaporated to obtain solid acid of formula I.

In some processes a formula (I) acid, for example obtained as described in the preceding paragraph, is dried. In a class of processes, the formula (I) acid is dried when it is in the organic solvent by contacting the solvent with a hygroscopic solid.

Included are processes in which the formula (I) acid, when in the organic solvent, is washed with an aqueous ammonium salt

Chirally purified boronic acid may be converted to a pharmaceutically acceptable base addition salt thereof, in particular by dissolving the acid in acetonitrile, combining the resultant solution with an aqueous solution or suspension of a pharmaceutically acceptable base, and causing or allowing the base and the acid to react, then evaporating to dryness to obtain an evaporation residue. The step of causing or allowing the acid and the base to react may comprise agitating the combination of the acetonitrile solution of the acid and the aqueous solution or suspension of the base at a temperature of not more than 35°C and often of not more than 30°C, e.g. not more than 25°C; an optimal temperature is room temperature, in which case a reaction time of about 2 hours might be appropriate. The process may further comprise:

(i) redissolving the evaporation residue in acetonitrile and evaporating the resulting solution to dryness; and
(ii) repeating step (i) as often as necessary to obtain a dry evaporation residue.

In some processes the dry evaporation residue is dissolved in acetonitrile or tetrahydrofuran to form a solution, and the solution is combined with (e.g. slowly added to, at a rate sufficiently slow to avoid lump formation) a 3:1 to 1:3 v/v mixture of diethylether and an aliphatic or cycloaliphatic solvent to form a precipitate, said solution being added to the diethylether/(cyclo)aliphatic solvent mixture in a ratio (solution:mixture) of from 1:5 to 1:15 v/v. The precipitate is recovered and some or substantially all remaining solvent is removed from the recovered precipitate whilst maintaining the temperature at no more than 35°C, e.g. is removed under reduced pressure. Included are processes in which the temperature at the start of the drying process is about 10°C and is increased during the process to 35°C. The aliphatic or cydoaliphatic solvent may have 6, 7 or 8 carbon atoms; the solvent may be an alkane, for example an n-alkane, e.g. n-heptane. Some reactions may be carried out at ambient temperature, which may e.g. be 15-30°C, e.g. 20-30°C; sometimes ambient temperature may be room temperature.

The salts produced by the invention may contain a trace amount of the aliphatic or cycloaliphatic solvent, e.g. an amount of less than 0.1%, particularly less than 0.01%, for example an amount of about 0.005%.

In the process for making the salt, the base may comprise a cation of valency n and be used in a stoichiometry (boronic acid:base) of about n:1. In particular processes, the base is an alkali metal or alkaline earth metal base, for example an alkali metal hydroxide or an alkaline earth metal hydroxide. As one base may be mentioned sodium hydroxide. As another base may be mentioned calcium hydroxide. The disclosure includes processes in which the base is sodium hydroxide and the dry evaporation residue is dissolved in acetonitrile. The disclosure includes processes in which the base is calcium hydroxide and the dry evaporation residue is dissolved in tetrahydrofuran.

The disclosure is not limited as to the method by which the boronic acids of Formula (I) are obtained (for example as an ester thereof). However, in one class of subject matter, the Formula (I) acid has an $R^1$ group of the formula $-(CH_2)_S-O-R^3$ in which $R^3$ is methyl or ethyl and s is independently 2, 3 or 4, and the Formula (I) acid is prepared via an intermediate of Formula (XXV):

$$(HO)_2B-(CH_2)_S-O-R^3 \qquad (XXV),$$

which intermediate is made by reaction between a borate ester and a suitable 1-metalloalkoxyalkane.

The Formula (XXV) intermediates may be made by reacting a 1-metalloalkoxyalkane, where the alkoxyalkane is of the formula $-(CH_2)_S-O-R^3$, with a borate ester to form a compound of Formula (XXV).

It will be appreciated that the above method provides a general procedure for making alkoxyalkylboronic acids, which may be presented by the formula $R^Z-O-R^Y-B(OH)_2$, Such alkoxyalkylboronic acids may be converted to aminoboronates, and the aminoboronates may be derivatised at their amino group to form an amide bond linked to another moiety. In other words, the aminoboronates may be converted to boropeptides. The method will now be described further with non-limiting reference to compounds of Formula (XXV).

The starting materials for the reaction may be a metalloalkoxyalkane, e.g. a Grignard reagent, obtainable from 1-haloalkoxyalkane of the formula $Hal-(CH_2)_S-O-R^3$ (where Hal is a halogen) and a borate ester. The metal is in particular magnesium. Another metal is lithium, in which case the metallo reagent may be prepared by reacting the 1-hafoalkoxyalkane with butyl lithium. Where the method includes preparation of the metallo reagent from the haloalkoxyalkane, the haloalkoxyalkane may be a chloroalkoxyalkane; the corresponding bromo compounds may also be used. To make a

Grignard reagent, magnesium may be reacted with the haloalkoxyalkane.

Suitable borate esters are esters of mono- and di-functional alcohols (e.g. of EtOH, MeOH, BuOH, pinacol, glycol, pinanediol etc). For example, the ester may be of the formula $B(OR^a)(OR^b)(OR^c)$ where $R^a$, $R^b$ and $R^c$ are $C_1$-$C_4$ alkyl and may be the same as each other.

An exemplary procedure for making a Formula (XXV) intermediate, illustrated with reference to methoxypropane as the alkoxyalkane species, is:

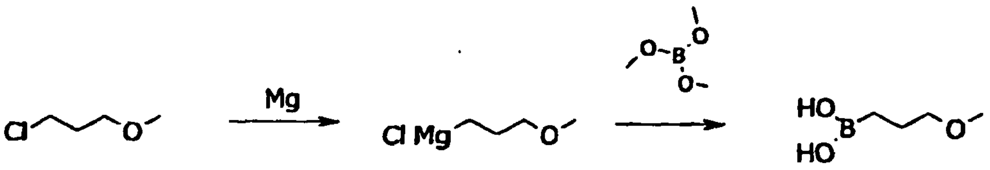


Z-DIPIN A

The reactions are suitably carried out in an organic solvent, e.g. THF.
The above-described procedure for making alkoxyalkylboronic acids avoids generation of Impurity IV (see above), or its analogues in those cases where the end product is not TRI 50c or a derivative (salt, ester etc) thereof. The procedure therefore provides a unique route to making TRI 50c, its esters and salts, uncontaminated by Impurity IV, and for making other aminoboronic acids which are substituted α- to the boron by an alkoxyalkyl group and are uncontaminated by impurities analogous to Impurity IV.

An alkoxyalkylboronic acid, i.e. a compound which may be represented by the formula $R^Z$-O-$R^Y$-$B(OH)_2$, may be converted to an aminoboronic compound, for example a boropeptide, by any suitable procedure, e.g. one known in the art. A reaction scheme for making alkoxyalkylboronic acids into aminoboronates, and for converting aminoboronates into peptide boronates is illustrated with reference to synthesis of TRI 50c at the start of the Examples of this specification. The reaction scheme may be modified as desired, e.g.: diethanolamine precipitation and subsequent steps may be omitted, and/or reagent substitutions may be made. For example, pinacol may be replaced by another diol. LDA is a non-nucleophilic strong base and may be replaced by another such base. Other examples include, but are not limited to, lithium diisopropylamide, lithium 2,2,6,6-tetramethylpiperidine, 1-lithium 4-methylpiperazide, 1,4-dilithium piperazide, lithium bis(trimethylsilyl) amide, sodium bis(brimethylsilyl)amide, potassium bis(trimethylsityl)amide, isopropyl magnesium chloride, phenyl magnesium chloride, lithium diethylamide, and potassium tert-butoxide. The reactions may be carried out in any suitable solvent: where n-heptane is used in the Examples, it may be replaced by another inert non-polar solvent, e.g. another aliphatic or cycloaliphatic solvent, for example an alkane, e.g. an n-alkane.

Thus, the disclosure includes a process for making an aminoboronate of Formula (XXI)

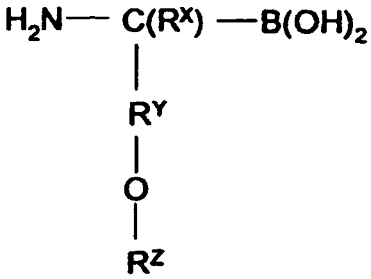

(XXI)

wherein
$R^X$ is H;
$R^Y$ is alkylene;
$R^Z$ is alkyl; and
$R^Y$-O-$R^Z$ is an $R^9$ group of formula (I), e.g. an $R^1$ group of formula (II),
the process comprising reacting a 1-metalloalkoxyalkane with a borate ester to form a boronic acid of the formula $R^Z$-O-$R^Y$-$B(OH)_2$, esterifying the acid, contacting the esterified acid with $CH_2Cl_2$ and $ZnCl_2$ in the presence of a strong base, contacting the resultant produce with UHMDS and in tum contacting the resultant product with hydrogen chloride.

The product is free of contaminant of Formula (XXII):

$H_2N\text{-}C(R^X)(R^Y)\text{-}B(OH)_2$ (XXII).

The aminoboronate (XXI) may be reacted with an optionally protected dipeptide corresponding to moiety YCO- of formula (I) to form a peptide boronate of formula (I). In general terms, therefore, the disdosure includes peptidoboronic acids of Formula (XXIII):

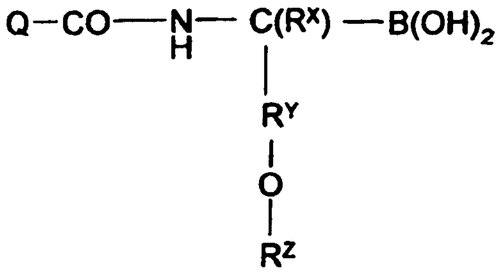

(XXIII)

wherein
Q-CO is Y-CO; and
$R^X$, $R^Y$ and $R^Z$ are as defined with reference to formula (XXI),
which organoboronic acid is free of an impurity of Formula (XXIV):

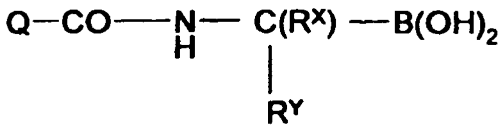

(XXIV).

The disclosure further includes derivatives of Formula (XXIII) acids (e.g. acid or base addition salts, esters) which are free of Formula (XXIV) impurity and derivatives thereof.

The exact identity of $R^Y$ and $R^Z$ is dependent on the identity of the end product, and not part of the process or its benefits.

It will be appreciated from the aforegoing that the above described methods may be used in the manufacture of organoboronic acids salts as desaibed. It is not necessary for sequential steps to be carried out as one operation or at the same site: they may be performed in this way or different processes (different parts of the overall synthesis) may be distributed in time and/or space. Particular end product salts are monosodium, monolithium, hemicalcium and hemimagnesium salts, for example of TRI 50c.

Generally, the reactions may suitably be carried out with a non-nucleophilic solvent. Where a nucleophilic solvent is present, minimum contact is preferred, for example in the case of hydrolysis of diethanolamine esters.

### *The High Purity Products*

The "high purity products" useful in the products and uses of the invention include salts obtainable by (having the characteristics of a product obtained by) the disclosed methods.

Particularly useful in the products and uses of the products of the invention are base addition salts of a boronic acid of formula (I) having the chiral purity of such salt when prepared by a method described herein. Also useful are base addition salts of a boronic acid of formula (I) having the purity of such salt when prepared by a method described herein.

The identities of such salts will be apparent from the preceding description and the following examples. In addition, products of the disclosure are described in the claims. Of particular note are the data in Example 43, indicating that the processes of the invention can remarkably achieve end product salts free of impurities detectable by HPLC. In other instances, the salts are substantially free of impurities, e.g. at least 98% pure, more usually at least 99% pure, e.g. at least 99.5% pure, in terms of reverse phase (RP) HPLC percentage peak area. Salts may at least 99.3%, 99.4%, 99.5% 99.6%, 99.7%, 99.8% or 99.9% pure, in terms of reverse phase (RP) HPLC percentage peak area. Suitable RP

HPLC procedures comply with reference 1 and/or reference 2 and/or reference 3 of Example 43. Included also are products at least substantially free of Impurity I and analogues, products free of Impurity IV and analogues, and products containing small traces of non-polar solvent, e.g. n-heptane. The trace amount of non-polar solvent may be less than 0.2%, 0.1%, 0.05%, 0.01% or 0.005% as determined by GC-headspace chromatography.

Included also are salts containing less than 410 ppm acetonitrile.

Some salts contain impurities of less than 10,000 ppm, 5000 ppm, 1000 ppm, or 500 ppm.

***Use of the Products of the Disclosure***

The salts of the disdosure are thrombin inhibitors. They are therefore useful for inhibiting thrombin. There are therefore provided compounds which have potential for controlling haemostasis and especially for inhibiting coagulation, for example in the treatment or prevention of secondary events after myocardial infarction. The medical use of the compounds may be prophylactic (including to treat thrombosis as well as to prevent occurrence of thrombosis) as well as therapeutic (induding to prevent re-occurrence of thrombosis or secondary thrombotic events).

The salts may be employed when an anti-thrombogenic agent is needed. Further, it has been found that the salts, including those of boronic acids of Formula (III), are beneficial in that the class is useful for treating arterial thrombosis by therapy or prophylaxis. The disclosed salts are thus indicated in the treatment or prophylaxis of thrombosis and hypercoagulability in blood and tissues of animals including man. The term "thrombosis" Includes *inter alia* atrophic thrombosis, arterial thrombosis, cardiac thrombosis, coronary thrombosis, creeping thrombosis, infective thrombosis, mesenteric thrombosis, placental thrombosis, propagating thrombosis, traumatic thrombosis and venous thrombosis.

It is known that hypercoagulability may lead to thromboembolic diseases.

Examples of venous thromboembolism which may be treated or prevented with compounds of the disclosure include obstruction of a vein, obstruction of a lung artery (pulmonary embolism), deep vein thrombosis, thrombosis associated with cancer and cancer chemotherapy, thrombosis inherited with thrombophilic diseases such as Protein C deficiency, Protein S deficiency, antithrombin III deficiency, and Factor V Leiden, and thrombosis resulting from acquired thrombophilic disorders such as systemic lupus erythematosus (inflammatory connective tissue disease). Also with regard to venous thromboembolism, compounds of the disdosure are useful for maintaining patency of indwelling catheters. Examples of cardiogenic thromboembolism which may be treated or prevented with compounds of the disclosure indude thromboembolic stroke (detached thrombus causing neurological affliction related to impaired cerebral blood supply), cardiogenic thromboembolism associated with atrial fibrillation (rapid, irregular twitching of upper heart chamber muscular fibrils), cardiogenic thromboembolism associated with prosthetic heart valves such as mechanical heart valves, and cardiogenic thromboembolism associated with heart disease.

Examples of conditions involving arterial thrombosis include unstable angina (severe constrictive pain in chest of coronary origin), myocardial infarction (heart muscle cell death resulting from insufficient blood supply), ischemic heart disease (local ischemia due to obstruction (such as by arterial narrowing) of blood supply), reocclusion during or after percutaneous transluminal coronary angioplasty, restenosis after percutaneous transluminal coronary angioplasty, occlusion of coronary artery bypass grafts, and occlusive cerebrovascular disease. Also with regard to arterio-venous (mixed) thrombosis, anti-thrombotic compounds of the disclosure are useful for maintaining patency in arteriovenous shunts.

Other conditions associated with hypercoagulability and thromboembolic diseases which may be mentioned are inherited or acquired deficiencies in heparin cofactor II, circulating antiphospholipid antibodies (Lupus anticoagulant), homocysteinemia, heparin induced thrombocytopenia and defects in fibrinolysis.

Particular uses which may be mentioned include the therapeutic and/or prophylactic treatment of venous thrombosis and pulmonary embolism. Preferred indications envisaged for the products of the disclosure (notably the salts of TRI 50c) include:

- Prevention of venous thromboembolic events (e.g. deep vein thrombosis and/or pulmonary embolism). Examples include patients undergoing orthopaedic surgery such as total hip replacement, total knee replacement, major hip or knee surgery; patients undergoing general surgery at high risk for thrombosis, such as abdominal or pelvic surgery for cancer; and in patients bedridden for more than 3 days and with acute cardiac failure, acute respiratory failure, infection.
- Prevention of thrombosis in the haemodialysis circuit in patients, in patients with end stage renal disease.
- Prevention of cardiovascular events (death, myocardial infarction, etc) in patients with end stage renal disease, whether or not requiring haemodialysis sessions.
- Prevention of venous thrombo-embolic events in patients receiving chemotherapy through an indwelling catheter.
- Prevention of thromboembolic events in patients undergoing lower limb arterial reconstructive procedures (bypass, endarteriectomy, transluminal angioplasty, etc).
- Treatment of venous thromboembolic events.

- Prevention of cardiovascular events in acute coronary syndromes (e.g. unstable angina, non Q wave myocardial ischaemia/infarction), in combination with another cardiovascular agent, for example aspirin (acetylsalicylic acid; aspirin is a registered trade mark in Germany), thrombolytics (see below for examples), antiplatelet agents (see below for examples).
- Treatment of patients with acute myocardial infarction in combination with acetylsalicylic acid, thrombolytics (see below for examples).

The thrombin inhibitors of the disclosure are thus indicated both in the therapeutic and/or prophylactic treatment of all the aforesaid disorders.

In one method, the products of the disclosure are used for the treatment of patients by haemodialysis, by providing the product in the dialysis solution, as described in relation to other thrombin inhibitors in WO 00/41715. The disclosure therefore includes dialysing solutions and dialysing concentrates which comprise a product of the disclosure, as well as a method of treatment by dialysis of a patient in need of such treatment, which method comprises the use of a dialysing solution including a low molecular weight thrombin inhibitor. Also included is the use of an anti-thrombotic product of the disclosure for the manufacture of a medicament for the treatment by dialysis of a patient, in which the anti-thrombotic product of the disclosure is provided in the dialysing solution.

In another method, the products of the disclosure are used to combat undesirable cell proliferation, as described in relation to other thrombin inhibitors in WO 01/41796. The undesirable cell proliferation is typically undesirable hyperplastic cell proliferation, for example proliferation of smooth muscle cells, especially vascular smooth muscle cells. The products of the disclosure particularly find application in the treatment of intimal hyperplasia, one component of which is proliferation of smooth muscle cells. Restenosis can be considered to be due to neointimal hyperplasia; accordingly intimal hyperplasia in the context of the disclosure includes restenosis.

The products of the disclosure are also contemplated for the treatment of ischemic disorders. More particularly, they may be used in the treatment (whether therapeutic or prophylactic) of an ischemic disorder in a patient having, or at risk of, non-valvular atrial fibrillation (NVAF) as described in rotation to other thrombin inhibitors in WO 02/36157. Ischemic disorders are conditions whose results include a restriction in blood flow to a part of the body. The term will be understood to include thrombosis and hypercoagulability in blood, tissues and/or organs. Particular uses that may be mentioned Include the prevention and/or treatment of ischemic heart disease, myocardial infarction, systemic embolic events in e.g. the kidneys or spteen, and more particularly of cerebral ischemia, including cerebral thrombosis, cerebral embolism and/or cerebral ischemia associated with non-cerebral thrombosis or embolism (in other words the treatment (whether therapeutic or prophylactic) of thrombotic or ischemic stroke and of transient ischemic attack), particularly in patients with, or at risk of, NVAF.

The products of the disclosure are also contemplated for the treatment of rheumatic/arthritic disorders, as described in relation to other thrombin inhibitors in WO 03/007984. Thus, the products of the disclosure may be used in the treatment of chronic arthritis, rheumatoid arthritis, osteoarthritis or ankylosing spondylitis

Moreover, the products of the disclosure are expected to have utility in prophylaxis of re-occlusion (i.e. thrombosis) after thrombolysis, percutaneous trans-luminal angioplasty (PTA) and coronary bypass operations; the prevention of re-thrombosis after microsurgery and vascular surgery in general. Further indications include the therapeutic and/or prophylactic treatment of disseminated intravascular coagulation caused by bacteria, multiple trauma, intoxication or any other mechanism; anticoagulant treatment when blood is in contact with foreign surfaces in the body such as vascular grafts, vascular stents, vascular catheters, mechanical and biological prosthetic valves or any other medical device; and anticoagulant treatment when blood is in contact with medical devices outside the body such as during cardiovascular surgery using a heart-lung machine or in haemodialysis.

The products of the disclosure are further indicated in the treatment of conditions where there is an undesirable excess of thrombin without signs of hypercoagulability, for example in neurodegenerative diseases such as Alzheimer's disease. In addition to its effects on the coagulation process, thrombin is known to activate a large number of cells (such as neutrophils, fibroblasts, endothelial cells and smooth muscle cells). Therefore, the compounds of the disclosure may also be useful for the therapeutic and/or prophylactic treatment of idiopathic and adult respiratory distress syndrome, pulmonary fibrosis following treatment with radiation or chemotherapy, septic shock, septicaemia, inflammatory responses, which indude, but are not limited to, edema, acute or chronic atherosclerosis such as coronary arterial disease, cerebral arterial disease, peripheral arterial disease, reperfusion damage, and restenosis after percutaneous transluminal angioplasty (PTA).

The salts may also be useful in the treatment of pancreatitis.

The salts described herein are further considered to be useful for inhibiting platelet procoagulant activity. The disclosure provides a method for inhibiting platelet pro-magulant activity by administering a salt of a boronic acid described herein to a mammal at risk of, or suffering from, arterial thrombosis, particularly a human patient. Also provided is the use of such salts for the manufacture of medicaments for inhibiting platelet procoagulant activity.

The use of products of the disdosure as inhibitors of platelet pro-coagulant activity is predicated on the observation that the boronic acids described herein are indicated to be effective at inhibiting arterial thrombosis as well as venous thrombosis. Indications involving arterial thrombosis include acute coronary syndromes (especially myocardial infarction and unstable angina), cerebrovascular thrombosis and peripheral arterial occlusion and arterial thrombosis occurring as a result of atrial fibrillation, valvular heart disease, arterio-venous shunts, indwelling catheters or coronary stents. Accordingly, in another aspect there is provided a method of treating a disease or condition selected from this group of indications, comprising administering to a mammal, especially a human patient, a salt of the disclosure. The disclosure includes products for use in an arterial environment, e.g. a coronary stent or other arterial implant, having a coating which comprises a salt according to the disclosure.

The salts of the disclosure may be used prophylactically to treat an individual believed to be at risk of suffering from arterial thrombosis or a condition or disease involving arterial thrombosis or therapeutically (including to prevent re-occurrence of thrombosis or secondary thrombotic events).

There is therefore included the use of selective thrombin inhibitors (organoboronic acid salts) described herein for treatment of the above disorders by prophylaxis or therapy as well as their use in pharmaceutical formulations and the manufacture of pharmaceutical formulations.

### *Administration and Pharmaceutical Formulations*

The salts may be administered to a host, for example, in the case where the drug has anti-thrombogenic activity, to obtain an anti-thrombogenic effect. In the case of larger animals, such as humans, the compounds may be administered alone or in combination with pharmaceutically acceptable diluents, exdpients or carriers. The term "pharmaceutically acceptable includes acceptability for both human and veterinary purposes, of which acceptability for human pharmaceutical use is preferred.

The salts of the disclosure may be combined and/or co-administered with any cardiovascular treatment agent There are large numbers of cardiovascular treatment agents available in commercial use, in clinical evaluation and in pre-clinical development, which could be selected for use with a product of the disclosure for the prevention of cardiovascular disorders by combination drug therapy. Such agent can be one or more agents selected from, but not limited to several major categories, namely, a lipid-lowering drug, including an IBAT (ileal $Na^+$/bile acid cotransporter) inhibitor, a fibrate, niacin, a statin, a CETP (cholesteryl ester transfer protein) inhibitor, and a bile acid sequestrant, an anti-oxidant, including vitamin E and probucol, a IIb/IIIa antagonist (e.g. abciximab, eptifibatide, tirofiban), an aldosterone inhibitor (e.g. spirolactone and epoxymexrenone), an adenosine A2 receptor antagonist (e.g. losartan), an adenosine A3 receptor agonist, a beta-blocker, acetylsalicylic acid, a loop diuretic and an ACE (angiotensin converting enzyme) inhibitor.

The salts of the disclosure may be combined and/or co-administered with any antithrombotic agent with a different mechanism of action, such as the antiplatelet agents acetylsalicylic acid, tidopidine, dopidogrel, thromboxane receptor and/or synthetase inhibitors, prostacyclin mimetics and phosphodiesterase inhibitors and ADP-receptor ($P_2T$) antagonists.

The products of the disclosure may further be combined and/or co-administered with thrombolytics such as tissue plasminogen activator (natural, recombinant or modified), streptokinase, urokinase, prourokinase, anisoylated plasminogen-streptokinase activator complex (APSAC), animal salivary gland plasminogen activators, and the like, in the treatment of thrombotic diseases, in particular myocardial infarction.

The salts of the disclosure may be combined and/or co-administered with a cardioprotectant, for example an adenosine A1 or A3 receptor agonist.

There is also provided a method for treating an inflammatory disease in a patient that comprises treating the patient with a product of the disclosure and an NSAID, e.g., a COX-2 inhibitor. Such diseases include but are not limited to nephritis, systemic lupus, erythematosus, rheumatoid arthritis, glomerulonephritis, vasculitis and sarmidosis. Accordingly, the anti-thrombotic salts of the disclosure may be combined and/or co-administered with an NSAID.

Typically, therefore, the salts described herein may be administered to a host to obtain a thrombin-inhibitory effect, or in any other thrombin-inhibitory or anti-thrombotic context mentioned herein.

Actual dosage levels of active ingredients in the pharmaceutical compositions of this disclosure may be varied so as to obtain an amount of the active compound(s) that is effective to achieve the desired therapeutic response for a particular patient, composition, and mode of administration (referred to herein as a "therapeutically effective amount"). The selected dosage level will depend upon the activity of the particular compound, the severity of the condition being treated and the condition and prior medical history of the patient being treated. However, it is within the skill of the art to start doses of the compound at levels lower than required for to achieve the desired therapeutic effect and to gradually increase the dosage until the desired effect is achieved.

According to a further aspect there is provided a parenteral formulation including a salt as described herein. The formulation may consist of the salt alone or it may contain additional components, in particular the salt may be in combination with a pharmaceutically acceptable diluent, exdpient or carrier, for example a tonicity agent for the purpose of making the formulation substantially isotonic with the body of the subject to receive the formulation, e.g. with human

plasma. The formulation may be in ready-to-use form or in a form requiring reconstitution prior to administration.

It is currentty contemplated that, in the case of parenteral administration, for example i.v. administration, of salts of TRI 50c, the salts might for instance be administered in an amount of from 0.5 to 2.5mg/Kg e.g. over a maximum period of 72 hours, calculated as TRI 50c. Other salts might be administered in equivalent molar amounts. The disclosure is not limited to administration in such quantities or regimens and includes dosages and regimens outside those described in the previous sentence.

Parenteral preparations can be administered by one or more routes, such as intravenous, subcutaneous, intradermal and infusion; a particular example is intravenous. A formulation disclosed herein may be administered using a syringe, injector, plunger for solid formulations, pump, or any other device recognized in the art for parenteral administration.

Liquid dosage forms for parenteral administration may include solutions, suspensions, liposome formulations, or emulsions in oily or aqueous vehicles. In addition to the active compounds, the liquid dosage forms may contain other compounds. Tonicity agents (for the purpose of making the formulations substantially isotonic with the subject's body, e.g. with human plasma) such as, for instance, sodium chloride, sodium sulfate, dextrose, mannitol and/or glycerol may be optionally added to the parenteral formulation. A pharmaceutically acceptable buffer may be added to control pH. Thickening or viscosity agents, for instance well known cellulose derivatives (e.g. methylcellulose, carboxymethylcellulose, hydroxyethylcellulose and hydroxypropylmethylcellulose), gelatin and/or acacia, may optionally be added to the parenteral formulation.

Solid dosage forms for parenteral administration may encompass solid and semi-solid forms and may include pellets, powders, granules, patches, and gels. In such solid dosage forms, the active compound is typically mixed with at least one inert, pharmaceutically acceptable exdpient or carrier. The disclosed salts may be presented as solids in finely divided solid form, for example they may be milled or micronised.

The formulations may also include antioxidants and/or preservatives. As antioxidants may be mentioned thiol derivatives (e.g. thioglycerol, cysteine, acetylcysteine, cystine, dithioerythreitol, dithlothreibol, glutathione), tocopherols, butylated hydroxyanisole, butylated hydroxytoluene, sulfurous acid salts (e.g. sodium sulfate, sodium bisulfite, acetone sodium bisuffite, sodium metabisulfite, sodium sulfite, sodium formaldehyde sulfoxylate, sodium thiosulfate) and nordihydroguaiareticacid. Suitable preservatives may for instance be phenol, chlorobutanol, benzylalcohol, methyl paraben, propyl paraben, benzalkonium chloride and cetylpyridinium chloride.

The parenteral formulations may be prepared as large volume parenterals (LVPs), e.g. larger than 100 ml, more particularly about 250 ml, of a liquid formulation of the active compound. Examples of LVPs are infusion bags. The parenteral formulations may alternatively be prepared as small volume parenterals (SVPs), e.g. about 100 ml or less of a liquid formulation of the active compound. Examples of SVPs are vials with solution, vials for reconstitution, prefilled syringes for injection and dual chamber syringe devices.

The formulations of the disclosure include those in which the salt is an alkali metal salt, for example a lithium, sodium or potassium salt, of which sodium salts may be mentioned as particular salts. Another class of formulations contains aminosugar salts of the disclosed boronic acids, for example N-methyl-D-glucamine salts. The salts mentioned in this paragraph may be administered as solutions in water, typically containing one or more additives, for example isotonicity agent(s) and/or antioxidant(s). A suitable way to store the salts is in solid form, for example as dry powder, and to make them up into solutions for administration prior to administration.

One class of formulations disclosed herein is intravenous formulations. For intravenously administered formulations, the active compound or compounds can be present at varying concentrations, with a carrier acceptable for parenteral preparations making up the remainder. Particularly, the carrier is water, particularly pyrogen free water, or is aqueous based. Particularly, the carrier for such parenteral preparations is an aqueous solution comprising a tonicity agent, for example a sodium chloride solution.

By "aqueous based" is meant that formulation comprises a solvent which consists of water or of water and water-miscible organic solvent or solvents; as well as containing a salt of disclosure in dissolved form, the solvent may have dissolved therein one or more other substances, for example an antioxidant and/or an isotonicity agent. As organic cosolvents may be mentioned those water-miscible solvents commonly used in the art, for example propyleneglycol, polyethyleneglycol 300, polyethyleneglycol 400 and ethanol. Preferably, organic co-solvents are only used in cases where the active agent is not sufficiently soluble in water for a therapeutically effective amount to be provided in a single dosage form. As previously indicated, the disclosure includes formulations of alkali metal salts of the disclosed boronic acids, e.g. TRI 50c, having a solvent which consists of water.

The solubility of the active compound in the present formulations may be such that the turbidity of the formulation is lower than 50 NTU, e.g. lower than 20 NTU such as lower than 10 NTU.

It is desirable that parenteral formulations are administered at or near physiological pH. It is believed that administration in a formulation at a high pH (i.e., greater than 8) or at a low pH (i.e., less than 5) is undesirable. In particular, it is contemplated that the formulations would be administered at a pH of between 6.0 and 7.0 such as a pH of 6.5.

The parenteral formulation may be purged of air when being packaged. The parenteral formulation may be

packaged in a sterile container, e.g. vial, as a solution, suspension, gel, emulsion, solid or a powder. Such formulations may be stored either in ready-to-use form or in a form requiring reconstitution prior to administration.

Parenteral formulations according to the disclosure may be packaged in containers. Containers may be chosen which are made of material which is non-reactive or substantially non-reactive with the parenteral formulation. Glass containers or plastics containers, e.g. plastics infusion bags, may be used. A concern of container systems is the protection they afford a solution against UV degradation. If desired, amber glass employing iron oxide or an opaque cover fitted over the container may afford the appropriate UV protection.

Plastics containers such as plastics infusion bags are advantageous in that they are relatively light weight and non-breakable and thus more easily stored. This is particularly the case for Large Volume parenterals.

The intravenous preparations may be prepared by combining the active compound or compounds with the carrier. After the formulation is mixed, it may be sterilized, for example using known methods. Once the formulation has been sterilized, it is ready to be administered or packaged, particularly in dark packaging (e.g. bottles or plastics packaging), for storage. It is envisaged, however, that the disclosed salts might not be stored in solution but as dry solids, particularly a finely divided form such as, for example, a lyophilisate, in order to prolong shelf life; this would of course apply to other parenteral formulations, not only intravenous ones.

The intravenous preparations may take the form of large volume parenterals or of small volume parenterals, as described above.

In a specific embodiment, the present disclosure is directed to products, particularly kits, for producing a single-dose administration unit. The products (kits) may each contain both a first container having the active compound (optionally combined with additives, for example anti-oxidant, preservative and, in some instances, tonidty agent) and a second container having the carrier/diluent (for example water, optionally containing one or more additives, for example tonicity agent). As examples of such products may be mentioned single and multi-chambered (e.g. dual-chamber) pre-filled syringes; exemplary pre-filled syringes are available from Vetter GmbH, Ravensburg, Germany. Such dual chamber syringes or binary syringes will have in one chamber a dry preparation including or consisting of the active compound and in another chamber a suitable carrier or diluent such as described herein. The two chambers are joined in such a way that the solid and the liquid mix to form the final solution.

One class of formulations disclosed herein comprises subcutaneous or intradermal formulations (for example formulations for injection) in which the active salt (or active agent combination) is formulated into a parenteral preparation that can be injected subcutaneously or intradermally. The formulation for administration will comprise the active salt and a liquid carrier.

The carrier utilized in a parenteral preparation that will be injected subcutaneously or intradermally may be an aqueous carrier (for example water, typically containing an additive e.g. an antioxidant and/or an isotonicity agent) or a nonaqueous carrier (again one or more additives may be incorporated). As a non-aqueous carrier for such parenteral preparations may be mentioned highly purified olive oil.

The active compound and the carrier are typically combined, for example in a mixer. After the formulation is mixed, it is preferably sterilized, such as with U.V. radiation. Once the formulation has been sterilized, it is ready to be injected or packaged for storage. It is envisaged, however, that the disclosed salts will not be stored in liquid formulation but as dry solids, in order to prolong shelf life.

For making subcutaneous implants, the active salt may suitably be formulated together with one or more polymers that are gradually eroded or degraded when in use, e.g. silicone polymers, ethylene vinylacetate, polyethylene or polypropylene.

Transdermal formulations may be prepared in the form of matrices or membranes, or as fluid or viscous formulations in oil or hydrogels or as a compressed powder pellet. For transdermal patches, an adhesive which is compatible with the skin may be included, such as polyacrylate, a silicone adhesive or polyisobutylene, as well as a foil made of, e.g., Polyethylene, polypropylene, ethylene vinylacetate, polyvinylchloride, polyvinylidene chloride or polyester, and a removable protective foil made from, e.g., polyester or paper coated with silicone or a fluoropolymer. For the preparation of transdermal solutions or gels, water or organic solvents or mixtures thereof may be used. Transdermal gels may furthermore contain one or more suitable gelling agents or thickeners such as silicone, tragacanth, starch or starch derivatives, cellulose or cellulose derivatives or polyacrylic acids or derivatives thereof. Transdermal formulations may also suitably contain one or more substances that enhance absorption though the skin, such as bile salts or derivatives thereof and/or phospholipids. Transdermal formulations may be prepared according to a method disclosed in, e.g., B W Barry, "Dermatological Formulations, Percutaneous Absorption", Marcel Dekker Inc., New YorkBasel, 1983, or Y W Chien, "Transdermal Controlled Systemic Medications", Marcel Dekker Inc., New York-Basel, 1987.

It will be understood from the aforegoing that there are provided pharmaceutical products comprising an alkali metal salt, particularly sodium salt, of a boronic acid of Formula (I) in dry fine particle form, suitable for reconstitution into an aqueous read-to-use parenteral formulation. The alkali metal salt is suitably an acid salt. The alkali metal salt may be in a small volume parenteral unit dosage form. The alkali metal salt may be presented in a form, e.g. dry powder form, suitable for reconstituting as a large volume parenteral. One example is a sodium salt of a boronic acid of Formula

(I), particularly TRI 50c, in dry powder form for reconstitution as a liquid intravenous formulation (solution) containing a tonicity agent, particularly sodium chloride. The dry powder form of a salt used in a parenteral formulation may be a lyophilisate. The reconstituted solution may be administered by injection or infusion.

## EXAMPLES

GUIDE TO THE EXAMPLES

All the Examples relate to the Novel Products of the disclosure. Specifically, the Examples relate to the different categories mentioned in the "Guide to the Specification" as follows:

- Examples 1 to 4 and 42 to 44 relate to Synthetic Methods II and thus to the High Purity Products (a class of Novel Product)
- Examples 5 to 35 and 40 relate to Synthetic Methods I and to the characterisation and testing of the Novel Products made using the techniques of Synthetic Methods I
- Examples 36 to 39 and 41 contain unpublished material serving to verify that TRI 50c, and hence the Novel Products (including therefore the High Purity Products), are effective in arterial as well as venous contexts.

EXAMPLES 1 TO 4 - INTRODUCTORY REMARKS

### Apparatus

Throughout the following procedures of Examples 1 to 4, standard laboratory glassware and, where appropriate, specialised apparatus for handling and transferring of air sensitive reagents are used.
All glassware is heated at 140-160°C for at least 4 hours before use and then cooled either in a desiocator or by assembling hot and purging with a stream of dry nitrogen.

### Solvents

The organic solvents used in the procedures of Examples 1 to 4 are all dry. Suitably, they are dried over sodium wire before use.

### Dryness

In the drying procedures of Example 1 to 4, products are tested for dryness (including dryness in terms of organic solvent) by observing weight loss on drying. The following procedure was followed to determine loss on drying: a sample was placed in a vacuum drier and dried at 40°C at 100 mbar for 2 hours. Products are considered dry when the decrease in weight upon drying is less than 0.5% of the total weight of the starting material.
Examples 1 to 4 describe performance of the following reaction scheme and conversion of the resultant TRI 50c to sodium and calcium salts thereof:

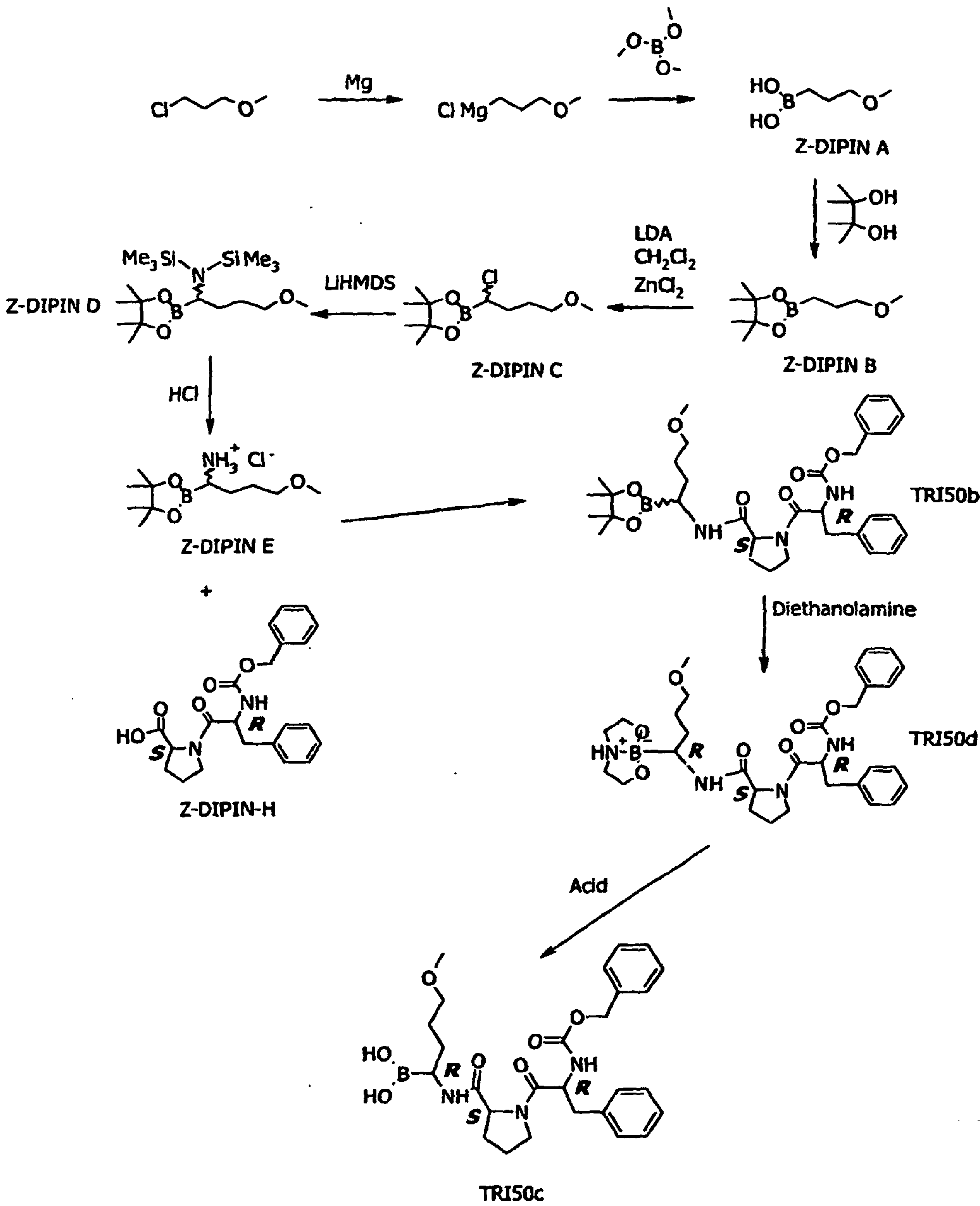


LDA = lithium diisopropylamide

LiHMDS = lithium hexamethyldisilazane, also known as lithium bis(trimethylsilyl)amide

EXAMPLE 1 - SYNTHESIS OF TR1 50D

**Step 1: Z-DIPIN B**

Procedure A

17.8 g (732.5 mmole) magnesium turnings, 0.1 g (0.4 mmole) iodine and 127 ml dry tetrahydrofuran are charged and heated to reflux. Then 15 ml of a solution of 66 g (608 mmole) 1-chloro-3-methoxypropane in 185 ml dry tetrahydrofuran are added and stirred under reflux until the vigorous reaction starts. After the initial exotherm ceases, the solution of 1-chloro-3-methoxypropane is added slowly to maintain gentle reflux until all the magnesium is consumed. After the reaction is finished, the reaction mixture is cooled to ambient temperature and slowly' added to a solution of 64.4 g (620 mmole) trimethylborate in 95 ml dry tetrahydrofuran; the latter solution is cooled to below 0°C and, if it warms up during the course of the reaction, the reaction mixture must be added to it sufficiency slowly to maintain the temperature of this solution below 65°C. Upon complete addition, the reaction mixture is allowed to warm to about 0°C and stirred for another 60 minutes. Then a solution of 22.4 ml sulfuric acid in 400 ml water is added slowly so as to maintain the temperature below 20°C. The layers are allowed to settle and the phases are separated. The aqueous layer is rewashed three times with 200 ml tert.-butylmethylether. The combined organic layers are allowed to settle and additional water separated from this solution is removed. The organic layer is dried over magnesium sulfate, filtered and evaporated to dryness. The evaporation residue is filtered from the precipitated solid and the filtrate dissolved in 175 ml toluene. 34.8 g (292 mmole) pinacol is charged to the solution followed by stirring at ambient temperature for not less than 10 hours. The solution is evaporated to dryness, dissolved In 475 ml n-heptane and washed three times with 290 ml saturated aqueous solution of sodium hydrogen carbonate. The n-heptane solution is evaporated to dryness and the evaporation residue distilled and the fraction with Bp 40-50°C at 0.1-0.5 mbar recovered.
Boiling point: 40-50°C /0.1-0.5 mbar
Yield: 40.9 g (70%) Z-DIPIN B (oil)

Procedure B

17.8 g (732.5 mmole) magnesium turnings, 0.1 g (0.4 mmole) iodine and 127 ml dry tetrahydrofuran are charged and heated to reflux. Then 15 ml of a solution of 66 g (608 mmote) 1-chloro-3-methoxypropane in 185 ml dry tetrahydrofuran are added and stirred under reflux until the vigorous reaction starts. After the initial exotherm ceases, the solution of 1-chloro-3-methoxypropane is added slowly to maintain gentle reflux. After the reaction is finished, the reaction mixture is cooled to ambient temperature and slowly added to a solution of 64.4 g (620 mmole) trimethylborate in 95 ml dry tetrahydrofuran, maintaining the temperature of this solution below minus 65°C. Upon complete addition, the reaction mixture is allowed to warm to about 0°C and stirred for another 60 minutes. Then a solution of 22.4 ml sulfuric acid in 400 ml water is added slowly so as to maintain the temperature below 20°C. The organic solvent is removed by distillation under vacuum. 300 ml n-heptane is charged to the aqueous solution of the evaporation residue followed by addition of 34.8 g (292 mmole) pinacol. The two-phase-mixture is stirred at ambient temperature for not less than 2 hours. After allowing the layers to settle, the aqueous phase is separated. 300 ml n-heptane is charged to the aqueous solution and the two-phase-mixture is stirred at ambient temperature for not less than 2 hours. After allowing the layers to settle, the aqueous phase is separated. The organic layers are combined and washed once with 200 ml water, followed by 200 ml saturated sodium hydrogen carbonate solution and two further washes with 200 ml water each. The n-heptane solution is evaporated to dryness and the evaporation residue distilled and the fraction with Bp 40-50°C at 0.1-0.5 mbar recovered.
Boiling point: 40-50°C / 0.1-0.5 mbar
Yield: 40.9 g (70-85%) Z-DIPIN B (oil)

**Step 2: Z-DIPIN C**

16.6 g (164 mmole) diisopropylamine and 220 ml tetrahydrofuran are charged and cooled to -30 to -40°C. To this solution 41.8 g (163 mmole) n-butyl lithium, 25% in n-heptane is added, followed by stirring at 0 to -5°C for one hour. This freshly prepared solution of lithium diisopropylamide is cooled to -30°C and then added to a solution of 27.9 g (139 mmole) Z-DIPIN B in 120 ml tetrahydrofuran and 35.5 g (418 mmole) dichloromethane at a temperature between -60 and - 75°C. The solution is stirred at that temperature for half an hour followed by addition of 480 ml (240 mmole) 0.5N anhydrous Zinc(II)-chloride in tetrahydrofuran or 32.5 g (240 mmole) anhydrous solid Zinc(II)-chloride. After stirring at -65°C for one hour, the reaction mixture is allowed to warm to ambient temperature and stirred for another 16-18 hours. The reaction mixture is evaporated to dryness (i.e. until solvent is removed) and followed by addition of 385 ml n-heptane. The reaction mixture is washed with 150 ml 5% sulfuric acid, with 190 ml saturated sodium hydrogen carbonate solution, and 180 ml saturated sodium chloride solution. The organic layer is dried over magnesium sulfate, filtered and evaporated

to dryness (i.e. until solvent is removed). The oily residue is transferred into the next step without further purification.
Yield: 19 g (55%) Z-DIPIN C

**Step 3: Z-DIPIN D**

To a solution of 23.8 g (148 mmole) hexamethyldisilazane in 400 ml tetrahydrofuran at -15°C is added 34.7 g (136 mmole) n-butyl lithium, 25% in n-heptane and stirred for one hour. The solution is cooled to -55°C followed by the addition of 30.6 g (123 mmole) Z-DIPIN C dissolved in 290 ml tetrahydrofuran and 35 ml tetrahydrofuran to this freshly prepared solution of UHMDS. The solution is allowed to warm to ambient temperature and stirred for 12 hours. The reaction mixture is evaporated to dryness, the evaporation residue dissolved in 174 ml n-heptane, washed with 170 ml water and 75 ml saturated sodium chloride solution. The organic phase is dried over magnesium sulfate, filtered and evaporated to complete dryness (i.e. until solvent is removed). The oily residue is dissolved in 100 g n-heptane. This solution is carried over into the next step without further purification.
Yield: 32.2 g (70%) Z-DIPIN D

**Step 4: Z-DIPIN (TRI50b, crude)**

A solution of 26.6 g (71 mmole) Z-DIPIN D in 82.6 g n-heptane is diluted with 60 ml n-heptane and cooled to -60°C followed by Introduction of 10.5 g (285 mmole) hydrogen chloride. The reaction mixture is subsequently evacuated and flushed with nitrogen, while the temperature is increased in increments of about 20°C to ambient temperature. The solvent is removed from the oily precipitate and replaced several times by 60 ml fresh n-heptane. The oily residue is dissolved in 60 ml tetrahydrofuran (Solution A).

To a different flask 130 ml tetrahydrofuran, 24.5 g (61.5 mmole) Z-D-Phe-Pro-OH and 6.22 g (61.5 mmole) N-methylmorpholine are charged and cooled to -20°C. To this solution a solution of 8.4 g (61.5 mmole) isobutylchloroformate in 20 ml tetrahydrofuran is added and stirred for 30 minutes, followed by addition of Solution A at -25°C. Upon complete addition, up to 16 ml (115 mmole) triethylamine is added to adjust the pH to 9-10, measured using a pH stick. The reaction mixture is allowed to warm to ambient temperature and stirred for 3 hours, still under nitrogen. The solvent is evaporated to dryness and the evaporation residue dissolved in 340 ml tert.-butylmethylether (t-BME). The solution of Z-DIPIN in t-BME is washed twice with 175 ml 1.5% hydrochloric acid. The combined acidic washes are given a rewash with 175 ml t-BME. The combined organic layers are washed with 175 ml water, with 175 ml saturated sodium hydrogen carbonate solution, with 175 ml 25% sodium chloride solution, dried over magnesium sulfate and filtered. This solution is carried over into the next step without further purification.
Yield: 29.9 g (80%) Z-DIPIN

EXAMPLE 2 - SYNTHESIS OF TRI 50D (DIETHANOLAMINE ADDUCT OF TRI 50C)

The starting material used in this Example is the solution of TRI 50b ("Z-DIPIN") obtained in Example 1. The solution is carried forward to the synthesis of TRI 50d without further purification. The solution of Z-DIPIN in t-BME (containing 7.0 g (11.5 mmole) (R,S,R) TRI50b, calculated based on HPLC results of Z-DIPIN) Is evaporated to dryness and the evaporation residue dissolved in 80 ml diethylether. 1.51 g (14.4 mmole) diethanolamine is added and the mixture heated at reflux for at least 10 hours, during which process the product precipitates. The suspension is cooled to 5-10°C, filtered and the filter residue washed with diethylether.

To improve chiral and chemical purity the wet filter cake (7 g) is dissolved in 7 ml dichloromethane, cooled to 0-5°C and the product precipitated by addition of 42 ml diethylether and filtered. The isolated wet product is dried at 35°C in vacuum or at least 4 hours, until dry.
Yield: 5.5 g (80%) Tri50d
Melting Point: 140-145°C

EXAMPLE 3 - PREPARATION OF SODIUM SALT OF TRI50C

1.5 kg (2.5 mole) TRI50d from Example 2 is dissolved in 10.5 L dichloromethane. 11 L 2% hydrochloric acid is added and the mixture is stirred for at most 30 minutes (optimally about 20 minutes) at room temperature. A precipitate forms in the organic phase. After stirring, the layers are allowed to settle and separated. The aqueous layer is rewashed twice with 2.2 L dichloromethane. The combined organic layers are washed with a solution of 625 g ammonium chloride in 2.25 L water. (The ammonium chloride buffers the pH of the aqueous extractions to be within a range of from about pH 1-2 to about pH 4-5, as strongly acidic conditions might cleave peptide bonds). The organic phase is dried over magnesium sulfate, filtered and the filtrate evaporated to dryness. An assay of the free boronic acid is performed (by the RP HPLC method of Example 43 for at most 30 mins (optionally about 20 min) at room temperature) and the amounts

of the solvents and base for conversion of the acid to the salt are calculated. If 2.5 mol of the free acid is obtained, the evaporation residue is dissolved in 5 L acetonitrile followed by addition of a solution of 100 g (2.5 mole) sodium hydroxide as a 5% solution in 2.2 L water. The solution is stirred for two hours at ambient temperature (e.g. 15-30°C, optimally room temperature) and then evaporated in vacuum (of ca. 10 mmHg) at a temperature not exceeding 35°C. The evaporation residue is repeatedly dissolved in 3.5 L fresh acetonitrile and evaporated to dryness to remove traces of water. If the evaporation residue is dry, it is dissolved in 3 L acetonitrile (or alternatively in 6 L THF) and slowly added to a mixture of 32 L n-heptane and 32 L diethylether. The addition is performed slowly enough to avoid lumping or sticking of the product and is carried out over a period of not less than 30 minutes. The precipitated product is filtered off, washed with n-heptane and dried under vacuum at a temperature initially of about 10°C and then increasing to a limit of about 35°C, until dry.
Yield: 1.0 kg (70%) Tri50c sodium salt.

EXAMPLE 4 - PREPARATION OF CALCIUM SALT OF TRI50C

1.5 kg (2.5 mole) TRI50d from Example 2 is dissolved in 10.5 L dichloromethane. 11 L 2% hydrochloric acid is added and the mixture is stirred for at most 30 minutes (optimally about 20 minutes) at room temperature. After stirring the layers are allowed to settle and separated. The aqueous layer is given a rewashed twice with 2.2 L dichloromethane. The combined organic layers are washed with a solution of 625 g ammonium chloride in 2.25 L water. The organic phase is dried over magnesium sulfate, filtered and the filtrate evaporated to dryness. An assay of the free boronic acid is performed and the amounts of the solvents and base for conversion of the acid to the salt are calculated. If 2.5 mol of the free acid is obtained, the evaporation residue is dissolved in 5 L acetonitrile followed by addition of a suspension of 93 g (1.25 mole) calcium hydroxide in 1 L water. The solution is stirred for two hours at ambient temperature (e.g. 15-30°C, optimally room temperature) and then evaporated under vacuum (of ca. 10 mmHg) at a temperature initially of about 10°C and then increasing to a limit of about 35°C. The evaporation residue is repeatedly dissolved in 3.5 L fresh acetonitrile and evaporated to dryness to remove traces of water. If the evaporation residue is dry, it is dissolved in 6 L tetrahydrofuran and slowly added to a mixture of 32 L n-heptane and 32 L diethylether. The addition is performed slowly enough to avoid lumping or sticking of the product and is carried out over a period of not less than 30 minutes. The precipitated product is filtered off, washed with n-heptane and dried under vacuum (of ca. 10 mmHg) at a temperature below 35°C until dry.
Yield: 0.98 kg (70%) Tri50c calcium salt.

The procedures of Examples 1 to 4 may be scaled up and, if operated carefully, will produce highly pure salts. In the diethanolamine precipitation step it is important to use 1.25 equivalents of diethanolamine per equivalent of (R, S,R) TRI 50b. In the hydrolysis of the diethanolamine ester, it is important to avoid excessively long contact with the aqueous acid. Likewise the TRI 50b should be synthesised via the Grignard reaction to Z-DIPIN A.

EXAMPLE 5 - ALTERNATIVE CONVERSION OF TRI 50B TO TRI 50C

The synthetic procedures described in this and subsequent synthetic examples were generally performed under nitrogen and using dry solvents as supplied from commercial sources.

1. Approximately 300 g of TRI 50b, obtained by the HPLC purification of racemic TRI 50b) were dissolved in approximately 2.5 L diethylether. It is estimated that different batches of TRI 50b had isomeric purities ranging from 85% R,S,R to in excess of 95% R,S,R.
2. Approximately 54 ml diethanolamine were added (1:1 stoichiometry with total TRI 50b content), and the mixture was refluxed at 40 °C.
3. The precipitated product was removed, washed several times with diethylether and dried.
4. The dry product was dissolved in $CHCl_3$. Hydrochloric acid (pH 1) was added and the mixture was stirred approximately 1h at room temperature.
5. The organic layer was removed and washed with $NH_4Cl$ solution.
6. The organic solvent was distilled off and the residual solid product was dried.

Typical yield: Approximately 230 g

EXAMPLE 6 - PREPARATION OF LITHIUM SALT OF TRI50C

Cbz-Phe-Pro-BoroMpg-OH obtained by the method of Example 5 (20.00g, 38.1mM) is dissolved in acetonitrile (200ml) with stirring at room temperature. To this solution is added LiOH as a 0.2M solution in distilled water (190ml). The resultant dear solution is stirred for 2 hours at room temperature and then evacuated to dryness under vacuum with

its temperature not exceeding 37°C. The resultant oil/tacky liquid is redissolved in 500ml distilled water necessary with light warming for about 20 minutes. The solution is filtered through filter paper and evacuated to dryness, again with the temperature of the solution not exceeding 37°C. The resultant product is dried under vacuum overnight to normally yield a white brittle solid.

The salt was then dried under vacuum over silica to constant weight (72 h).
Yield 17.89g.
Microanalysis:

| C % Found (Calc.) | H % Found (Calc.) | N % Found (Calc.) | B % Found (Calc.) | Metal % Found (Calc.) |
|---|---|---|---|---|
| 57.14<br>(61.03) | 6.60<br>(6.64) | 7.34<br>(7.90) | 2.07<br>(2.03) | Li 1.26<br>(1.31) |

EXAMPLE 7 - UV/VISIBLE SPECTRA OF LITHIUM SALT OF TRI50C

UV/Visible spectra of the salt resulting from the procedure of Example 6 were recorded In distilled water at 20°C from 190nm to 400nm. The salt gave $\lambda_{max}$ at 210 and 258nm. The weight of the dried salt was then measured for the purposes of calculating the extinction coefficient. The $\lambda_{max}$ at 258nm was used. The extinction coefficient was calculated using the formula:-

$$A = \varepsilon cl$$

where

A is the absorbance.
C is the concentration
I the path length of the UV cell
and ε is the extinction coefficient.

Extinction coefficient: 451

EXAMPLE 8 - AQUEOUS SOLUBILITY OF LITHIUM SALT OF TRI50C

The salt used in this Example was made using a modification of the process described in Example 6. The modified process differs from that described in that 100mg of TRI 50c was used as starting material, the product of the redissolution in water was dried by freeze drying and the filtration was carried out through a 0.2μm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material. The lithium salt was comparatively soluble and so was redissolved at 50mg/ml in the same manner previously described.

Solubility when dissolved at 25mg/ml: 43mM (23 mg/ml).
Solubility when dissolved at 50mg/ml: 81mM (43 mg/ml).

EXAMPLE 9 - PREPARATION OF SODIUM SALT OF TRI50C

Cbz-Phe-Pro-BoroMpg-OH obtained by the method of Example 5 (20.00g, 38.1mM) is dissolved in acetonitrile (200ml) with stirring at room temperature. To this solution is added NaOH as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 500ml distilled water with light warming for about 15-20 minutes. The solution is filtered through filter paper and evacuated to dryness, again with the temperature of the solution not exceeding 37°C. The resultant product is dried under vacuum overnight to normally yield a white brittle solid. The product may be present as an oil or tacky solid due to residual water, in which case it is dissolved in ethyl acetate and evacuated to dryness to produce the product as a white solid.

The salt was then dried under vacuum over silica to constant weight (72 h).
Yield: Over 50%.
Microanalysis:

| C % Found (Calc.) | H % Found (Calc.) | N % Found (Calc.) | B % Found (Calc.) | Metal % Found (Calc.) |
|---|---|---|---|---|
| 59.93<br>(59.24) | 6.47<br>(6.44) | 7.31<br>(7.67) | 1.91<br>(1.98) | Na 3.81<br>(4.20) |

EXAMPLE 10 - UV/VISIBLE SPECTRA OF SODIUM SALT OF TRI50C

UV/Visible spectra of the sodium salt resulting from the procedure of Example 9 were recorded in distilled water at 20°C from 190nm to 400nm. The salt gave $\lambda_{max}$ at 210 and 258nm. The weight of the dried salt was then measured for the purposes of calculating the extinction coefficient. The $\lambda_{max}$ at 258nm was used. The extinction coefficient was calculated using the formula:

$$A = \varepsilon cl$$

where

A is the absorbance
C is the concentration
I the path length of the UV cell
and ε is the extinction coefficient.

Extinction coefficient: 415.

EXAMPLE 11- AQUEOUS SOLUBILITY OF SODIUM SALT OF TRI50C

The salt used in this Example was made using a modification of the process described in Example 9. The modified process differs from that described In that 100mg of TRI 50c was used as starting material, the product of the redissolution in water was dried by freeze drying and the filtration was carried out through a 0.2μm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material. The sodium salt was comparatively soluble and so was redissolved at 50mg/ml in the same manner previously described.

Solubility when dissolved at 25mg/ml: 44mM (25 mg/ml).
Solubility when dissolved at 50mg/ml: 90mM (50 mg/ml).

EXAMPLE 12 - PREPARATION OF POTASSIUM SALT OF TRI50C

Cbz-Phe-Pro-BoroMpg-OH obtained by the method of Example 5 (20.00g, 38.1mM) is dissolved in acetonitrile (200ml) with stirring at room temperature. To this solution is added KOH as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 1L distilled water with warming to 37°C for about 2 hours. The solution is filtered through filter paper and evacuated to dryness, again with the temperature of the solution not exceeding 37°C. The resultant product is dried under vacuum overnight to normally yield a white brittle solid.
Yield: 14.45 mg.

The salt was then dried under vacuum over silica to constant weight (72 h).
Microanalysis:

| C % Found (Calc.) | H % Found (Calc.) | N % Found (Calc.) | B % Found (Calc.) | Metal % Found (Calc.) |
|---|---|---|---|---|
| 54.84<br>(57.55) | 6.25<br>(6.26) | 7.02<br>(7.45) | 2.01<br>(1.92) | K 4.29<br>(6.94) |

EXAMPLE 13 - UV/VISIBLE SPECTRA OF POTASSIUM SALT OF TRI50C

UV/Visible spectra of the potassium salt resulting from the procedure of Example 12 were recorded in distilled water at 20°C from 190nm to 400nm. TRI50C and the salt gave $\lambda_{max}$ at 210 and 258nm. The weight of the dried salt was then measured for the purposes of calculating the extinction coefficient The $\lambda_{max}$ at 258nm was used. The extinction coefficient was calculated using the formula:-

$$A = \varepsilon cl$$

where

A is the absorbance
c is the concentration
I the path length of the UV cell
and ε is the extinction coefficient

Extinction coefficient: 438.

EXAMPLE 14 - AQUEOUS SOLUBILITY OF POTASSIUM SALT OF TRI50C

The salt used in this Example was made using a modification of the process described in Example 12. The modified process differs from that described in that 100mg of TRI 50c was used as starting material, the product of the redissolution in water was dried by freeze drying and the nitration was carried out through a 0.2μm filter. The salt is believed to contain about 85% of R,S,R isomer.
To determine maximum aqueous solubility 25mg of the dried salt were shaken in water at 37°C, the sample filtered and the UV spectrum measured. The salt left a white residue of undissolved material.
Solubility when dissolved at 25mg/ml: 29mM (16 mg/ml).

EXAMPLE 15 - PREPARATION OF ZINC SALT OF TRI 50C

The relative solubility of zinc hydroxide is such that, if the hydroxide had been used to prepare the corresponding TRI 50c salt using the procedure of Example 6, they would not have resulted in homogeneous salt formation. A new method was therefore developed to prepare the zinc salt, as described in this and the next examples.
TRI 50c sodium salt (2.24g, 4.10mM) was dissolved in distilled water (100ml) at room temperature and zinc chloride in THF (4.27ml, 0.5M) was carefully added with stirring. A white precipitate that immediately formed was filtered off and washed with distilled water. This solid was dissolved in ethyl acetate and washed with distilled water (2 x 50ml). The organic solution was evacuated to dryness and the white solid produced dried over silica in a desiccator for 3 days before microanalysis. Yield 1.20g.
$^1$H NMR 400MHz, $\delta_H(CD_3OD)$ 7.23-7.33 (20H, m, ArH), 5.14 (4H, m, $PhCH_2O$), 4.52 (4H, m, αCH), 3.65 (2H, m), 3.31 (12H, m), 3.23 (6H, s, $OCH_3$), 2.96 (4H, d, *J*7.8Hz), 2.78 (2H, m), 2.58 (2H, m), 1.86 (6H, m), 1.40 (10H, m).
$^{13}$C NMR 75MHz $\delta_C(CD_3OD)$ 178.50, 159.00, 138.05, 137.66, 130.54, 129.62, 129.50, 129.07, 128.79, 128.22, 73.90, 67.90, 58.64, 58.18, 56.02, 38.81, 30.06, 28.57, 28.36, 25.29.
FTIR (KBr disc) $\nu_{max}$ ($cm^{-1}$) 3291.1, 3062.7, 3031.1, 2932.9, 2875.7, 2346.0, 1956.2, 1711.8, 1647.6, 1536.0, 1498.2, 1452.1, 1392.4, 1343.1, 1253.8, 1116.8, 1084.3, 1027.7, 916.0, 887.6, 748.6, 699.4, 595.5, 506.5.

EXAMPLE 16 - PREPARATION OF ARGININE SALT OF TRI50C

Cbz-Phe-Pro-BoroMpg-OH obtained by the method of Example 5 (20.00g, 38.1mM) is dissolved in acetonitrile (200ml) with stirring at room temperature. To this solution is added arginine as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 2L distilled water with warming to 37°C for 2 hours. The solution is filtered through filter paper and evacuated to dryness, again with the temperature of the solution not exceeding 37°C. The resultant product is dried under vacuum overnight to normally yield a white brittle solid.
The salt was then dried under vacuum over silica to constant weight (72 h).
Yield: 10.54g.

Microanalysis:

| C % Found (Calc.) | H % Found (Calc.) | N % Found (Calc.) | B % Found (Calc.) |
|---|---|---|---|
| 52.47 (56.65) | 7.12 (7.20) | 15.25 (14.01) | 1.52 (1.54) |

EXAMPLE 17 - UV/VISIBLE SPECTRA OF ARGININE SALT OF TRI50C

UV/Visible spectra of the salt resulting from the procedure of Example 15 were recorded in distilled water at 20°C from 190nm to 400nm. TRI50C and the salt gave $\lambda_{max}$ at 210 and 258nm. The weight of the dried salt was then measured for the purposes of calculating the extinction coefficient. The $\lambda_{max}$ at 258nm was used. The extinction coefficient was calculated using the formula:-

$$A = \varepsilon cl$$

where

A is the absorbance
C is the concentration
l the path length of the UV cell
and ε is the extinction coefficient.

Extinction ooeffident: 406.

EXAMPLE 18 - AQUEOUS SOLUBILITY OF ARGININE SALT OF TRI50C

The salt used in this Example was made using a modification of the process described in Example 16. The modified process differs from that described in that 100mg of TRI 50c was used as starting material, the product of the redissolution in water was dried by freeze drying and the filtration was carried out through a 0.2μm filter. The salt is believed to contain about 85% of R,S,R isomer.

To determine maximum aqueous solubility 25mg of the dried salt were shaken in water at 37°C, the sample filtered and the UV spectrum measured. The salt left a white residue of undissolved material.

Solubility when dissolved at 25mg/ml: 14mM (10 mg/ml).

EXAMPLE 19 - PREPARATION OF LYSINE SALT OF TRI50C

Cbz-Phe-Pro-BoroMpg-OH obtained by the method of Example 5 (20.00g, 38.1mM) is dissolved in acetonitrile (200ml) with stirring at room temperature. To this solution is added L-lysine as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved in 3L distilled water with warming to 37°C for 2 hours. The solution is filtered through filter paper and evacuated to dryness, again with the temperature of the solution not exceeding 37°C. The resultant product is dried under vacuum overnight to normally yield a white brittle solid. The product may be present as an oil or tacky solid (due to residual water), in which case it is then dissolved in ethyl acetate and evacuated to dryness to produce the product as a white solid.

The salt was then dried under vacuum over silica to constant weight (72 h).
Yield: 17.89.
Microanalysis:

| C % Found (Calc.) | H % Found (Calc.) | N % Found (Calc.) | B % Found (Calc.) |
|---|---|---|---|
| 57.03 (59.11) | 7.43 (7.36) | 10.50 (10.44) | 1.72 (1.61) |

EXAMPLE 20 - UV/VISIBLE SPECTRA OF LYSINE SALT OF TRI50C

UV/Visible spectra of the salt resulting from the procedure of Example 19 were recorded in distilled water at 20°C from 190nm to 400nm. TRI50C and the salt gave $\lambda_{max}$ at 210 and 258nm. The weight of the dried salt was then measured for the purposes of calculating the extinction coefficient. The $\lambda_{max}$ at 258nm was used. The extinction coefficient was calculated using the formula:-

$$A = \varepsilon cl$$

where

A is the absorbance
C is the concentration
I the path length of the UV cell
and ε is the extinction coefficient.

Extinction coefficient: 437.

EXAMPLE 21 - AQUEOUS SOLUBILITY OF LYSINE SALT OF TRI50C

The salt used in this Example was made using a modification of the process described in Example 19. The modified process differs from that described in that 100mg of TRI 50c was used as starting material, the product of the redissolution in water was dried by freeze drying and the filtration was carried out through a 0.2μm filter. The salt is believed to contain about 85% of R,S,R isomer.
To determine maximum aqueous solubility 25mg of the dried salt were shaken in water at 37°C, the sample filtered and the UV spectrum measured. The salt left a white residue of undissolved material.
Solubility when dissolved at 25mg/ml: 13mM (8.6 mg/ml).

EXAMPLE 22 - PREPARATION OF N-METHYL-D-GLUCAMINE SALT OF TRI50C

Cbz-Phe-Pro-BoroMpg-OH obtained by the method of Example 5 (20.00g, 38.1mM) is dissolved in acetonitrile (200ml) with stirring at room temperature. To this solution is added N-methyl-D-glucamine as a 0.2M solution in distilled water (190ml). The resultant dear solution is stirred for 2 hours at room temperature and then evacuated to dryness under vacuum with its temperature not exceeding 37°C. The resultant oil/tacky liquid is redissolved In 500ml distilled water with light warming for about 20 minutes. The solution is filtered through filer paper and evacuated to dryness, again with the temperature of the solution not exceeding 37°C, or freeze dried. The resultant product is dried under vacuum overnight to normally yield a white brittle solid.
The salt was then dried under vacuum over silica to constant weight (72 h).
Yield: 21.31g.
Microanalysis:

| C % Found (Calc.) | H % Found (Calc.) | N % Found (Calc.) | B% Found (Calc.) |
|---|---|---|---|
| 56.67 (56.67) | 7.28 (7.41) | 7.74 (7.77) | 1.63 (1.50) |

EXAMPLE 23 - UV/VISIBLE SPECTRA OF N-METHYL-D-GLUCAMINE SALT OF TRI50C

UV/Visible spectra of the salt resulting from the procedure of Example 22 were recorded in distilled water at 20°C from 190nm to 400nm. TRI50C and the salt gave $\lambda_{max}$ at 210 and 258nm. The weight of the dried salt was then measured for the purposes of calculating the extinction coefficient. The $\lambda_{max}$ at 258nm was used. The extinction coefficient was calculated using the formula:-

$$A = \varepsilon cl$$

where

A is the absorbance
C is the concentration
I the path length of the UV cell
and ε is the extinction coefficient.

Extinction coefficient: 433.

EXAMPLE 24 - AQUEOUS SOLUBILITY OF N-METHYL-D-GLUCAMINE SALT OF TRI50C

The salt used in this Example was made using a modification of the process described in Example 22. The modified process differs from that described in that 100mg of TRI 50c was used as starting material, the product of the redissolution in water was dried by freeze drying and the filtration was carried out through a 0.2μm filter. The salt is believed to contain about 85% of R,S,R isomer.

To determine maximum aqueous solubility 25mg of the dried salt were shaken in water at 37°C, the sample filtered and the UV spectrum measured. The salt was observed to fully dissolve. The salt was comparatively soluble and so was redissolved at 50mg/ml in the same manner previously described.

Solubility when dissolved at 25mg/ml: 35mM (25 mg/ml).
Solubility when dissolved at 50mg/ml: 70mM (50 mg/ml).

EXAMPLE 25 - ALTERNATIVE PREPARATION OF ARGININE SALT OF TRI50C

The arginine salt is formed simply by adding a slight molar excess of L-arginine to a solution of 0.2-0.3mmol of TRI50c in 10ml of ethyl acetate. The solvent is evaporated after one hour, and the residue is triturated twice with hexane to remove excess arginine.

EXAMPLE 26 - FIRST PREPARATION OF CALCIUM SALT OF TRI 50C

Cbz-Phe-Pro-BoroMpg-OH (20.00g, 38.1mM) obtained by the method of Example 5 is dissolved in acetonitrile (200ml) with stirring at room temperature. To this solution is added $Ca(OH)_2$ as a 0.1M solution in distilled water (190ml). The resultant dear solution is stirred for 2 hours at room temperature and then evacuated to dryness under vacuum with its temperature not exceeding 37°C. The resultant product is a white brittle solid.

The salt was then dried under vacuum over silica to constant weight (72 h).
Yield: 17.69g.

EXAMPLE 27-SECOND ALTERNATIVE PREPARATION OF CALCIUM SALT OF TRI 50C

50.0 g TRI 50c (95.2 mmol) were dissolved under stirring in 250 ml acetonitrile at room temperature and then cooled with an ice bath. To this ice cooled solution 100 ml of an aqueous suspension of 3.5 g (47.6 mmol) calcium hydroxide was added dropwise, stirred for 2.5 hours at room temperature, filtered and the resulting mixture evaporated to dryness, the temperature not exceeding 35°C. The dear yellowish oily residue was redissolved in 200 ml acetone and evaporated to dryness. The procedure of redissolving in acetone was repeated one more time to obtain colourless foam.

This foam was redissolved in 100 ml acetone, filtered and added dropwise to an ice cooled solution of 1100 ml petrol ether 40/60 and 1100 ml diethylether. The resulting colourless precipitate was filtered, washed two times with petrol ether 40/60 and dried under high vacuum, yielding 49.48 g of a colourless solid (92%), with a purity of 99.4% according to an HPLC measurement.

EXAMPLE 28 - UV/VISIBLE SPECTRA OF CALCIUM SALT OF TRI 50C

UV/Visible spectra of the salt resulting from the procedure of Example 26 were recorded in distilled water at 20°C from 190nm to 400nm. TRI 50C and the salt gave $\lambda_{max}$ at 210 and 258nm. The weight of the dried salt was then measured for the purposes of calculating the extinction coefficient. The $\lambda_{max}$ at 258nm was used. The extinction coefficient was calculated using the formula:-

$$A = \varepsilon cl$$

where

A is the absorbance
C is the concentration
I the path length of the UV cell
and ε is the extinction coefficient.

Extinction coefficient: 955.

EXAMPLE 29 - AQUEOUS SOLUBILITY OF CALCIUM SALT OF TRI 50C

The salt used in this Example was made using a modification of the process described in Example 27. The modified process differs from that described in that 100mg of TRI 50c was used as starting material, the product of the redissolution in water was dried by freeze drying and the filtration was carried out through a 0.2μm filter. The salt is believed to contain about 85% of R;S,R isomer.
To determine maximum aqueous solubility 25mg of the dried salt were shaken In water at 37°C, the sample filtered and the UV spectrum measured. The salt left a white residue of undissolved material.
Solubility when dissolved at 25mg/ml: 5mM (5 mg/ml).

EXAMPLE 30 - *IN VITRO* ACTIVITY OF CALCIUM SALT OF TRI 50C

TRI 50c calcium salt was assayed as an inhibitor of human α-thrombin by an amidolytic assay (J. Deadman et al, *J. Med. Chem.* 38:15111-1522, 1995, which reports a Ki value of 7nM for TRI 50b).
The inhibition of human α-thrombin therefore, was determined by the inhibition of the enzyme catalysed hydrolysis of three different concentrations of the chromogenic substrate S-2238.
200μl of sample or buffer and 50μl of S-2238 were incubated at 37°C for 1 minute and 50μl of human α-*thrombin* (0.25 NIHμ/ml) was added. The initial rates of inhibited and uninhibited reactions were recorded at 405nm. The increase in optical density was plotted according to the method of Lineweaver and Burke. The Km and apparent Km were determined and Ki was calculated using the relationship.

$$V = \frac{V\max}{1 + \frac{Km}{[S]} \cdot \left(1 + \frac{[I]}{Ki}\right)}$$

The buffer used contained 0.1M sodium phosphate, 0.2M NaCl, 0.5% PEG and 0.02% sodium azide, adjusted to pH 7.5 with orthophosphoric acid.
The samples consist of the compound dissolved in DMSO.
The reader is referred to Dixon, M and Webb, E.C., "Enzymes", third edition, 1979, Academic Press, the disclosure of which is incorporated herein by reference, for a further description of the measurement of Ki.
TRI 50c calcium salt was observed to have a Ki of 10nM.

EXAMPLE 31- PREPARATION OF MAGNESIUM SALT OF TRI 50C

TRI 50c (1.00g, 1.90mM) was dissolved in methanol (10ml) and stirred at room temperature. To this solution was added magnesium methoxide ($Mg(CH_3O)_2$) in methanol (1.05ml, 7.84 wt%). This solution was stirred for 2 hours at room temperature filtered and evacuated to 5ml. Water (25ml) was then added and the solution evacuated down to dryness to yield a white solid. This was dried over silica for 72 hours before being sent for microanalysis. Yield 760mg.
$^1$H NMR 300MHz, $\delta_H(CD_3C(O)CD_3)$ 7.14 - 7.22 (20H, m), 6.90 (2H, m), 4.89 (4H, m, $PhCH_2O$), 4.38 (2H, m), 3.40 (2H, br s), 2.73 - 3.17 (20H, broad unresolved multiplets), 1.05 - 2.10 (16H, broad unresolved multiplets).
$^{13}$C NMR 75MHz $\delta_c(CD_3C(O)CD_3)$ 206.56, 138.30, 130.76, 129.64, 129.31, 129.19, 129.09, 128.20, 128.04, 74.23, 73.55, 67.78, 58.76, 56.37, 56.03, 48.38, 47.87, 39.00, 25.42, 25.29.
FTIR (KBr disc) $\nu_{max}$ ($cm^{-1}$) 3331.3, 3031.4, 2935.3, 2876.9, 2341.9, 1956.1, 1711.6, 1639.9, 1534.3, 1498.1, 1453.0, 1255.3, 1115.3, 1084.6, 1027.6, 917.3, 748.9, 699.6, 594.9, 504.5, 467.8.

EXAMPLE 32 - SOLUBILITY OF TRI50C

The UV/visible spectra of TRI50c resulting from the procedure of Example 5 and its solubility were obtained as described above in relation to the salts. The solubility of TRI50c when dissolved at 50mg/ml was 8mM (4mg/ml).

EXAMPLE 33 - ANALYSIS OF SODIUM, CALCIUM, MAGNESIUM AND ZINC SALTS OF (R,S,R) TRI 50C

The following salts were prepared using a boronate:metal stoichiometry of n:1, where n is the valency of the metal, using (R,S,R) TRI 50c of higher chiral purity than that used to prepare the salts described in Examples 8, 11, 14, 18, 21, 24 and 29.

***A. Sodium Salt (Product of Example 9)***

| | | | | |
|---|---|---|---|---|
| **Analytical data** | | | | **Physical Properties** |
| HPLC or LC/MS: HPLC betabasic C18 Column, $CH_3CN$, Water | | | | Form: Amorphous solid<br>Colour: White |
| Estimated Purity: >95% by UV ($\lambda_{215nm}$) | | | | Melting Point: N/A |
| Micro analysis: | | | | |
| | | *Calcd.* | *Found.* | Solubility: Soluble in aqueous media ca~50mg/mi |
| C: | | 59.24 | 59.93 | |
| H: | | 6.44 | 6.47 | |
| N: | | 7.67 | 7.31 | $M_w$: 547.40 |
| Other: | B: | 1.98 | 1.91 | |
| | Na: | 4.20 | 3.81 | |

***B. Calcium Salt (Product of Example 26)***

| | | | |
|---|---|---|---|
| **Analytical data** | | | **Physical Properties** |
| HPLC or LC/MS: HPLC beta basic C18 Column, $CH_3CN$, Water | | | Form: Amorphous solid<br>Colour: White |
| Estimated Purity: >95% by UV ($\lambda_{215nm}$) | | | Melting Point: N/A |
| Micro analysis: | | | |
| | *Calcd.* | *Found.* | Solubility: Soluble in aqueous media ca~4mg/ml |
| C: | 59.27 | 55.08 | |
| H: | 6.48 | 6.43 | |
| N: | 7.71 | 7.08 | $M_w$: 1088.89 |
| Other: B: | 1.99 | 2.01 | |
| Ca: | 3.68 | 3.65 | |

***C. Magnesium Salt (Product of Example 31)***

| | |
|---|---|
| **Analytical data** | **Physical Properties** |
| HPLC or LC/MS: HPLC betabasic C18 Column, $CH_3CN$, Water | Form: Amorphous solid<br>Colour: White |
| Estimated Purity: >90% by UV ($\lambda_{215nm}$) | Melting Point: N/A |

Table continued

| Micro analysis: | | | | |
|---|---|---|---|---|
| | | *Calcd.* | *Found.* | Solubility: Soluble in aqueous media ca~7mg/ml |
| C: | | 60.44 | 57.25 | |
| H: | | 6.57 | 6.71 | |
| N: | | 7.83 | 7.45 | $M_w$: 1073.12 |
| Other: | B: | 2.01 | 2.02 | |
| | Mg: | 2.26 | 2.12 | |

***D. Zinc Salt (Product of Example 15)***

**Analytical data**

HPLC or LC/MS: HPLC betabasic C18 Column, $CH_3CN$, Water

Estimated Purity: >95% by UV ($\lambda_{215nm}$)

**Physical Properties**

Form: Amorphous solid

Colour: White

Melting Point: N/A

| Micro analysis: | | | | |
|---|---|---|---|---|
| | | *Calcd.* | *Found.* | Solubility: Soluble in aqueous media ca~2mg/ml |
| C: | | 58.21 | 56.20 | |
| H: | | 6.33 | 6.33 | |
| N: | | 7.54 | 7.18 | $M_w$: 1114.18 |
| Other: | B: | 1.94 | 1.84 | |
| | Zn: | 5.87 | 7.26 | |

*Notes:* The trigonal formula of the acid boronate is used in the calculated microanalyses. It is believed that a lower sodium salt solubility is reported in example 11 because the salt tested in example 11 had lower chiral purity.

**Conclusion**

The zinc, calcium and magnesium salts have all been prepared with a stoichiometry of one metal ion to two molecules of TRI 50c. The values found for the calcium and magnesium salts are dose to and thus consistent with those calculated for this 1:2 stoichiometry. For the zinc salt an excess of zinc was found; nonetheless, the zinc salt comprises a significant proportion of acid boronate. The sodium salt has been prepared with a stoichiometry of one metal ion to one molecule of TRI 50c. The value found for the sodium salt is dose to and thus consistent with that calculated for this 1:1 stoichiometry.

EXAMPLE 34 - STABILITY

An assay of TRI 50c and its sodium and lysine salts before and after drying.

**1. Tabulated Results**

**Table 1**

| Compound | Amount [μg/mL] | Purity (% area) |
|---|---|---|
| TRI 50c dry | 1000.0 | 82.00 |
| TRI 50c non-dried | 947.3 | 85.54 |
| TRI 50c Na salt dry | 1024 | 98.81 |
| TRI 50c Na salt non-dried | 1005.8 | 98.61 |

Table continued

| Compound | Amount [μg/mL] | Purity (% area) |
|---|---|---|
| TRI 50c Lys salt dry | 813.3 | 90.17 |
| TRI 50c Lys salt non-dried | 809.8 | 92.25 |

The purity of the acid was lowered by the drying process but the purity of the salts was less affected; the purity of the sodium salt was not significantly reduced. Large differences in response factors will reduce the actual impurity levels, however.

**2. Analytical procedure**

**2.1 Sample preparation**

TRI 50c and its Na, Li and Lys salts were weighed into HPLC vials and stored in a desiccator over phosphorus pentoxide for 1 week. For sample analysis, 5 mg of dried and non-dried material was weighed in a 5 mL volumetric flask and dissolved In 1 mL acetonitrile and filled up with demineralised water to 5 mL.

**3. Data evaluation**

The quantitative evaluation was performed using an HPLC-PDA method.

**4.Analytical parameters**

**4.1 Equipment and software**

| | |
|---|---|
| Autosampler | Waters Alliance 2795 |
| Pump | Waters Alliance 2795 |
| Column oven | Waters Alliance 2795 |
| Detection | Waters 996 diode array, MS-ZQ 2000 single quad |
| Software version | Waters Millennium Release 4.0 |

**4.2 Stationary phase**

| | |
|---|---|
| Analytical Column ID | S71 |
| Material | X-Terra™ MS $C_{18}$, 5 μm |
| Supplier | Waters, Eschbom, Germany |
| Dimensions | 150 mm x 2.1 mm (length, internal diameter) |

**4.3 Mobile phase**

Aqueous phase: A: $H_2O$ + 0.1%
Organic phase: C: ACN
Gradient conditions:

| Time | Flow | %A | %C |
|---|---|---|---|
| 0.00 | 0.5 | 90 | 10 |
| 27.0 | 0.5 | 10 | 90 |

Table continued

| Time | Flow | %A | %C |
|---|---|---|---|
| 27.1 | 0.5 | 90 | 10 |
| 30.0 | 0.5 | 90 | 10 |

This example indicates that the salts of the disclosure, particularly the metal salts, e.g. alkali metal salts, are more stable than the acids, notably TRI 50c.

EXAMPLE 35 - *IN-VITRO* ASSAY AS THROMBIN INHIBITOR OF MAGNESIUM SALT OF TRI 50C

Thrombin Amidolytic Assay

TRI 50c magnesium salt (TRI 1405) was tested in a thrombin amidolytic assay.

Reagents:

Assay Buffer:
100mM Na phosphate
200mM NaCl (11.688g/l)
0.5% PEG 6000 (5g/l)
0.02% Na azide
pH 7.5

Chromogenic substrate S2238 dissolved to 4mM (25mg + 10ml) in water. Diluted to 50uM with assay buffer for use in assay at 5μM. (S2238 is H-D-Phe-Pip-Arg-pNA).

Thrombin obtained from HTI, via Cambridge Bioscience, and aliquoted at 1mg/ml with assay buffer. Dilute to 100ng/ml with assay buffer and then a further 1 in 3 for use in the assay.

Assay:

110μl assay buffer

50ul 5μg/ml thrombin

20μl vehicle or compound solution

5 min at 37°C

20μl 50μM S2238

Read at 405nm at 37°C for 10minutes and record Vmax

**Results:**

The results are presented in Fig. 1.

**Discussion:**

In this assay the magnesium salt of TRI 50c shows the same activity as TRI 50b as an external control.

EXAMPLE 36 - TRI 50B INHIBITION OF PLATELET PROCOAGULANT ACTIVITY

Platelet pro-coagulant activity may be observed as the increase, in rate of activation of prothrombin by factor Xa in the presence of factor Va upon the addition of platelets pretreated with thrombin, caused by thrombin alone, collagen alone or a mixture of thrombin and collagen. This property is due to an increase in anionic phospholipid on the surface of the platelet with concomitant release of microveside from the surface. This is an essential physiological reaction and people whose platelets have reduced ability to generate procoagulant activity (Scott syndrome) show an increased tendency for bleeding.

**Method:**

Washed platelets were treated with either 1.15nM thrombin, 23μg/ml collagen or a mixture of both at the same concentration at 37°C. TRI 50b was added either for 1 minute prior to the addition of activator or immediately after the incubation with activator. Platelet procoagulant activity was determined as described previously (Goodwin C A et al, *Biochem J*. **1995** 8, 308: 15-21).

TRI 50b proved to be a potent inhibitor of platelet procoagulant activity with $IC_{50}$'s as summarised below:

Table 2: Influence of TRI 50b on the induction of platelet procoagulant activity by various agonists:

**Table 2**

| Agonist | Fold acceleration without TRI 50b | IC50 plus pre-Incubation (nM) | IC50 without incubation (nM) |
|---|---|---|---|
| Thrombin | 30 | 8 | 3000 |
| Collagen | 45 | 200 | 300 |
| Thrombin/Collagen | 110 | 3 | 80 |

Table 2 records, for example, that when platelets were treated with thrombin they caused a 30-fold acceleration of the rate of activation of prothrombin in comparison with control platelets. Treatment with TRI 50 reduced such acceleration by half at the various TRI 50 concentration levels given. The significant potency of TRI 50 is evidenced by the fact that the $IC_{50}$ values are in the nanomolar range.

TRI 50b does not have an effect on ADP, collagen or epinephrine induced aggregation of washed platelets.

EXAMPLE 37 - RABBIT EXTRACORPOREAL SHUNT MODEL

**Introduction**

The technique describes an animal model in which a platelet rich thrombus is produced. The activity of TRI 50b and heparin are compared.

The carotid artery and jugular vein of anaesthetised rabbits were used to create an extracorporeal circuit containing a suspended foreign surface (silk thread). Thrombus deposition is initiated by creation of high shear stress turbulent arterial blood flow, platelet activation, followed by coagulation in the presence of thrombogenic surfaces. Histopathological studies have shown that the thrombus is platelet rich.

**Material and Methods**

**Animals:**

NZW rabbits (males 2.5-3.5 kg) were used. The animals were allowed food and water up to the induction of anaesthesia.

**Anaesthesia:**

Animals were premedicated with fentanyl/fluanisone (Hypnorm) 0.15 ml total by intramuscular injection. General anaesthesia was induced with methohexitone (10 mg/ml) to effect, followed by endotracheal intubation. Anaesthesia was maintained with isoflurane (1-2.0 %) carried in oxygen /nitrous oxide.

**Surgical Preparation:**

The animals were placed in dorsal recumbency and the ventral cervical region prepared for surgery. The left carotid artery and right jugular vein were exposed. The artery was cannulated with a large Portex® catheter (yellow gauge), cut to a suitable length. The vein was cannulated with a Silastic® catheter. The shunt comprised of a 5 cm length of 'auto analyser' line (purple /white gauge). Joins to the shunt on the arterial side were made with intermediate size Silastic® tubing. The shunt was filled with saline before exposure to the circulation. The right femoral artery was cannulated for the measurement of blood pressure.

**Thread Preparation and insertion:**

The central section of the shunt contained a thread 3 centimetres in length. This consisted of 000 gauge Gutterman sewing silk so as to give four strands with a single knot at the end. (The knot section was outside the shunt).

**Blood Flow**

Blood flow velocity was determined by use of 'Doppler' probes (Crystal Biotech). A silastic probe was positioned over the carotid artery at the point of insertion of the arterial catheter. Flow was recorded on a chart recorder using heat sensitive paper.

**RESULTS**

**Table 3**

| TREATMENT | DOSE | THROMBUS WEIGHT AFTER 20 minute run | ANTITHROMBOTIC ACTIVITY |
|---|---|---|---|
| Control | N/A | 22.4 ±2.2 mg (n=5) | |
| TRI 50b | 10mg/kg iv | 9.78 ±1.9 mg (n=5) | Active |
| | 3.0mg/kg iv | 15.3 ±2.2 mg (n=5) | Active |
| HEPARIN | 100 u/kg iv | 22.9 ±1.65 mg (n=4) | Inactive |
| | 300 u/kg iv | 10.5 ±1.4 mg (n=4) | Active (Severe bleeding) |

**Discussion**

Table 3 shows that, under high arterial shear conditions, a TRI 50b dose of 3mg/kg to 10mg/kg iv significantly inhibits thrombus formation without bleeding, whereas a heparin dose within the normal clinical range for treating venous thrombosis (100u/kg iv heparin) was ineffective. The higher dose of heparin, though active, caused severe bleeding. These results, which show TRI 50b effectively inhibiting arterial thrombosis without causing bleeding, are consistent with TRI 50b inhibiting platelet procoagulant activity. In contrast, the thrombin inhibitor heparin, when administered at an approximately equi-effective dose (in terms of inhibition of arterial thrombosis), produced the severe bleeding normal when thrombin inhibitors are used to treat arterial thrombosis.

<u>EXAMPLE 38 - COMPARISON OF BLEEDING TIMES</u>

The aim of the study was to compare the bleeding times of heparin with TRI 50b in a suitable model. It is accepted that heparin is a poor inhibitor of platelet procoagulant activity (*J. Biol. Chem.* **1978** Oct 10; 253(19):6908-16; Miletich JP, Jackson CM, Majerus PW1: *J. Clin. Invest.* **1983** May; 71(5):1383-91).

Bleeding times were determined in a rat tail bleeding model following intravenous administration of heparin and TRI 50b. The doses employed were chosen on the basis of their efficacy in the rat Wessler and dynamic models and were as follows:

TRI 50b: 5 and 10 mg/kg
Heparin: 100 units/kg

## MATERIALS AND METHODS

### Anaesthesia

Rats were anaesthetised with sodium pentabarbitone at 60 mg/kg (2.0 ml/kg of 30 mg/ml solution by i.p. injection). Supplemental anaesthetic was given i.p. as required.

### Surgical preparation

A jugular vein was cannulated for the administration of test compound. The trachea was also cannulated with a suitable cannula and the animals allowed to breathe 'room air' spontaneously.

### Compound administration

These were given in the appropriate vehicle at 1.0 ml/kg intravenously. Heparin was administered in saline, whilst TRI 50b was dissolved in ethanol, and then the resultant solution added to water for injection (1 part ethanol to 5 parts water).

### Technique

Two minutes following compound administration the distal 2mm of the animal's tail was sectioned with a new scalpel blade and the tail immersed in warm saline (37°C) contained in a standard 'universal' container, so that the blood stream was clearly visible. The bleeding time recording was started immediately following transection until the cessation of blood flow from the tip of the tail. A period of 30 seconds was allowed after the blood flow from the tail had stopped to ensure that bleeding did not re-commence, if bleeding did start again the recording time was continued for up to a maximum of 45 minutes.

### Results

Table 4 gives a summary of the bleeding results and shows the increases above base line values.

### Table 4

Summary table of bleeding results

| Treatment | Bleeding time min (± SEM†) |
|---|---|
| Saline | 5.1± 0.6 |
| Heparin 100 u/kg iv | >40* |
| TRI 50b 5 mg/kg iv | 11.3 ± 1.2 |
| TRI 50b 10 mg/kg iv | 30.4 ± 5.2 |
| *Severe bleeding in all animals, with no cessation after 40 minutes. †SEM = standard error of the mean | |

### Discussion

The results show that TRI 50b was superior to heparin (produced less bleeding) at all doses. It should be noted that when 100 u/kg heparin is compared with 5 mg/kg TRI 50b, heparin-treated animals bled more extensively than those receiving TRI 50b; it was previously established (Example 25) that heparin at a dose of 100 u/kg is a less effective inhibitor of arterial thrombosis than TRI 50b at a dose of 3.0 mg/kg. Heparin is primarily a thrombin inhibitor and a poor inhibitor of platelet procoagulant activity; the results are therefore consistent with TRI 50b exerting anti-coagulant activity by inhibition of platelet coagulant activity in addition to thrombin inhibiting activity.

EXAMPLE 39 - TRI 50B AS A PRODRUG FOR TRI 50C: PHARMACOKINETICS AND ABSORPTION

**MATERIALS AND METHODS**

**Animals**

Rats, body weight circa 250-300g, were used. The animals were fasted only on the day of use for the iv stage.

**Table 5:** iv phase

| Treatment | Dose mg/kg iv | n |
|---|---|---|
| TRI 50b | 1.0mg/kg | 3 |
| TRI 50c | 1.0mg/kg | 3 |

**Dose**

**Formulation (TRI 50b/TRI 50c)**

These were dosed in a formulation prepared as follows: 48 mg/ml of TRI 50b is dissolved in ethanol: PEG 300 (2:3 vol: vol). Just before administration, 5 volumes of this solution are mixed with 3 volumes of 5% kollidon 17 8F.

**i.v. Phase**

Both compounds were given at a dose of 1.0mg/kg iv.
The compounds were dosed in a PEG/ethanol/kollidon formulation which was prepared immediately before, as described immediately under the heading "Dose": Stock 15.0mg/ml. This was dosed at 1.33ml/kg (equivalent to 30mg/kg).

**Blood sampling**

A pre dose sample was taken followed by: 0, 2, 5, 10, 20, 30, 40, 60 and 90 minutes post dose.

**Plasma**

This was obtained by centrifugation (3000 RPM for 10 min) and stored at -20°C prior to analysis.

**RESULTS**

**PHARMACOKINETIC ANALYSIS**

**Table 6:** i.v. pharmacokinetic data

| | TRI 50b | TRI 50c |
|---|---|---|
| Elimination half life: minutes | 35 minutes | 36.6 minutes |
| Area under curve | 1.68 | 1.48 |
| Mean Residence Time | 46 minutes | 45 minutes |
| Clearance: ml/min/kg | 10 | 11.3 |
| Volume Distribution Litres/kg | 0.5 | 0.59 |
| Max Plasma Concentration (observed) | 2.24 | 2.35 |

The following results are represented in Figure 2:

Fig 2: intravenous phase clearance and kinetics following a single dose of TRI 50b or its free acid (TRI 50c). The figure shows the observed assay data.

**CONCLUSION**

The i.v. kinetics were similar for both TRI 50b and TRI 50c. The data are consistent with TRI 50b being rapidly hydrolysed in plasma to TRI 50c and with TRI 50c being the active principle.
The results of examples 36 to 39 indicate that administration of TRI 50c as a salt will provide a way to treat arterial thrombosis and/or venous thrombosis.

EXAMPLE 40 - INTRAVENOUS ADMINISTRATION OF TRI 50C SODIUM SALT

The pharmacokinetics (PK) and pharmacodynamics (PD) of TRI 50c sodium salt were studied in beagle dogs following intravenous administration.
The PD was measured as thrombin time and APTT using an automated coagulometer. Plasma concentrations were measured using an LCMS /MS method.
TRI 50c monosodium salt (108.8g) was dissolved in 0.9% sodium chloride (100ml) and dosed i.v. at 1.0 mg/kg (1.0 ml/kg over 30 seconds). Blood samples were taken into 3.8% tri-sodium citrate (1 + 8) at pre dose, 2, 5, 10, 20, 30, minutes post dose and then at 1, 2, 3, 4, 6, 8, 12 and 24 hours post dose. Plasma was prepared by centrifugation and frozen at minus 20°C pending analysis.

**RESULTS**

The sodium salt was tolerated well with no adverse events for the total duration of the study.
Male and female dogs responded similarly with a pharmacodynamic C max: at 2 minutes (thrombin time of 154 seconds raised from a base line of 14.3 seconds). Thrombin time was 26 seconds at one hour post dose.
There was an exceptionally good therapeutic ratio between the APTT and thrombin dotting time in dogs receiving the sodium salt at a dose of 1.0 mg/kg i.v. Thrombin dotting time was elevated 10.8 times above base line (154.4 seconds from14.3 seconds) two minutes following dosing, compared to only 1.3 times elevation in the APTT (19 seconds to 25 seconds post dose).

EXAMPLE 41 - HUMAN CLINICAL STUDIES

In human clinical volunteer studies with doses of up to 2.5mg/kg i.v. (dosages which significantly prolong the thrombin dotting time), TRI 50b had no effect on Simplate bleeding time (i.e. bleeding time measured using a Simplate® bleeding time device).

EXAMPLE 42 - RESIDUAL n-HEPTANE OF TRI 50C CALCIUM SALT

Salt prepared following the methods of Examples 1 and 3 was tested by headspace gas chromatography. Data are shown below:

| **Residual solvents: Headspace gas chromatography** | |
|---|---|
| **GC Parameter:** | |
| Column: | DB-wax, 30 m, 0.32 mm ID, 5μ |
| Carrier Gas: | Helium 5.0, 80 kPas |
| Detector: | FID, 220°C |
| Injector Temp: | 150°C |
| Operating Conditions: | 35°C/7 min; 10°C/ min up to 80°C/2 min; 40°C up to 180°C/2 min |
| Injection volume: | 1 ml |
| Split: | On |
| **Headspace Parameter:** | |
| Oven temperature: | 70°C |
| Needle temperature: | 90°C |
| Transfer temperature: | 100°C |

Table continued

| Residual solvents: Headspace gas chromatography | |
|---|---|
| **GC Parameter:** | |
| Other parameters: | temper time: 15 min, GC-cycle time: 28 min, injection time: 0.03 min, duration: 0.4 min |

| Calibration Standards: sample weight/dilution | | | | |
|---|---|---|---|---|
| | | | | |
| **standard** | **weight (mg)** | **volume (ml)** | **concentration (m4/ml)** | **area (average, n=3)** |
| n-heptane | 103.12 | 100 | 1.0312 | 2757.74756 |
| **sample no.** | **weight (mg)** | **volume (ml)** | **concentration (mg/ml)** | |
| 1 | 100.84 | 5 | 20.17 | |
| 2 | 99.12 | 5 | 19.82 | |
| 3 | 100.03 | 5 | 20.01 | |

| n-heptane | | | |
|---|---|---|---|
| | **sample** | **concentration (mg/ml)** | **content (%)** |
| | 1 | 0.0010 | 0.0048 |
| | 2 | 0.0009 | 0.0044 |
| | 3 | 0.0010 | 0.0050 |
| | | **0.00095** | **0.005** |

EXAMPLE 43- HPLC CHROMATOGRAMS

TRI 50c monosodium salt made by the method of Examples 1, 2 & 3 and TRI 50c hemicalcium salt made by the method of Examples 1, 2 & 4 were analysed by HPLC chromatography.

***1. Method***

**1.1 Equipment and software**

| | |
|---|---|
| Autosampler | Waters Alliance 2795 |
| Pump | Waters Alliance 2795 |
| Column oven | Waters Alliance 2795 |
| Detection | Waters 2996 diode array, MS-ZQ single quad |
| Software version | Waters Millennium 4.0 |

**1.2 Stationary phase**

| | |
|---|---|
| Analytical Column ID | S-71 |
| Material | XTerra™ MS $C_{18}$, 5 µm |
| Supplier | Waters, Eschborn, Germany |

Table continued

| | |
|---|---|
| Dimension | 150 mm x 2.1 mm (length, ID) |
| Pre-column ID | no pre-column |

Xterra MS $C_{18}$, 5 µm is a column packing material supplied by Waters Corporation, 34 Maple Street, Milford, MA 01757, US and local offices, as in years 2002/2003. It comprises hybrid organic/inorganic particles, consisting of spherical particles of 5 µm size, 125 Å pore size and 15.5% carbon load.

### 1.3 Mobile Phase

| | |
|---|---|
| Aqueous phase: | A: $H_2O$ + 0.1% HCOOH |
| Organic phase: | C: ACN |

$H_2O$ = $H_2O$ by Ultra Clear water purification system
ACN = gradient grade acetonitrile

Gradient conditions

| time [min] | A% | C% | flow [mL/min] | gradient shape |
|---|---|---|---|---|
| 0.0 | 90.0 | 10.0 | 0.5 | |
| 27.00 | 10.0 | 90.0 | 0.5 | linear |
| 27.10 | 90.0 | 10.0 | 0.5 | linear |
| 30.00 | 90.0 | 10.0 | 0.5 | linear |

### 1.4 Instrumental Parameters

| | |
|---|---|
| Flow | 0.5 $mL\text{-}min^{-1}$ |
| Temperature | 40±5°C |
| HPLC control | Waters Millennium Release 4.0 |
| Calculation | Waters Millenium 4.0 |

## *2. Parameters*

### 2.1 Wavelength/Retention time/Response factors

Table: retention and detection parameter (k' F: 0.5 ml/min, t0 = 0.9 ml/min)

| Substance | RetTime [min] | λ [nm] | m/z | response factor [area/µg] | Reciprocal Response factor |
|---|---|---|---|---|---|
| TRI 50c | 11.68 | 258 | 508.33 | 660 | 1 |
| Benzyl alcohol | 3.862 | 258 | n.d. | 1960 | 0.337 |
| Benzaldehyde | 6.13 | 258 | n.d. | 79939 | 0.0083 |
| Benzoic acid | 5.52 | 258 | n.d. | 5967 | 0.111 |
| Impurity I | 11.18 | 258 | 396.17 | 886 | 0.745 |
| Impurity II | 13.39 | 258 | 482.22 | 552 | 1.196 |

**2.2 Linearity**

**Linearity Range 4000- 10 μg/mL (detection UV 258 nm)**

Table Unearity data UV 258nm

| calibration solution | area [μAU's] | target conc. [μg/mL] | conc. found[1] [μg/mL] |
|---|---|---|---|
| Tri 50c | 5353 | 10 | 20.44 |
| Tri 50c | 5301 | 10 | 20.37 |
| Tri 50c | 65809 | 100 | 113.35 |
| Tri 50c | 66365 | 100 | 114.17 |
| Tri 50c | 172019 | 250 | 270.43 |
| Tri 50c | 162587 | 250 | 256.48 |
| Tri 50c | 339503 | 500 | 518.13 |
| Tri 50c | 326912 | 500 | 499.51 |
| Tri 50C | 659257 | 1000 | 991.02 |
| Tri 50c | 647495 | 1000 | 973.63 |
| Tri 50c | 1322371 | 2000 | 1971.72 |
| Tri 50c | 1305196 | 2000 | 1946.32 |
| Tri 50c | 2724410 | 4000 | 4045.24 |
| [1] recalculated with linear equation | | | |

**Linear equation parameters:**

Y = 6.75e+002 X - 8.45e+003
r = 0.99975
$r^2$ = 0.99950

**Linearity Range 10 - 0,10 μg/mL (detection SIR m/z 508,33)**

Table: Linearity data SIR 508.33

| calibration solution | mean area [μAU's] | target conc. [μg/mL] | conc. found[1] [μg/mL] |
|---|---|---|---|
| Tri 50c | 2188860 | 0.01 | 0.022 |
| Tri 50c | 2702839 | 0.01 | 0.045 |
| Tri 50c | 3817226 | 0.1 | 0.094 |
| Tri 50c | 3833799 | 0.1 | 0.095 |
| Tri 50c | 23153550 | 1 | 0.947 |
| Tri 50c | 24646892 | 1 | 1.013 |
| Tri 50c | 223007852 | 10 | 9.765 |

Table continued

| calibration solution | mean area [μAU's] | target conc. [μg/mL] | conc. found[1] [μg/mL] |
|---|---|---|---|
| Tri 50c | 233753043 | 10 | 10.239 |
| [1] recalculated with linear equation | | | |

**Equation parameter**

$Y = 2.27e{+}007\ X + 1.69e{+}006$
$r = 0.99958$
$r^2 = 0.99916$

**2.3 Quantitation limit**

The quantitation limit was determined using the signal to noise ratio criterion S/N > 19,

UV 258 nm: 10 μg/mL
M/z 508.3: 0.1 μg/mL

**2.4 Precision**

| Injection | Target concentration [μg/mL] | Area | Amount [μg/mL] | Retention time [min] |
|---|---|---|---|---|
| 1 | 250 | 165805 | 261.24 | 11.690 |
| 2 | 250 | 168644 | 265.44 | 11.662 |
| 3 | 250 | 167858 | 264.27 | 11.686 |
| 4 | 250 | 166947 | 262.93 | 11.692 |
| 5 | 250 | 166925 | 262.89 | 11.679 |
| 6 | 250 | 166294 | 261.96 | 11.696 |
| Mean | | 167079 | 263.12 | 11.684 |
| Std. Dev. | | 1033 | 1.528 | 0.01 |
| %RSD | | 0.6 | 0.6 | 0.1 |

**2.5 Robustness**

Table: robustness data; Standard 250 μg/mL aqueous solution (containing < 1% ACN)

| calibration solution | temp./time [°C/h] | area [μAU's] | recovery [%] |
|---|---|---|---|
| 250μg/mL Tri50c | - | 172020 | - |
| 250μg/mL Tri50c | 4°C. 16h | 166294 | 96.67 |
| 2.5μg/mL TRI50c - | | 88034891 | - |
| 2.5μg/mL TRI50c | 37°C. 4h | 88833175 | 100.9 |

**References**

1. ICH HARMONISED TRIPARTITE GUIDELINE. TEXT ON VALIDATION OF ANALYTICAL PROCEDURES Recommended for Adoption at Step 4 of the ICH Process on 27 October 1994 by the ICH Steering Committee

2. FDA Reviewer Guidance. Validation of chromatographic methods. Center for Drug Evaluation and Research. Nov. 1994

3. USP 23. <621> Chromatography

4. L. Huber. Validation of analytical Methods. LC-GC International Feb. 1998

5. Handbuch Validierung in der Analytik. Dr. Stavros Kromidas (Ed.) Wiley-VCH Verlag. 2000. ISBN 3-527-29811-8

***3. Results***

<u>3.1 Sample Name: TRI 50c monosodium salt</u>

<u>Infection volume: 10μL</u>

| **Name** | **Ret Time (Min)** | **Area %** | **Area [μAU's]** | **Peak Height μAU** |
|---|---|---|---|---|
| TRI 50c | 12.136 | 100.0000 | 604.27228 | 32.05369 |

<u>3.2 Sample Name: TRI 50c hemicalcium salt</u>

<u>Injection volume: 10μL</u>

| **Name** | **Ret Time (Min)** | **Area %** | **Area [μAU's]** | **Peak Height μAU** |
|---|---|---|---|---|
| TRI 50c | 12.126 | 100.0000 | 597.11279 | 32-29640 |

The disclosed methods have been used to obtain salts substantially free of C-B bond degradation products, in particular salts containing no such products in an amount detectable by HPLC, specifically the method of Example 43. The disclosed methods have been used to obtain salts substantially free of Impurity I, in particular containing no Impurity I in an amount detectable by HPLC, specifically by the method of Example 43. The disclosed methods have been used to obtain salts substantially free of Impurity IV, in particular containing no Impurity IV in an amount detectable by HPLC, specifically by the method of Example 43.

<u>EXAMPLE 44 - DETERMINATION OF DIASTEREOMERIC EXCESS</u>

TRI 50b, crude, contains three chiral centres. Two of them are fixed by the use of enantiomerically pure amino acids ((R)-Phe and (S)-Pro). The third one is formed during the synthesis. The favoured epimer is the desired TRI 50b, Isomer I (R,S,R-TRI 50b). Both epimers of TRI 50b are dearly baseline separated by the HPLC method, thus allowing determination of the diasteromeric excess (de) of TRI 50b.

TRI 50d is not stable under the conditions applied for HPLC purity determination, but decomposes rapidly on sample preparation to TRI 50c, so that TRI 50d and TRI 50c show the same HPLC traces.

The two isomers of TRI 50c are not baseline separated in HPLC, but both isomers are clearly visible. This becomes obvious, when TRI 50b, crude (mixture of both isomers) is converted with phenylboronic acid to TRI 50c, crude. Both isomers of TRI 50c are observed in HPLC nearly at the same relation as before in TRI 50b, crude.

Upon synthesis of TRI 50d from TRI 50b, crude, only one diastereoisomer is precipitated. In this case HPLC shows only one peak for TRI 50c, where a very small fronting is observed. Precipitation from dichloromethane/diethylether

removes the fronting efficiently. The level of removal of Isomer II cannot be quantified by this HPLC method. Therefore samples before reprecipitation and after one and two reprecipitations were esterified with pinacol and the resulting samples of TRI 50b analysed by HPLC. Thus a de of 95.4% was determined for the crude sample. The reprecipitated sample resulted in a de of 99.0% and finally the sample that was reprecipitated twice showed a de of 99.5%.

These results clearly show the preferred precipitation of Isomer I, whereas Isomer II remains in solution.

It will be appreciated from the foregoing that the disclosure provides boronic acid salts useful for pharmaceutical purposes and which feature one or more of the following attributes: (1) improved hydrolytic stability; (2) improved stability against deboronation; and (3), in any event, not suggested by the prior art

**Claims**

1. A parenteral pharmaceutical formulation comprising a pharmaceutically acceptable base addition salt of a boronic acid of formula (I):

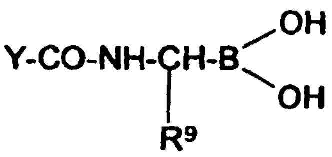

(I)

wherein
Y comprises a hydrophobic moiety which, together with the aminoboronic acid residue -NHCH($R^9$)-B$(OH)_2$, has affinity for the substrate binding site of thrombin; and
$R^9$ is a straight chain alkyl group interrupted by one or more ether linkages and in which the total number of oxygen and carbon atoms is 3, 4, 5 or 6 or $R^9$ is $-(CH_2)_m$-W where m is 2, 3, 4 or 5 and W is -OH or halogen (F, Cl, Br or I), and, when YCO- is an optionally N-terminally protected dipeptide residue, the peptide linkages in the acid are optionally and independently N-substituted by a $C_1$-$C_{13}$ hydrocarbyl group optionally containing in-chain or in-ring nitrogen, oxygen or sulfur and optionally substituted by a substituent selected from halo, hydroxy and trifluoromethyl.

2. A formulation of claim 1 wherein $R^9$ is an alkoxyalkyl group.

3. A formulation of claim 1 or claim 2 wherein all the peptide linkages in the acid are unsubstituted.

4. A formulation of claim 1 or claim 2 wherein said $C_1$-$C_{13}$ hydrocarbyl group is a $C_1$-$C_6$ saturated hydrocarbyl group.

5. A formulation of any of claims 1 to 4 wherein YCO- comprises an amino acid which binds to the S2 subsite of thrombin, the amino acid being N-terminally linked to a moiety which binds the S3 subsite of thrombin.

6. A formulation of any of claims 1 to 4 wherein YCO- is an optionally N-terminally protected dipeptide which binds to the S3 and S2 binding sites of thrombin.

7. A formulation of claim 6 wherein said dipeptide is N-terminally protected.

8. A formulation of claim 6 or claim 7 wherein Y comprises a P3 residue which has a side chain of formula (B):

$$-(CO))_a-(CH_2)_b-D_c-C_e(E^1)(E^2)(E^3) \qquad (B)$$

wherein
a is 0 or 1;
e is 1;
b is 0 or an integer such that (b+e) is from 1 to 4;
cis 0 or 1;
Dis O or S;
$E^1$, $E^2$ and $E^3$ are each independently selected from $-R^{15}$ and J-$R^{15}$, where J is a 5-6 membered ring and $-R^{15}$ is selected from $C_1$-$C_6$ trialkylsilyl, -CN, $-R^{13}$, $-R^{12}OR^{13}$, $-R^{12}COR^{13}$, $-R^{12}CO_2R^{13}$, $-R^{12}O_2CR^{13}$. and one or two hal-

ogens, wherein $R^{12}$ is $-(CH_2)_f-$ and $R^{13}$ is $-(CH_2)_gH$ wherein f and g are each independently from 0 to 10, provided that (f+g) is 1, 2, 3 or 4.

**9.** A formulation of claim 8 wherein, when $E^1$, $E^2$ or $E^3$ contains an $R^{13}$ group, g is 0 or 1.

**10.** A formulation of any of claims 6 to 9 wherein YCO- comprises a P2 residue which is a residue of an imino acid.

**11.** A formulation of any of claims 6 to 10 wherein the dipeptide residue comprises a P3 amino acid residue of (R)-configuration and a P2 residue of (S)-configuration, and wherein the residue $-NHCH(R^9)-B(OH)$ is of (R)-configuration.

**12.** A formulation of any of claims 1 to 11 wherein the boronic acid has a Ki for thrombin of about 100 nM or less.

**13.** A formulation of claim 12 wherein the boronic acid has a Ki for thrombin of about 20 nM or less.

**14.** A formulation of claim 1 wherein the boronic acid is of formula (11):

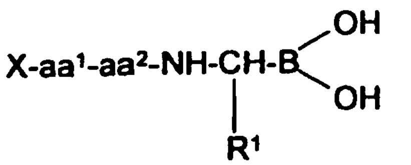

(II)

where:

X is H (to form $NH_2$) or an amino-protecting group;
$aa^1$ is an amino acid having a hydrocarbyl side chain containing no more than 20 carbon atoms and comprising at least one cyclic group having up to 13 carbon atoms;
$aa^2$ is an imino acid having from 4 to 6 ring members or is Gly N-substituted by a $C_3$-$C_{13}$ hydrocarbyl group;
$R^1$ is a group of the formula $-(CH_2)_S-Z$, where s is 2, 3 or 4 and Z is -OH, -OMe, -OEt or halogen (F, Cl, Br or I).

**15.** A formulation of claim 14 wherein $aa^1$ is selected from Phe, Dpa and wholly or partially hydrogenated analogues thereof.

**16.** A formulation of claim 14 wherein $aa^1$ is selected from Dpa, Phe, Dcha and Cha.

**17.** A formulation of any of claims 14 to 16 wherein $aa^1$ is of (R)-configuration.

**18.** A formulation of daim 14 wherein $aa^1$ is (R)-Phe or (R)-Dpa.

**19.** A formulation of daim 14 wherein $aa^1$ is (R)-Phe.

**20.** A formulation of any of daims 14 to 19 wherein $aa^2$ is a residue of an imino acid of formula (IV)

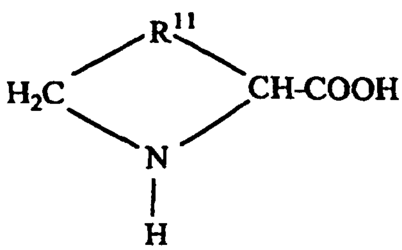

(IV),

where $R^{11}$ is $-CH_2-$, $-CH_2-CH_2-$, $-S-CH_2-$, $-S-C(CH_3)_2-$ or $-CH_2-CH_2-CH_2-$, which imino acid, when the ring is 5- or 6- membered, is optionally substituted at one or more $-CH_2-$ groups by from 1 to 3 $C_1$-$C_3$ alkyl groups.

**21.** A formulation of any of claims 14 to 20 wherein $aa^2$ is of (S)-configuration.

**22.** A formulation of claim 20 wherein $aa^2$ is an (S)-proline residue.

**23.** A formulation of claim 14, wherein $aa^1$-$aa^2$ is (R)-Phe-(S)-Pro.

**24.** A formulation of any of claims 14 to 23 wherein the residue $-NH-CH(R^1)-B(OH)_2$ is of (R)-configuration.

**25.** A formulation of any of daims 14 to 24 wherein $R^1$ is 2-bromoethyl, 2-chloroethyl, 2-methoxyethyl, 3-bromopropyl, 3-chloropropyl or 3-methoxypropyl.

**26.** A formulation of any of claims 14 to 24 wherein $R^1$ is 3-methoxypropyl.

**27.** A formulation of claim 14 wherein the boronic acid is of formula (VIII):

$$X\text{-(R)-Phe-(S)-Pro-(R)-Mpg-B(OH)}_2 \qquad \text{(VIII).}$$

**28.** A formulation of any of claims 14 to 27 where X is $R^6$-$(CH_2)_p$-C(O)-, $R^6$-$(CH_2)_p$-$S(O)_2$-, $R^6$-$(CH_2)_p$-NH-C(O)- or $R^6$-$(CH_2)$p-O-C(O)- wherein p is 0, 1, 2, 3, 4, 5 or 6 and $R^6$ is H or a 5 to 13-membered cyclic group optionally substituted by 1, 2 or 3 substituents selected from halogen, amino, nitro, hydroxy, a $C_5$-$C_6$ cyclic group, $C_1$-$C_4$ alkyl and $C_1$-$C_4$ alkyl containing, and/or linked to the cyclic group through, an in-chain O, the aforesaid alkyl groups optionally being substituted by a substituent selected from halogen, amino, nitro, hydroxy and a $C_5$-$C_6$ cyclic group.

**29.** A formulation of claim 28 wherein said 5 to 13-membered cyclic group is aromatic or heteroaromatic.

**30.** A formulation of claim 29 wherein said 5 to 13-membered cyclic group is phenyl or a 6-membered heteroaromatic group.

**31.** A formulation of any of claims 28 to 30 wherein X is $R^6$-$(CH_2)_p$-O-C(O)- and p is 0 or 1.

**32.** A formulation of any of claims 14 to 26 wherein X is benzyloxycarbonyl.

**33.** A formulation of claim 14 wherein the boronic acid is Cbz-(R)-Phe-(S)-Pro-(R)-Mpg-$B(OH)_2$.

**34.** A formulation of any of claims 1 to 33 wherein the salt does not comprise a choline or ammonium salt.

**35.** A formulation of any of claims 1 to 34 wherein the salt comprises boronate ions derived from the boronic acid and monovalent counter-ions.

**36.** A formulation of any of claims 1 to 34 wherein the salt is a salt of the peptide boronic acid with an alkali metal or a strongly basic organic nitrogen-containing compound.

**37.** A formulation of claim 36 wherein the strongly basic organic nitrogen compound has a pKb of about 7 or more, e.g. about 7.5 or more.

**38.** A formulation of any of claims 1 to 35 wherein the salt is a salt of the boronic acid with a metal.

**39.** A formulation of any of claims 1 to 33 or 37 wherein the salt is a salt of the boronic acid with an alkali metal, an aminosugar, a guanidine or an amine of formula (XI):

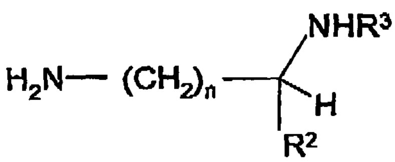

(XI)

where n is from 1 to 6, $R^2$ is H, carboxylate or derivatised carboxylate, $R^3$ is H, $C_1$-$C_4$ alkyl or a residue of a natural or unnatural amino acid.

**40.** A formulation of any of claims 1 to 33 wherein the salt is an L-arginine salt.

**41.** A formulation of any of claims 1 to 33 wherein the salt is an L-lysine salt.

**42.** A formulation of any of claims 1 to 33 wherein the salt is a divalent metal salt.

**43.** A formulation of any of claims 1 to 33 wherein the salt is a sodium salt.

**44.** A formulation of any of daims 1 to 33 wherein the salt is a lithium salt.

**45.** A formulation of any of claims 1 to 33 wherein the salt is a glucamine salt, e.g. is an N-methyl-D-glucamine salt.

**46.** A formulation of any of claims 1 to 33 wherein the salt is a calcium or magnesium salt.

**47.** A formulation of any of claims 1 to 46 wherein the salt comprises boronate ions derived from the peptide boronic acid and has a stoichiometry consistent with the boronate ions carrying a single negative charge.

**48.** A formulation of any of claims 1 to 47 wherein the salt comprises a boronate ion derived from the peptide boronic acid and a counter-ion and wherein the salt consists essentially of a salt having a single type of counter-ion.

**49.** A formulation of any of claims 1 to 33 wherein the salt is a monolithium salt.

**50.** A formulation of any of claims 1 to 32 wherein the salt is a hemicalcium salt.

**51.** A formulation of any of daims 1 to 32 wherein the salt is a monosodium salt

**52.** A formulation of claim 1 wherein the salt is the monosodium salt of Cbz-(R)-Phe-(S)-Pro-(R)-Mpg-$B(OH)_2$.

**53.** A formulation of claim 1 wherein the salt is the hemicalcium salt of Cbz-(R)-Phe-(S)-Pro-(R)-Mpg-$S(OH)_2$.

**54.** A formulation of any of claims 1 to 53 which further comprises a pharmaceutically acceptable diluent, excipient or carrier.

**55.** A formulation of any of daims 1 to 54 wherein the salt comprises the boronate in anhydride form.

**56.** A formulation of any of claims 1 to 55 which is adapted for intravenous administration.

**57.** A formulation of claim 56 which includes a tonicity agent.

**58.** A formulation of claim 56 or claim 57 which is in a form requiring reconstitution before administration.

**59.** A formulation of claim 56 or daim 55 which is in the form of a solution.

**60.** A formulation of any of claim 1 to 35 which is adapted for intravenous administration and comprises a solution containing as the salt the following first and second species and optionally other components, for example a tonicity agent or a buffer:

a) a first species selected from (a) boronic acids of formula (I) or, as the case may be, formula (II), (b) boronate anions thereof, and (c) any equilibrium form of the aforegoing (e.g. anhydride); and
b) a second species selected from the group consisting of pharmaceutically acceptable alkali metal ions and basic organic nitrogen-containing compounds having a pKb of about 7 or more (which compounds will be understood to comprise the compounds in protonated form),

wherein the formulation has an observed stoichiometry which would be consistent with the first species being boronate ions carrying a single negative charge,

**61.** A formulation of claim 60 which has a Ki for thrombin of about 20 nM or less,

**62.** A formulation of any of claims 1 to 61 which contains a trace amount of an aliphatic or cycloaliphatic solvent.

**63.** A formulation of claim 62 wherein the solvent is an n-alkane, optionally n-heptane.

**64.** A formulation of any of claims 1 to 63 wherein $R^9$ (in the case of a formulation containing a formula (I) boronic acid salt) or $R^1$ (in the case of a formulation containing a formula (II) boronic acid salt) is an alkoxyalkyl group which may be represented as $R^Z$-O-$R^Y$- and the formulation is free of an impurity boronate species whose structure corresponds to that of the formula (I) or formula (II) species when $R^9$ or, as the case may be, $R^1$ is replaced by an alkyl group $R^Y$.

**65.** A formulation of any of claims 1 to 64 which is substantially free of any degradation product resulting from C-B bond cleavage.

**66.** A formulation of claim 14 or any of claims 15 to 65 in combination with claim 14 in which boronate ions in which $aa^1$ is of (R)-configuration, $aa^2$ is of (S)-configuration and the residue -NH-CH($R^1$)-B$(OH)_2$ is of (R)-configuration are in a diastereomeric excess of 99% or more, e.g. 99.5% or more over the (R,S,S) isomer.

**67.** A formulation of claim 33 or of any of claims 34 to 63 or 66 in combination with daim 33 which is substantially free of the compound:

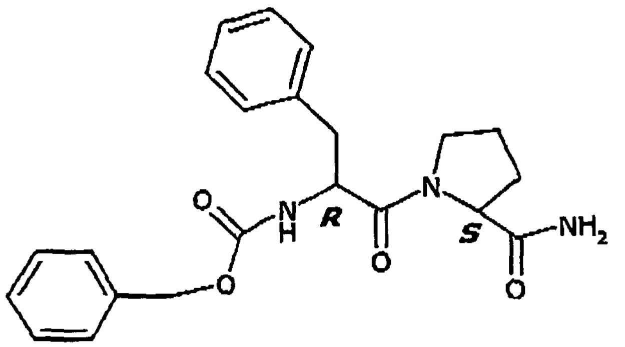

**68.** A formulation of claim 33 or any of daims 34 to 63 or 66 in combination with daim 33 or of claim 67 which is substantially free of the compound:

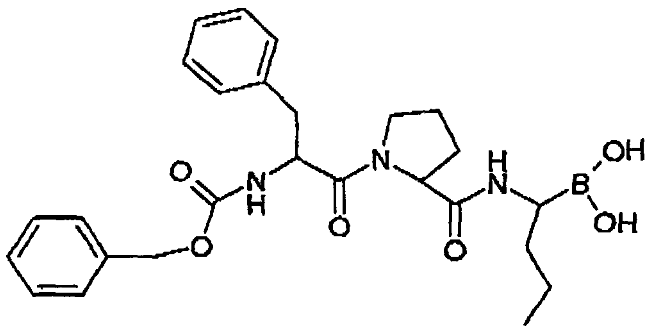

and its equilibrium forms.

**69.** A product for use as a parenteral pharmaceutical comprising a salt as defined in any of claims 1 to 51.

**70.** A product of claim 68 wherein the salt comprises boronic groups of the acid in anhydride form.

**71.** The use of a salt as defined in any of claims 1 to 51, or of a salt as defined in any of said claims and having the

characteristics recited in one or a permitted combination of claims 61 to 67, for the manufacture of a parenteral medicament for treating thrombosis by way of prophylaxis or therapythe medicament optionally being for use in an anticoagulant treatment when blood is in contact with a medical device outside the body, such as during cardiovascular surgery using a heart-lung machine or in haemodialysis.

**72.** The use of a salt as defined in any of claims 1 to 53, or of a salt as defined in any of said claims and having the characteristics recited in one or a permitted combination of claims 62 to 68, for the manufacture of a parenteral medicament for preventing thrombosis in a haemodialysis circuit of a patient, for preventing a cardiovascular event in a patient with end stage renal disease, for preventing venous thromboembolic events in a patient receiving chemotherapy through an indwelling catheter, for preventing thromboembolic events in a patient undergoing a lower limb arterial reconstructive procedure, or for treating by way of therapy or prophylaxis an arterial disease selected from acute coronary syndromes, cerebrovascular thrombosis, peripheral arterial occlusion and arterial thrombosis resulting from atrial fibrillation, valvular heart disease, arterio-venous shunts, indwelling catheters or coronary stents.

**73.** The use of daim daim 71 or claim 72 wherein the medicament is an intravenous medicament, whether for use directly or after reconstitution.

**74.** A parenteral pharmaceutical formulation comprising a combination of (i) a salt as defined in any of claims 1 to 53, or a salt as defined in any of said claims and having the characteristics recited in one or a permitted combination of claims 62 to 68, and (ii) a further pharmaceutically active agent, e.g. another cardiovascular treatment agent, for example a lipid-lowering drug, a fibrate, niacin, a statin, a CETP inhibitor, a bile acid sequestrant, an anti-oxidant, a IIb/IIIa antagonist, an aldosterone inhibitor, an A2 antagonist, an A3 agonist, a beta-blocker, acetylsalicylic acid, a loop diuretic, an ace inhibitor, an antithrombotic agent with a different mechanism of action, an antiplatelet agent, a thromboxane receptor and/or synthetase inhibitor, a fibrinogen receptor antagonist, a prostacyclin mimetic, a phosphodiesterase inhibitor, an ADP-receptor ($P_2$ T) antagonist, a thrombolytic, a cardioprotectant or a COX-2 inhibitor.

**75.** The use of a salt as defined in any of claims 1 to 53, or of a salt as defined in any of said claims and having the characteristics recited in one or a permitted combination of claims 63 to 68, for the manufacture of a parenteral medicament for treating, for example preventing, a cardiovascular disorder in co-administration with another cardiovascular treatment agent, for example one listed in daim 74.

**76.** A parenteral medicament comprising a salt of a boronic acid which is a selective thrombin inhibitor and has a neutral aminoboronic acid residue capable of binding to the thrombin 51 subsite linked through a peptide linkage to a hydrophobic moiety capable of binding to the thrombin S2 and S3 subsites, the salt comprising a boronate species derived from the boronic acid and a cation having a valency n and having an observed stoichiometry consistent with a notional stoichiometry (boronic acid:cation) of n:1.

**77.** A medicament of daim 76 wherein the boronic acid has a Ki for thrombin of about 100 nM or less, and optionally of about 20 nM or less.

**78.** A product for intravenous administration after reconstitution and comprising, in the form of a finely divided solid, a salt consisting essentially of a monosodium or monolithium salt of an acid of the formula Cbz-(R)-Phe-(S)-Pro-(R)-Mpg-$B(OH)_2$, the salt optionally being in admixture with one or more anti-oxidants, preservatives or other additives.

**79.** A product for intravenous administration comprising, in the form of an isotonic aqueous solution, a salt consisting essentially of a monosodium or monolithium salt of an acid of the formula Cbz-(R)-Phe-(S)-Pro-(R)-Mpg-$B(OH)_2$, the product optionally further containing one or more anti-oxidants, preservatives or other additives.

**80.** The product of claim 78 or claim 79 which further has the characteristics recited in one or a permitted combination of claims 61 to 67.

**81.** The product of any of claims 78 to 80 wherein the salt comprises anhydride species.

**82.** A method for making a parenteral pharmaceutical formulation, comprising:

contacting a boronic acid as defined in any of claims 1 to 33 with a pharmaceutically acceptable base having

a pKb of 7 or more; and
formulating the resultant product into the formulation.

**83.** A method of claim 82 which further comprises obtaining the organoboronic acid by:

forming a solution of a diol ester of the boronic acid as defined in any of claims 1 to 33;
combining the solution with diethanolamine and allowing or causing the diethanolamine to react with the ester to form an insoluble precipitate;
recovering the precipitate;
converting the precipitate into the free boronic acid.

**84.** A method of claim 83 wherein the ether is diethyl ether and the ester is a pinacol ester.

**85.** A method of any of claims 82 to 84 wherein the base is an organic nitrogen-containing compound or is a base of a metal.

**86.** A method of claim 85 wherein the organic base has a pKb of 7.5 or more and is not choline or ammonia.

**87.** A method of any of claims 82 to 84 wherein the base is of an alkali metal or alkaline earth metal.

**88.** A method of claim 87 wherein the base is a sodium base.

**89.** A method of any of claims 82 to 88 wherein the boronic acid is as defined in any of claims 27 to 32.

**90.** A method of any of claim 82 to 88 wherein the boronic acid is Cbz-(R)-Phe-(S)-Pro-(R)-Mpg-$B(OH)_2$.

**91.** A method of any of claims 82 to 90 wherein said product comprises boronate anhydride species.

**92.** A method of any of claims 82 to 91 wherein the formulation comprises said product in solid form for reconstitution for intravenous administration.

**93.** A method of any of claims 82 to 91 wherein the formulation is a liquid intravenous formulation in an aqueous based carrier.

**94.** The use of a diol ester of a boronic acid as defined in any of claims 1 to 33 to make a parenteral pharmaceutical formulation by allowing the boronic acid, produced by hydrolysis of the diol ester or of a diethanolamine ester made by transesterification of said diol ester, to react with a pharmaceutically acceptable base as defined in any of claims 83 to 88 and formulating the resultant product into a pharmaceutical formulation.

**95.** The use of claim 94 wherein the diol ester is a pinacol, pinanediol or diethanolamine ester.

## Patentansprüche

**1.** Eine parenterale pharmazeutische Formulierung, die ein pharmazeutisch akzeptables Salz einer Boronsäure nach Zusatz einer Base mit der Formel (I) umfasst:

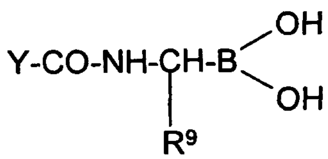

(I)

wobei
Y einen hydrophoben Anteil umfasst, der zusammen mit dem Aminoboronsäurerest -NHCH($R^9$)-$B(OH)_2$ Affinität zur Substratbindungsstelle von Thrombin aufweist; und

$R^9$ eine geradkettige Alkylgruppe ist, die durch eine oder mehrere Etherbindungen unterbrochen wird und in der die Gesamtzahl von Sauerstoff- und Kohlenstoffatomen 3, 4, 5 oder 6 ist oder $R^9$ $-(CH_2)_m-W$ ist, wobei m 2, 3, 4 oder 5 ist und W -OH oder Halogen (F, Cl, Br oder I) ist,
und, wenn YCO, ein optional N-terminal geschützter Dipeptidrest ist, die Peptidbindungen in der Säure optional und unabhängig durch eine $C_1$-$C_{13}$ Hydrocarbylgruppe N-substituiert werden, welche optional kettenförmigen oder ringförmigen Stickstoff, Sauerstoff oder Schwefel enthält, welche optional durch einen aus Halo-, Hydroxy- und Trifluormethyl ausgewählten Substituent substituiert werden können.

**2.** Eine Formulierung gemäß Anspruch 1, wobei $R^9$ eine Alkoxyalkylgruppe ist.

**3.** Eine Formulierung gemäß Anspruch 1 oder Anspruch 2, wobei alle Peptidbindungen in der Säure unsubstituiert sind.

**4.** Eine Formulierung gemäß Anspruch 1 oder Anspruch 2 wobei die besagte $C_1$-$C_{13}$ Hydrocarbylgruppe eine $C_1$-$C_6$ gesättigte Hydrocarbylgruppe ist.

**5.** Eine Formulierung gemäß einem der Ansprüche 1 bis 4, wobei YCO- eine Aminosäure umfasst, die sich an die S2-Subsite von Thrombin bindet, wobei die Aminosäure N-terminal mit einem Anteil verknüpft ist, der die S3-Subsite von Thrombin bindet.

**6.** Eine Formulierung gemäß einem der Ansprüche 1 bis 4 wobei YCO- ein optional N-terminal geschütztes Dipeptid ist, das sich an die S3- und S2-Bindungsstellen von Thrombin bindet.

**7.** Eine Formulierung gemäß Anspruch 6 wobei das besagte Dipeptid N-terminal geschützt ist.

**8.** Eine Formulierung gemäß Anspruch 6 oder Anspruch 7, wobei Y einen P3-Rest umfasst, der eine Seitenkette der folgenden Formel (B) aufweist:

$$-(CO)_a-(CH_2)_b-D_c-C_e(E^1)(E^2)(E^3) \qquad (B)$$

wobei
a 0 oder 1 ist;
e 1 ist;
b 0 oder eine Ganzzahl ist, so dass (b+e) zwischen 1 und 4 liegt;
c 0 oder 1 ist;
D 0 oder S ist;
$E^1$, $E^2$ und $E^3$ jeweils unabhängig voneinander aus $-R^{15}$ and $-J-R^{15}$ ausgewählt wurden, wobei J ein Ring mit 5-6 Gliedern ist und $-R^{15}$ aus $C_1$-$C_6$ Trialkylsilyl, -CN, $-R^{13}$, $-R^{12}OR^{13}$, $-R^{12}COR^{13}$, $-R^{12}CO_2R^{13}$, $-R^{12}O_2CR^{13}$ und einem oder zwei Halogenen ausgewählt wird, wobei $R^{12}$ $-(CH_2)_f-$ ist und $R^{13}$ $-(CH_2)_gH$ ist, wobei f und g jeweils unabhängig zwischen 0 und 10 liegen, vorausgesetzt, dass (f+g) 1, 2, 3 oder 4 ist.

**9.** Eine Formulierung gemäß Anspruch 8, wobei, wenn $E^1$, $E^2$ oder $E^3$ eine $R^{13}$-Gruppe enthalten, g 0 oder 1 ist.

**10.** Eine Formulierung gemäß einem der Ansprüche 6 bis 9, wobei YCO- einen P2-Rest umfasst, der ein Rest einer Iminosäure ist.

**11.** Eine Formulierung gemäß einem der Ansprüche 6 bis 10, wobei der Dipeptidrest einen Rest einer P3- Aminosäure mit (R)-Konfiguration und einen P2-Rest mit (S)-Konfiguration umfasst, wobei der Rest $-NHCH(R^9)-B(OH)$ eine (R)-Konfiguration aufweist.

**12.** Eine Formulierung gemäß einem der Ansprüche 1 bis 11, wobei die Boronsäure für Thrombin einen Ki-Wert von ungefähr 100nM oder weniger aufweist.

**13.** Eine Formulierung gemäß Anspruch 12, wobei die Boronsäure für Thrombin einen Ki-Wert von ungefähr 20nM oder weniger aufweist.

**14.** Eine Formulierung gemäß Anspruch 1, wobei die Boronsäure die folgende Formel (II) aufweist:

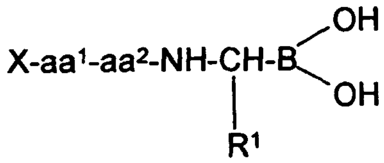

(II)

wobei
X H (um $NH_2$ zu formen) oder eine Aminoschutzgruppe ist:

$aa^1$ eine Aminosäure ist, die eine Hydrocarbyl-Seitenkette aufweist, welche nicht mehr als 20 Kohlenstoffatome enthält und mindestens eine zyklische Gruppe umfasst, die bis zu 13 Kohlenstoffatome aufweist;
$aa^2$ eine Iminosäure mit 4 bis 6 Ringgliedem oder Gly ist, die durch eine $C_3$-$C_{13}$ Hydrocarbylgruppe N-substituiert wurde;
$R^1$ eine Gruppe mit der Formel $-(CH_2)_S-Z$ ist, wobei s 2, 3 oder 4 ist und Z -OH, -OMe, -OEt oder Halogen (F, Cl, Br oder I) ist.

**15.** Eine Formulierung gemäß Anspruch 14, wobei $aa^1$ aus Phe, Dpa und ganz oder teilweise hydrogenisierten Analogen dieser ausgewählt wird.

**16.** Eine Formulierung gemäß Anspruch 14, wobei $aa^1$ aus Dpa, Phe, Dcha und Cha ausgewählt wird.

**17.** Eine Formulierung gemäß einem der Ansprüche 14 bis 16, wobei $aa^1$ eine (R)-Konfiguration aufweist.

**18.** Eine Formulierung gemäß Anspruch 14, wobei $aa^1$ (R)-Phe oder (R)-Dpa ist.

**19.** Eine Formulierung gemäß Anspruch 14, wobei $aa^1$ (R)-Phe ist.

**20.** Eine Formulierung gemäß einem der Ansprüche 14 bis 19, wobei $aa^2$ ein Rest einer Iminosäure mit der folgenden Formel (IV) ist:

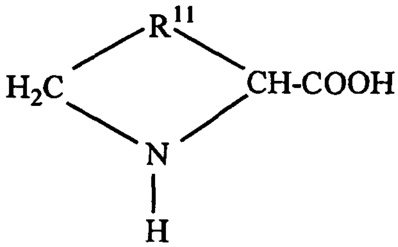

(IV),

wobei $R^{11}$ $-CH_2-$, $-CH_2-CH_2-$, $-S-CH_2-$, $-S-C(CH_3)_2-$ oder $-CH_2-CH_2-CH_2-$ ist, wobei die Iminosäure, wenn der Ring 5 oder 6 Glieder aufweist, optional an einer oder mehreren $-CH_2$-Gruppen durch 1 bis 3 $C_1$-$C_3$ Alkylgruppen substituiert wird.

**21.** Eine Formulierung gemäß einem der Ansprüche 14 bis 20, wobei $aa^2$ eine (S)-Konfiguration aufweist.

**22.** Eine Formulierung gemäß Anspruch 20, wobei $aa^2$ ein (S)-Prolinrest ist.

**23.** Eine Formulierung gemäß Anspruch 14, wobei $aa^1$-$aa^2$ (R)-Phe-(S)-Pro ist.

**24.** Eine Formulierung gemäß einem der Ansprüche 14 bis 23, wobei der Rest $-NH-CH(R^1)-B(OH)_2$ eine (R)-Konfiguration aufweist.

**25.** Eine Formulierung gemäß einem der Ansprüche 14 bis 24, wobei $R^1$ 2-Bromethyl, 2-Chlorethyl, 2-Methoxyethyl, 3-Brompropyl, 3-Chlorpropyl oder 3-Methoxypropyl ist.

**26.** Eine Formulierung gemäß einem der Ansprüche 14 bis 24, wobei $R^1$ 3-Methoxypropyl ist.

**27.** Eine Formulierung gemäß Anspruch 14, wobei die Boronsäure die folgende Formel (VIII) aufweist:

$$X\text{-(R)-Phe-(S)-Pro-(R)-Mpg-B(OH)}_2 \qquad \text{(VIII)}.$$

**28.** Eine Formulierung gemäß einem der Ansprüche 14 bis 27, wobei X $R^6$-$(CH_2)$p-C(O)-, $R^6$-$(CH_2)$p $S(O)_2$-, $R^6$-$(CH_2)$p-NH-C(O)- oder $R^6$-$(CH_2)_p$-O-C(O)- ist, wobei p 0, 1, 2, 3, 4, 5 oder 6 ist und $R^6$ H oder eine zyklische Gruppe mit 5 bis 13 Gliedern ist, die optional durch 1, 2 oder 3 Substituenten substituiert werden kann, die aus Halogen, Amino, Nitro, Hydroxy, einer zyklischen $C_5$-$C_6$-Gruppe, $C_1$-$C_4$-Alkyl, oder $C_1$-$C_4$-Alkyl, das entweder ein in der Kette enthaltenes O enthält und/oder durch dieses mit der zyklischen Gruppe verknüpft ist, ausgewählt werden, wobei die erwähnten Alkylgruppen optional durch einen Substituent substituiert werden können, der aus Halogen, Amino, Nitro, Hydroxy und einer zyklischen $C_{5}$-$C_6$-Gruppe ausgewählt werden kann.

**29.** Eine Formulierung gemäß Anspruch 28, wobei die besagte 5- bis 13-gliedrige zyklische Gruppe aromatisch oder heteroaromatisch ist.

**30.** Eine Formulierung gemäß Anspruch 29, wobei die besagte 5- bis 13-gliedrige zyklische Gruppe Phenyl oder eine 6-gliedrige heteroaromatische Gruppe ist.

**31.** Eine Formulierung gemäß einem der Ansprüche 28 bis 30, wobei X $R^6$-$(CH_2)_p$-O-C(O)- und p 0 oder 1 ist.

**32.** Eine Formulierung gemäß einem der Ansprüche 14 bis 26, wobei X Benzyloxycarbonyl ist.

**33.** Eine Formulierung gemäß Anspruch 14, wobei die Boronsäure Cbz-(R)-Phe-(S)-Pro-(R)-Mpg-$B(OH)_2$ ist.

**34.** Eine Formulierung gemäß einem der Ansprüche 1 bis 33, wobei das Salz kein Cholin oder Ammoniumsalz umfasst.

**35.** Eine Formulierung gemäß einem der Ansprüche 1 bis 34, wobei das Salz von der Boronsäure derivierte Boronsäure-Ione und monovalente Gegenione umfasst.

**36.** Eine Formulierung gemäß einem der Ansprüche 1 bis 34, wobei das Salz ein Salz der Peptid-Boronsäure mit einem Alkalimetall oder einer stark basischen, organischen, Stickstoff enthaltenden Verbindung ist.

**37.** Eine Formulierung gemäß Anspruch 36, wobei die stark basische, organische Stickstoffverbindung einen pKb von ungefähr 7 oder mehr aufweist, z. B. ungefähr 7,5 oder mehr.

**38.** Eine Formulierung gemäß einem der Ansprüche 1 bis 35, wobei das Salz ein Salz der Boronsäure mit einem Metall ist.

**39.** Eine Formulierung gemäß einem der Ansprüche 1 bis 33 oder 37, wobei das Salz ein Salz der Boronsäure mit einem Alkalimetall, einem Aminozucker, einem Guanidin oder einem Amin mit der folgenden Formel (XI) ist:

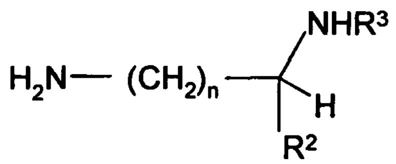

(XI)

wobei n zwischen 1 uns 6 liegt, $R^2$ H, Carboxylat oder derivatisiertes Carboxylat ist, $R^3$ H, $C_1$-$C_4$ Alkyl oder ein Rest einer natürlichen oder unnatürlichen Aminosäure ist.

**40.** Eine Formulierung gemäß einem der Ansprüche 1 bis 33, wobei das Salz ein L-Arginin-Salz ist.

**41.** Eine Formulierung gemäß einem der Ansprüche 1 bis 33, wobei das Salz ein L-Lysin-Salz ist.

**42.** Eine Formulierung gemäß einem der Ansprüche 1 bis 33, wobei das Salz ein zweiwertiges Metallsalz ist.

**43.** Eine Formulierung gemäß einem der Ansprüche 1 bis 33, wobei das Salz ein Natriumsalz ist.

**44.** Eine Formulierung gemäß einem der Ansprüche 1 bis 33, wobei das Salz ein Lithiumsalz ist.

**45.** Eine Formulierung gemäß einem der Ansprüche 1 bis 33, wobei das Salz ein Glucaminsalz ist, z. B. ein N-Methyl-D-Glucaminsalz.

**46.** Eine Formulierung gemäß einem der Ansprüche 1 bis 33, wobei das Salz ein Calcium- oder Magnesiumsalz ist.

**47.** Eine Formulierung gemäß einem der Ansprüche 1 bis 46, die von der Peptid-Boronsäure derivierte Boronsäure-Ione umfasst und eine mit den Boronsäure-Ionen übereinstimmende Stöchiometrie aufweist, die eine einfache, negative Ladung tragen.

**48.** Eine Formulierung gemäß einem der Ansprüche 1 bis 47, wobei das Salz ein von der Peptid-Boronsäure deriviertes Boronsäure-Ion und ein Gegenion umfasst und wobei das Salz im Wesentlichen aus einem Salz besteht, das nur eine Art von Gegenion aufweist.

**49.** Eine Formulierung gemäß einem der Ansprüche 1 bis 33, wobei das Salz ein Monolithiumsalz ist.

**50.** Eine Formulierung gemäß einem der Ansprüche 1 bis 32, wobei das Salz ein Hemicalciumsalz ist.

**51.** Eine Formulierung gemäß einem der Ansprüche 1 bis 32, wobei das Salz ein Mononatriumsalz ist.

**52.** Eine Formulierung gemäß Anspruch 1, wobei das Salz das Mononatriumsalz von Cbz-(R)-Phe-(S)-Pro-(R)-Mpg-B $(OH)_2$ ist.

**53.** Eine Formulierung gemäß Anspruch 1, wobei das Salz das Hemicalciumsalz von Cbz-(R)-Phe-(S)-Pro-(R)-Mpg-B $(OH)_2$ ist.

**54.** Eine Formulierung gemäß einem der Ansprüche 1 bis 53, die außerdem ein pharmazeutisch akzeptables Verdünnungsmittel, Bindemittel oder einen Träger umfasst.

**55.** Eine Formulierung gemäß einem der Ansprüche 1 bis 54, wobei das Salz das Boronat in anhydrider Form umfasst.

**56.** Eine Formulierung gemäß einem der Ansprüche 1 bis 55, die für die intravenöse Verabreichung angepasst wurde.

**57.** Eine Formulierung gemäß Anspruch 56, die ein Tonikum beinhaltet.

**58.** Eine Formulierung gemäß Anspruch 56 oder Anspruch 57, deren Form vor der Verabreichung der Rekonstitution bedarf.

**59.** Eine Formulierung gemäß Anspruch 56 oder Anspruch 55, die die Form einer Lösung annimmt.

**60.** Eine Formulierung gemäß einem der Ansprüche 1 bis 35, die für die intravenöse Verabreichung angepasst wurde und eine Lösung umfasst, welche als das Salz die folgende erste und zweite Spezies und optional andere Komponenten enthält, zum Beispiel ein Tonikum oder einen Puffer:

a) eine erste Spezies, die aus (a) Boronsäuren mit der Formel (I) oder gegebenenfalls der Formel (II), (b) Boronsäure-Anionen dieser und (c) einer Gleichgewichtsform der zuvor erwähnten (z. B. Anydrid) ausgewählt wird; und
b) eine zweite Spezies, die aus der aus pharmazeutisch akzeptablen Alkalimetallionen und basischen, organischen, Stickstoff enthaltenden Verbindungen bestehenden Gruppe ausgewählt wird, die einen pKb von ungefähr 7 oder mehr aufweisen (wobei diese Verbindungen die Verbindungen in protonierter Form umfassen sollen),

wobei die Formulierung eine beobachtete Stöchiometrie aufweist, die mit der ersten Spezies, welche aus Boronsäure-Ionen besteht, die eine einfache negative Ladung tragen, übereinstimmen würde.

**61.** Eine Formulierung gemäß Anspruch 60, die für Thrombin einen Ki-Wert von ungefähr 20nM oder weniger aufweist.

**62.** Eine Formulierung gemäß einem der Ansprüche 1 bis 61, die eine Spurenmenge eines aliphatischen oder cycloaliphatischen Lösungsmittels enthält.

**63.** Eine Formulierung gemäß Anspruch 62, wobei das Lösungsmittel ein N-Alkan ist, optional N-Heptan.

**64.** Eine Formulierung gemäß einem der Ansprüche 1 bis 63, wobei $R^9$ (im Falle einer Formulierung, die ein Boronsäure-Salz der Formel (I) enthält) oder $R^1$ (im Falle einer Formulierung, die ein Boronsäure-Salz der Formel (II) enthält) eine Alkoxyalkylgruppe ist, die durch $R^Z$-O-$R^Y$- dargestellt werden kann und die Formulierung frei von einer Fremdstoff-Boronsäure-Spezies ist, deren Struktur der der Spezies mit der Formel (I) oder Formel (II) entspricht, wenn $R^9$ oder gegebenenfalls $R^1$ durch eine Alkylgruppe $R^Y$ ersetzt wird.

**65.** Eine Formulierung gemäß einem der Ansprüche 1 bis 64, die im Wesentlichen frei von jeglichen Abbauprodukten ist, die durch C-B Bindungsspaltung entstehen.

**66.** Eine Formulierung gemäß Anspruch 14 oder einem der Ansprüche 15 bis 65 in Kombination mit Anspruch 14, in der Boronsäure-Ione, in denen $aa^1$ eine (R)-Konfiguration aufweist, $aa^2$ eine (S)-Konfiguration aufweist und der Rest -NH-CH($R^1$)-B$(OH)_2$ eine (R)-Konfiguration aufweist, einen Diastereomerenüberschuss von 99% oder mehr aufweisen, z. B. 99,5% oder mehr über dem (R,S,S) Isomer.

**67.** Eine Formulierung gemäß Anspruch 33 oder einem der Ansprüche 34 bis 63 oder 66 in Kombination mit Anspruch 33, die im Wesentlichen frei von der folgenden Verbindung ist:

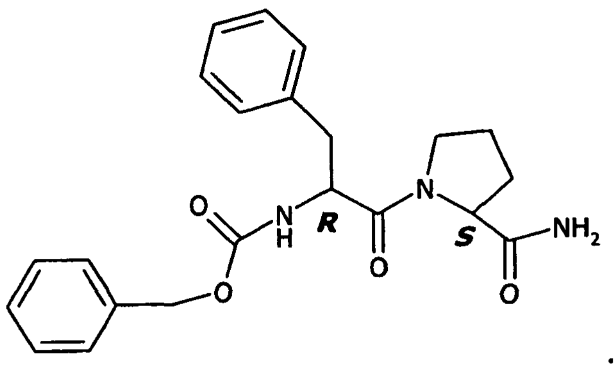

**68.** Eine Formulierung gemäß Anspruch 33 oder einem der Ansprüche 34 bis 63 oder 66 in Kombination mit Anspruch 33 oder Anspruch 67, die im Wesentlichen frei von der folgenden Verbindung ist:

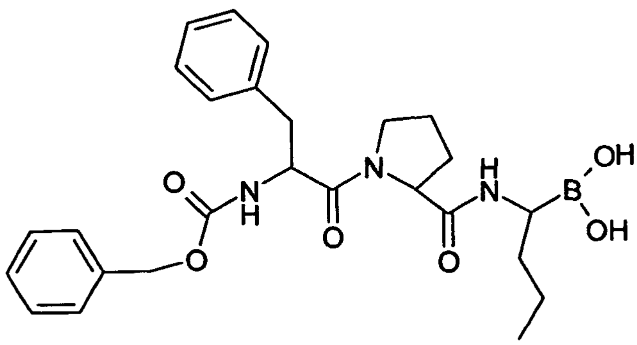

und ihren Gleichgewichtsformen.

**69.** Ein Produkt zur Verwendung als ein parenterales Pharmazeutikum, das ein wie in einem der Ansprüche 1 bis 51 definiertes Salz umfasst.

**70.** Ein Produkt gemäß Anspruch 68, wobei das Salz Boronsäure-Gruppen der Säure in anhydrider Form umfasst.

**71.** Die Verwendung eines wie in einem der Ansprüche 1 bis 51 definierten Salzes oder eines Salzes, wie es in einem der besagten Ansprüche definiert wird und das die in einem oder einer erlaubten Kombination der Ansprüche 61 bis 67 erwähnten Charakteristika aufweist, zur Herstellung eines parenteralen Medikaments zur Behandlung von Thrombosen durch Prophylaxe oder Therapie, wobei das Medikament optional zur antikoagulierenden Behandlung verwendet werden kann, wenn Blut mit einem medizinischen Gerät außerhalb des Körpers in Berührung kommt, wie zum Beispiel während einer kardiovaskulären Operation unter Verwendung einer Herz-Lungen-Maschine oder während der Hämodialyse.

**72.** Die Verwendung eines wie in einem der Ansprüche 1 bis 53 definierten Salzes oder eines Salzes, wie es in einem der besagten Ansprüche definiert wird und das die in einem oder einer erlaubten Kombination der Ansprüche 62 bis 68 erwähnten Charakteristika aufweist, zur Herstellung eines parenteralen Medikaments zur Vorbeugung von Thrombosen im Hämodialyse-Kreislauf eines Patienten, zur Vorbeugung eines kardiovaskulären Ereignisses bei einem Patienten mit chronischem Nierenversagen, zur Vorbeugung venöser thromboembolischer Ereignisse bei einem Patienten, der über einen Dauerkatheter chemotherapiert wird, oder zur Vorbeugung thromboembolischer Ereignisse bei einem Patienten, bei dem durch einen rekonstruktiven Eingriff Arterien in unteren Körpergliedern wiederhergestellt werden, oder zur Behandlung einer Arterienerkrankung ausgewählt aus akutem Koronarsyndrom, zerebrovaskulärer Thrombose, peripherem Arterienverschluss und arterieller Thrombose, die als Folge von Vorhofflimmern, Herzklappernerkrankung, arteriovenösen Shunts, Dauerkathetern oder Koronarstents auftreten können, durch Therapie oder Prophylaxe.

**73.** Die Verwendung gemäß Anspruch 71 oder Anspruch 72, wobei das Medikament ein intravenöses Medikament ist, entweder zur direkten Anwendung oder zur Anwendung nach der Rekonstitution.

**74.** Eine parenterale pharmazeutische Formulierung, die eine Kombination (i) eines wie in einem der Ansprüche 1 bis 53 definierten Salzes oder eines wie in einem der besagten Ansprüche definierten Salzes mit den in einem oder einer erlaubten Kombination der Ansprüche 62 bis 68 erwähnten Charakteristika und (ii) eines weiteren pharmazeutischen Wirkstoffs, z. B. eines weiteren Wirkstoffs zur kardiovaskulären Behandlung umfasst, beispielsweise ein lipidsenkendes Medikament, ein Fibrat, Niacin, ein Statin, einen CETP-Hemmstoff, ein Gallensäure-Maskierungsmittel, ein Antioxidans, einen IIb/IIIa Antagonisten, einen Aldosteron-Hemmstoff, einen A2-Antagonisten, einen A3-Agonisten, einen Beta-Blokker, Acetylsalizylsäure, ein Schleifendiuretikum, einen ACE-Hemmstoff, einen antithrombotischen Wirkstoff mit einem anderen Aktionsmechanismus, einen Antiplättchen-Wirkstoff, einen Thromboxan-Rezeptor und/oder Synthetase-Hemmstoff, einen Fibrinogen-Rezeptor-Antagonist, einen Prostazyklin nachahmenden Wirkstoff, einen Phosphodiesterase-Hemmstoff, einen ADP-Rezeptor ($P_2T$) Antagonisten, ein Thrombolytikum, ein Medikament zur Kardioprotektion oder einen COX-2 Hemmstoff.

**75.** Die Verwendung eines wie in einem der Ansprüche 1 bis 53 definierten Salzes oder eines wie in einem der besagten Ansprüche definierten Salzes, das die in einem oder einer erlaubten Kombination der Ansprüche 63 bis 68 erwähnten Charakteristika aufweist, zur Herstellung eines parenteralen Medikaments zur Behandlung, zum Beispiel durch Vorbeugung, einer kardiovaskulären Störung, indem es zusammen mit einem anderen kardiovaskulären Behandlungswirkstoff verabreicht wird, zum Beispiel einem der in Anspruch 74 aufgelisteten.

**76.** Ein parenterales Medikament, das ein Salz einer Boronsäure umfasst, welches ein selektiver Thrombinhemmstoff ist und einen neutralen Aminoboronsäurerest aufweist, der in der Lage ist, sich an die S1-Thrombin-Subsite zu binden, die über eine Peptidbindung mit einem hydrophoben Anteil verknüpft ist, der in der Lage ist, sich an die S2- und S3-Thrombin-Subsites zu binden, wobei das Salz eine Boronsäure-Spezies umfasst, die von der Boronsäure und einem Kation mit einer Valenz n deriviert wurde, wobei das Salz eine beobachtete Stöchiometrie aufweist, die einer fiktiven Stöchiometrie (Boronsäure:Kation) von n:1 entspricht.

**77.** Ein Medikament gemäß Anspruch 76, wobei die Boronsäure für Thrombin einen Ki-Wert von ungefähr 100 nM oder weniger aufweist und optional von ungefähr 20 nM oder weniger.

**78.** Ein Produkt zur intravenösen Verabreichung nach der Rekonstitution, welches in Form eines fein zerteilten Festkörpers ein Salz umfasst, das im Wesentlichen aus einem Mononatrium- oder einem Monolithiumsalz einer Säure mit der Formel Cbz-(R)-Phe-(S)-Pro-(R)-Mpg-B$(OH)_2$ besteht, wobei das Salz optional mit einem oder mehreren Antioxidanzien, Konservierungsmitteln oder anderen Zusatzstoffen gemischt wird.

**79.** Ein Produkt zur intravenösen Verabreichung, welches in Form einer isotonischen wässrigen Lösung ein Salz umfasst, das im Wesentlichen aus einem Mononatrium- oder einem Monolithiumsalz einer Säure mit der Formel

Cbz-(R)-Phe-(S)-Pro-(R)-Mpg-$B(OH)_2$ besteht, wobei das Produkt zusätzlich optional einen oder mehrere Antioxidanzien, Konservierungsmittel oder andere Zusatzstoffe enthält.

**80.** Das Produkt gemäß Anspruch 78 oder Anspruch 79, das außerdem die in einem oder einer erlaubten Kombination der Ansprüche 61 bis 67 erwähnten Charakteristika aufweist.

**81.** Das Produkt gemäß einem der Ansprüche 78 bis 80, wobei das Salz anhydride Spezies umfasst.

**82.** Ein Verfahren zur Herstellung einer parenteralen pharmazeutischen Formulierung, folgendes umfassend:

eine Boronsäure, wie in den Ansprüchen 1 bis 33 definiert, wird mit einer pharmazeutisch akzeptablen Base mit einem pKb von 7 oder mehr in Berührung gebracht; und
das entstehende Produkt wird in die Formulierung formuliert.

**83.** Ein Verfahren gemäß Anspruch 82, das außerdem die Gewinnung der Organoboronsäure wie folgt umfasst:

das Formen einer Lösung eines Diolesters der Boronsäure, wie in den Ansprüchen 1 bis 33 definiert;
das Kombinieren der Lösung mit Diethanolamin und das Zulassen oder Anregen einer
Reaktion des Diethanolamins mit dem Ester, damit diese einen unlöslichen Niederschlag formen;
die Rückgewinnung des Niederschlags;
die Umwandlung des Niederschlags in die freie Boronsäure.

**84.** Ein Verfahren gemäß Anspruch 83, wobei der Ether Diethylether ist und der Ester ein Pinacolester ist.

**85.** Ein Verfahren gemäß einem der Ansprüche 82 bis 84, wobei die Base eine organische, Stickstoff enthaltende Verbindung ist oder eine Base eines Metalls ist.

**86.** Ein Verfahren gemäß Anspruch 85, wobei die organische Base einen pKb von 7,5 oder mehr aufweist und nicht Cholin oder Ammoniak ist.

**87.** Ein Verfahren gemäß einem der Ansprüche 82 bis 84, wobei die Base die eines Alkalimetalls oder Erdalkalimetalls ist.

**88.** Ein Verfahren gemäß Anspruch 87, wobei die Base eine Natriumbase ist.

**89.** Ein Verfahren gemäß einem der Ansprüche 82 bis 88, wobei die Boronsäure so wie in den Ansprüchen 27 bis 32 definiert ist.

**90.** Ein Verfahren gemäß einem der Ansprüche 82 bis 88, wobei die Boronsäure Cbz-(R)-Phe-(S)-Pro-(R)-Mpg-$B(OH)_2$ ist.

**91.** Ein Verfahren gemäß einem der Ansprüche 82 bis 90, wobei das besagte Produkt Boronsäure-Anhydrid-Spezies umfasst.

**92.** Ein Verfahren gemäß einem der Ansprüche 82 bis 91, wobei die Formulierung das besagte Produkt in solider Form umfasst, das per Rekonstitution zur intravenösen Verabreichung angepasst wird.

**93.** Ein Verfahren gemäß einem der Ansprüche 82 bis 91, wobei die Formulierung eine flüssige intravenöse Formulierung in einem Träger mit wässriger Basis ist.

**94.** Die Verwendung eines Diolesters einer Boronsäure, wie in einem der Ansprüche 1 bis 33 definiert, um eine parenterale pharmazeutische Formulierung zu produzieren, indem man die Boronsäure, die durch Hydrolyse des Diolesters oder eines Diethanolaminesters, der durch Transesterifizierung des besagten Diolesters hergestellt wird, mit einer pharmazeutisch akzeptablen Base, wie in Ansprüchen 83 bis 88 definiert, reagieren lässt und das entstehende Produkt in eine pharmazeutische Formulierung formuliert.

**95.** Die Verwendung gemäß Anspruch 94, wobei der Diolester ein Pinacol-, Pinanediol- oder Diethanolaminester ist.

**Revendications**

1. Formulation pharmaceutique parentérale comprenant un sel par addition de base pharmaceutiquement acceptable d'un acide boronique de formule (I):

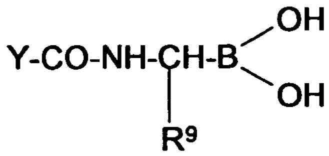

(I)

dans lequel :

Y comprend une fraction hydrophobe qui, en compagnie du résidu d'acide aminoboronique $-NHCH(R^9)-B(OH)_2$, possède une affinité pour le site de liaison à un substrat de la thrombine; et
$R^9$ est un groupe alkyle à chaîne droite interrompue par une ou plusieurs liaisons éther et dans lequel le nombre total d'atomes d'oxygène et de carbone est de 3, 4, 5 ou 6, ou $R^9$ est $-(CH_2)_m-W$, m étant égal à 2, 3, 4 ou 5 et W étant un -OH ou un halogène (F, Cl, Br ou I),
et, lorsque YCO- est un résidu dipeptidique optionnellement protégé au niveau de la partie N-terminale, les liaisons peptidiques dans l'acide sont optionnellement et indépendamment N-substituées par un groupement hydrocarbyle en $C_1$-$C_{13}$ contenant optionnellement un azote, un oxygène ou un soufre dans la chaîne ou dans le cycle et optionnellement substituées par un substituant sélectionné parmi un halogène, un hydroxyle et un trifluorométhyle.

2. Formulation selon la revendication 1 dans laquelle $R^9$ est un groupement alkoxyalkyle.

3. Formualtion selon la revendication 1 ou la revendication 2 dans laquelle toutes les liaisons peptidiques dans l'acide sont non substituées.

4. Formulation selon la revendication 1 ou la revendication 2 dans laquelle ledit groupement hydrocarbyle en $C_1$-$C_{13}$ est un groupement hydrocarbyle saturé en $C_1$-$C_6$.

5. Formulation selon l'une quelconque des revendications 1 à 4 dans laquelle le YCO- comprend un acide aminé qui se lie au sous-site S2 de la thrombine, l'acide aminé étant lié par son extrémité N-terminale à une fraction qui se lie au sous-site S3 de la thrombine.

6. Formulation selon l'une quelconque des revendications 1 à 4 dans laquelle le YCO- est un dipeptide optionnellement protégé au niveau de son extrémité N-terminale qui se lie aux sites de liaison S3 et S2 de la thrombine.

7. Formulation selon la revendication 6 dans laquelle ledit dipeptide est protégé au niveau de son extrémité N-terminale.

8. Formulation selon la revendication 6 ou la revendication 7 dans laquelle Y comprend un résidu P3 ayant une chaîne latérale de formule (B) :

$$-(CO)_a-(CH_2)_b-D_c-C_e(E^1)(E^2)(E^3) \qquad (B)$$

où :

a est égal à 0 ou 1;
e est égal à 1;
b est égal à 0 ou un nombre entier tel que (b+e) aille de 1 à 4;
c est égal à 0 ou 1;
D est 0 ou S;
$E^1$, $E^2$ et $E^3$ sont chacun sélectionnés indépendamment parmi $-R^{15}$ et $-J-R^{15}$, J étant un cycle à 5-6 côtés et $-R^{15}$ étant sélectionné parmi un trialkylsilyl en $C_1$-$C_6$, -CN, $-R^{13}$, $-R^{12}OR^{13}$, $-R^{12}COR^{13}$, $-R^{12}CO_2R^{13}$, $-R^{12}O_2CR^{13}$, et un ou deux atomes d'halogène, $R^{12}$ étant $-(CH_2)_f-$ et $R^{13}$ étant $-(CH_2)_gH$, f et g pouvant chacun

indépendamment avoir une valeur de 0 à 10, pourvu que (f+g) soit égal à 1, 2, 3 ou 4.

**9.** Formulation selon la revendication 8 dans laquelle, lorsque $E^1$, $E^2$ ou $E^3$ contient un groupement $R^{13}$, g est égal à 0 ou 1.

**10.** Formulation selon l'une quelconque des revendications 6 à 9 dans laquelle YCO- comprend un résidu P2 qui est un résidu d'un imino-acide.

**11.** Formulation selon l'une quelconque des revendications 6 à 10 dans lquelle le résidu dipeptide comprend un résidu d'acide aminé P3 de configuration (R)- et un résidu P2 de configuration (S)-, et dans laquelle le résidu $-NHCH(R^9)-B(OH)$ a une configuration (R)-.

**12.** Formulation selon l'une quelconque des revendications 1 à 11 dans laquelle l'acide boronique a un Ki vis-à-vis de la thrombine d'environ 100 nM ou moins.

**13.** Formulation selon la revendication 12 dans laquelle l'acide boronique a un Ki vis-à-vis de la thrombine d'environ 20 nM ou moins.

**14.** Formulation selon la revendication 1 dans laquelle l'acide boronique a la formule (II):

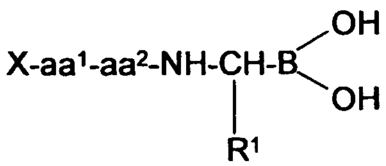

(II)

dans laquelle :

X est un H (pour former un $NH_2$) ou un groupe aminoprotecteur ;
$aa^1$ est un acide aminé ayant une chaîne latérale hydrocarbyle contenant au maximum 20 atomes de carbone et comprenant au moins un groupe cyclique ayant jusqu'à 13 atomes de carbone;
$aa^2$ est un imino-acide ayant un cycle à 4 à 6 côtés ou est Gly N-substitué par un groupement hydrocarbyle en $C_3$-$C_{13}$;
$R^1$ est un groupement de formule $-(CH_2)_S-Z$, s étant égal à 2, 3 ou 4 et Z étant un -OH, -OMe, -OEt ou un halogène (F, Cl, Br ou I).

**15.** Formulation selon la revendication 14 dans laquelle $aa^1$ est sélectionné parmi Phe, Dpa et des analogues totalement ou partiellement hydrogénés de ces derniers.

**16.** Formulation selon la revendication 14 dans laquelle $aa^1$ est sélectionné parmi Dpa, Phe, Dcha et Cha.

**17.** Formulation selon l'une quelconque des revendications 14 à 16 dans laquelle $aa^1$ a une configuration (R)-.

**18.** Formulation selon la revendication 14 dans laquelle $aa^1$ est un (R)-Phe ou (R)-Dpa.

**19.** Formulation selon la revendication 14 dans laquelle $aa^1$ est un (R)-Phe.

**20.** Formulation selon l'une quelconque des revendications 14 à 19 dans laquelle $aa^2$ est un résidu d'un imino-acide de formule (IV)

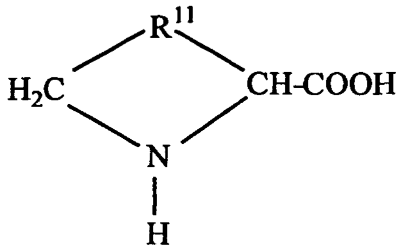

(IV),

dans lequel $R^{11}$ est un $-CH_2-$, $-CH_2-CH_2-$, $-S-CH_2-$, $-S-C(CH_3)_2-$ ou $-CH_2-CH_2-CH_2-$, lequel imino-acide, lorsque le cycle a 5 ou 6 côtés, est optionnellement substitué au niveau de un ou plusieurs groupements $-CH_2-$ par de 1 à 3 groupes alkyle en $C_1$-$C_3$.

**21.** Formulation selon l'une quelconque des revendications 14 à 20 dans laquelle $aa^2$ a une configuration (S).

**22.** Formulation selon la revendication 20 dans laquelle $aa^2$ est un résidu de la (S)-proline.

**23.** Formulation selon la revendication 14, dans lequel $aa^1$-$aa^2$ est (R)-Phe-(S)-Pro.

**24.** Formulation selon l'une quelconque des revendications 14 à 23 dans laquelle le résidu $-NH-CH(R^1)-B(OH)_2$ a la configuration (R).

**25.** Formulation selon l'une quelconque des revendications 14 à 24 dans laquelle $R^1$ est un 2-bromoéthyle, 2-chloroéthyle, 2-méthoxyéthyle, 3-bromopropyle, 3-chloropropyle ou 3-méthoxypropyle.

**26.** Formulation selon l'une quelconque des revendications 14 à 24 dans laquelle $R^1$ est un 3-méthoxypropyle.

**27.** Formulation selon la revendication 14 dans laquelle l'acide boronique a la formule (VIII):

$$X\text{-(R)-Phe-(S)-Pro-(R)-Mpg-B(OH)}_2 \qquad \text{(VIII).}$$

**28.** Formulation selon l'une quelconque des revendications 14 à 27 dans laquelle X est $R^6$-$(CH_2)_p$-C(O)-, $R^6$-$(CH_2)_p$-S$(O)_2$-, $R^6$-$(CH_2)_p$-NH-C(O)- ou $R^6$-$(CH_2)_p$-O-C(O)-, p étant égal à 0, 1, 2, 3, 4, 5 ou 6 et $R^6$ étant un H ou un groupe cyclique à 5 à 13 côtés optionnellement substitué par 1, 2 ou 3 substituants sélectionnés parmi un halogène, un amino, un nitro, un hydroxy, un groupe cyclique en $C_5$-$C_6$, un alkyle en $C_1$-$C_4$ et un alkyle en $C_1$-$C_4$ contenant, et/ou lié au groupe cyclique par, un O dans la chaîne, les groupes alkyle précités étant optionnellement substitués par un substituant sélectionné parmi un halogène, un amino, un nitro, un hydroxy et un groupe cyclique en $C_5$-$C_6$.

**29.** Formulation selon la revendication 28 dans laquelle ledit groupe cyclique à 5 à 13 côtés est aromatique ou hétéroaromatique.

**30.** Formulation selon la revendication 29 dans laquelle ledit groupe cyclique à 5 à 13 côtés est un phényle ou un groupe hétéroaromatique hexagonal.

**31.** Formulation selon l'une quelconque des revendications 28 à 30 dans laquelle X est $R^{6-}$$(CH_2)_p$-O-C(O)- et p est égal à 0 ou 1.

**32.** Formulation selon l'une quelconque des revendications 14 à 26 dans laquelle X est un benzyloxycarbonyle.

**33.** Formulation selon la revendication 14 dans laquelle l'acide boronique est Cbz-(R)-Phe-(S)-Pro-(R)-Mpg-$B(OH)_2$.

**34.** Formulation selon l'une quelconque des revendications 1 à 33 dans laquelle le sel ne comprend pas un sel de choline ou un sel d'ammonium.

**35.** Formulation selon l'une quelconque des revendications 1 à 34, le sel comprenant des ions boronate dérivés de l'acide boronique et des contre-ions monovalents.

**36.** Formulation selon l'une quelconque des revendications 1 à 34 qui est un sel de l'acide boronique peptidique avec un métal alcalin ou un composé organique contenant de l'azote et fortement basique.

**37.** Formulation selon la revendication 36 dans laquelle le composé organique azoté fortement basique a un pKb d'environ 7 ou plus, p.ex. 7,5 ou plus.

**38.** Formulation selon l'une quelconque des revendications 1 à 35 qui est un sel de l'acide boronique avec un métal.

**39.** Formulation selon l'une quelconque des revendications 1 à 33 ou 37 qui est un sel de l'acide boronique avec un métal alcalin, un sucre aminé, une guanidine ou une amine de formule (XI):

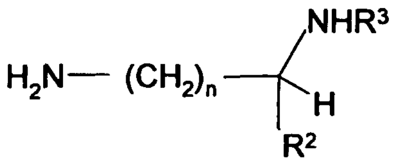

(XI)

où n va de 1 à 6, $R^2$ est un H, un carboxylate ou un carboxylate dérivé, $R^3$ est un H, un alkyle en $C_1$-$C_4$ ou un résidu d'un acide aminé naturel ou non naturel.

**40.** Formulation selon l'une quelconque des revendications 1 à 33 qui est un sel de la L-arginine.

**41.** Formulation selon l'une quelconque des revendications 1 à 33 qui est un sel de la L-lysine.

**42.** Formulation selon l'une quelconque des revendications 1 à 33 qui est un sel d'un métal divalent.

**43.** Formulation selon l'une quelconque des revendications 1 à 33 qui est un sel de sodium.

**44.** Formulation selon l'une quelconque des revendications 1 à 33 qui est un sel de lithium.

**45.** Formulation selon l'une quelconque des revendications 1 à 33 dans laquelle le sel est un sel de la glucamine, p.ex. un sel de la N-méthyl-D-glucamine.

**46.** Formulation selon l'une quelconque des revendications 1 à 33 qui est un sel de calcium ou de magnésium.

**47.** Formulation selon l'une quelconque des revendications 1 à 46 comprenant des ions boronate dérivés de l'acide boronique peptidique et ayant une stoechiométrie compatible avec le fait que les ions boronate portent une unique charge négative.

**48.** Formulation selon l'une quelconque des revendications 1 à 47 dans laquelle le sel comprend un ion boronate dérivé de l'acide boronique peptidique et un contre-ion et dans laquelle le sel consiste essentiellement en un sel ayant un seul type de contre-ion.

**49.** Formulation selon l'une quelconque des revendications 1 à 33 qui est un sel monolithium.

**50.** Formulation selon l'une quelconque des revendications 1 à 32 qui est un sel hémicalcique.

**51.** Formulation selon l'une quelconque des revendications 1 à 32 qui est un sel monosodique.

**52.** Formulation selon la revendication 1 qui est le sel monosodique de Cbz-(R)-Phe-(S)-Pro-(R)-Mpg-$B(OH)_2$.

**53.** Formulation selon la revendication 1 qui est le sel hémicalcique de Cbz-(R)-Phe-(S)-Pro-(R)-Mpg-$B(OH)_2$.

**54.** Formulation selon l'une quelconque des revendications 1 à 53 comprenant en outre un diluant, excipient ou véhicule pharmaceutiquement acceptable.

**55.** Formulation selon l'une quelconque des revendications 1 à 54 qui comprend le boronate sous forme d'anhydride.

**56.** Formulation selon l'une quelconque des revendications 1 à 55 adaptée à l'administration intraveineuse.

**57.** Formulation selon la revendication 56 comprenant un agent de tonicité.

**58.** Formulation selon la revendication 56 ou la revendication 57 sous une forme nécessitant une reconstitution avant l'administration.

**59.** Formulation selon la revendication 56 ou la revendication 55 sous la forme d'une solution.

**60.** Formulation selon l'une quelconque des revendications 1 à 35, adaptée à une administration intraveineuse et comprenant une solution contenant pour le sel la première et la deuxième espèces suivantes et optionnellement d'autres ingrédients, par exemple un agent de tonicité ou un tampon :

a) une première espèce sélectionnée parmi (a) des acids boroniques ayant la formule (I) ou, selon le cas, la formule (II), (b) des anions boronate de celle-ci, et (c) n'importe quelle forme à l'équilibre des composés précités (p.ex. anhydride); et
(b) une deuxième espèce sélectionnée parmi le groupe consistant en des ions de métaux alcalins et des composés organiques basiques contenant de l'azote pharmaceutiquement acceptables ayant un pKb d'environ 7 ou plus (lesquels composés seront censés comprendre les composés sous forme protonée),

ladite formulation ayant une stoechiométrie observée compatible avec la première espèce, à savoir des ions boronate portant une unique charge négative.

**61.** Formulation selon la revendication 60 ayant un Ki pour la thrombine d'environ 20 nM ou moins.

**62.** Formulation selon l'une quelconque des revendications 1 à 61 contenant des traces d'un solvant aliphatique ou cycloaliphatique.

**63.** Formulation selon la revendication 62 dans laquelle le solvant est un n-alcane, optionnellement le n-heptane.

**64.** Formulation selon l'une quelconque des revendications 1 à 63 dans laquelle $R^9$ (dans le cas d'une formulation contenant un sel d'acide boronique de formule (I)) ou $R^1$ (dans le cas d'une formulation contenant un sel d'acide boronique de formule (II)) est un groupe alkoxyalkyle qui peut être représenté par $R^Z$-O-$R^Y$-, ladite formulation étant dépourvue d'une impureté, un type de boronate dont la structure correspond à celle d'un type selon la formule (I) ou de la formule (II) dans laquelle $R^9$ ou, selon le cas, $R^1$ est replacé par un groupe alkyle $R^Y$.

**65.** Formulation selon l'une quelconque des revendications 1 à 64 essentiellement dépourvue d'un quelconque produit de dégradation résultant d'un clivage de la liaison C-B.

**66.** Formulation selon la revendication 14 ou l'une quelconque des revendications 15 à 65 combinée à la revendication 14 dans laquelle des ions boronate dans lesquels $aa^1$ a une configuration (R)-, $aa^2$ a une configuration (S)- et le résidu -NH-CH($R^1$)-$B(OH)_{2a}$ a la configuration (R)- sont dans un excédent diastéréoisomère de 99% ou plus, p.ex. 99,5 % ou plus par rapport à l'isomère (R,S,S).

**67.** Formulation selon la revendication 33 ou n'importe laquelle des revendications 34 à 63 ou 66 combinée à la revendication 33 qui est essentiellement dépourvue du composé :

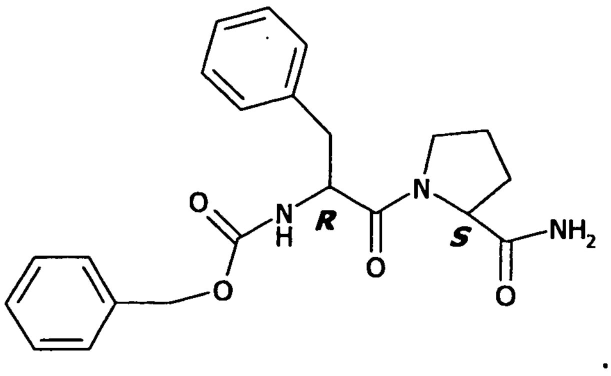

**68.** Formulation selon la revendication 33 ou selon l'une quelconque des revendications 34 à 63 ou 66 combinée à la revendication 33 ou à la revendication 67 qui est essentiellement dépourvue du composé:

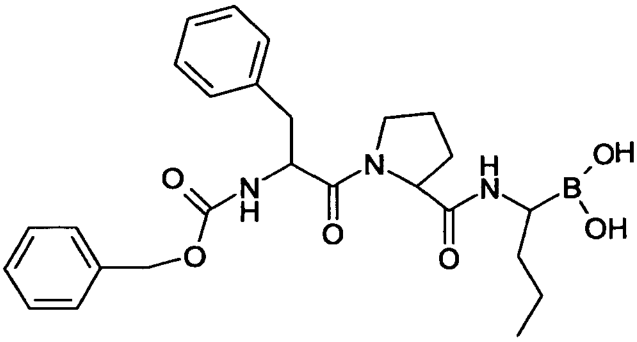

et de ses formes à l'équilibre.

**69.** Produit destiné à être utilisé comme produit pharmaceutique parentéral, comprenant un sel tel que défini dans n'importe laquelle des revendications 1 à 51.

**70.** Produit selon la revendication 68 dans laquelle le sel comprend les groupes boroniques de l'acide sous forme d'anhydride.

**71.** Utiliisation d'un sel tel que défini dans l'une quelconque des revendications 1 à 51, ou d'un sel tel que défini dans l'une quelconque des revendications précitées et ayant les caractéristiques exposées dans une ou une combinaison autorisée des revendications 61 à 67, pour la fabrication d'un médicament parentéral pour le traitement prophylactique ou thérapeutique des thromboses, ce médicament étant optionnellement utilisé dans un traitement anticoagulant lorsque le sang est en contact avec un dispositif médical extra-corporel, comme par exemple durant une intervention cardio-vasculaire utilisant une machine coeur-poumon artificiel ou pour une hémodialyse.

**72.** Utiliisation d'un sel tel que défini dans l'une quelconque des revendications 1 à 53, ou d'un sel tel que défini dans l'une quelconque des revendications précitées et ayant les caractéristiques exposées dans une ou une combinaison autorisée des revendications 62 à 68, pour la fabrication d'un médicament parentéral pour prévenir les thromboses dans un circuit d'hémodialyse d'un patient, pour empêcher un évènement cardio-vasculaire chez un patient ayant une insuffisance rénale au stade terminal, pour empêcher un évènement thromboembolique veineux chez un patient recevant une chimiothérapie par l'intermédiaire d'un cathéter à demeure, pour empêcher des évènements thromboemboliques chez un patient subissant une procédure de reconstruction d'une artère d'un membre inférieur, ou pour traiter à titre thérapeutique ou prophylactique une maladie artérielle sélectionnée parmi les syndromes coronariens aigus, une thrombose cérébro-vasculaire, une occlusion artérielle périphérique et une thrombose artérielle résultant d'une fibrillation auriculaire, d'une valwlopathie cardiaque, de pontages artério-veineux, de cathéters à demeure ou de stents coronariens.

**73.** Utilisation de la revendication 71 ou de la revendication 72, le médicament étant un médicament intraveineux, pour

une utilisation directe ou après reconstitution.

**74.** Formulation pharmaceutique parentérale comprenant une combinaison (i) d'un sel tel que défini dans l'une quelconque des revendications 1 à 53, ou un sel tel que défini dans l'une quelconque desdites revendications et ayant les caractéristiques exposées dans une ou une combinaison autorisée des revendications 62 à 68, et (ii) un principe pharmaceutiquement actif supplémentaire, p.ex. un autre agent de traitement cardio-vasculaire, par exemple un médicament hypolipémiant, un fibrate, de la niacine, une statine, un inhibiteur de la CETP, un séquestrant d'acide biliaire, un antioxydant, un antagoniste IIb/IIIa, un inhibiteur de l'aldostérone, un antagoniste a2, un agoniste a3, un béta-bloquant, l'acide acétylsalicylique, un diurétique de l'anse, un inhibiteur de l'enzyme de conversion, un agent antithrombotique avec un mécanisme d'action différent, un agent antiplaquettaire, un récepteur du thromboxane et/ou un inhibiteur de la synthétase, un antagoniste des récepteurs du fibrinogène, un agent mimétique de la prostacycline, un inhibiteur de la phosphodiestérase, un antagoniste des récepteurs ADP ($P_2$ T), un thrombolytique, un agent cardioprotecteur ou un inhibiteur COX-2.

**75.** Utilisation d'un sel selon l'une quelconque des revendications 1 à 53, ou d'un sel tel que défini dans l'une quelconque desdites revendications et ayant les caractéristiques exposées dans une ou une combinaison autorisée des revendications 63 à 68, pour la fabrication d'un médicament parentéral pour traiter, par exemple pour prévenir, un trouble cardio-vasculaire en co-administration avec un autre agent de traitement cardio-vasculaire, par exemple un de ceux énumérés dans la revendication 74.

**76.** Médicament parentéral comprenant un sel d'un acide boronique qui est un inhibiteur sélectif de la thrombine et a un résidu d'acide aminoboronique neutre capable de se lier au sous-site S1 de la thrombine lié par l'intermédiaire d'une liaison peptidique à une fraction hydrophobe capable de se lier aux sous-sites S2 et S3 de la thrombine, le sel comprenant une espèce boronate dérivée de l'acide boronique et un cation ayant une valence n et ayant une stoechiométrie observée compatible avec une stoechiométrie fictive (acide boronique:cation) de n:1.

**77.** Médicament selon la revendication 76 dans lequel l'acide boronique a un Ki pour la thrombine d'environ 100 nM ou moins, et optionnellement d'environ 20 nM ou moins.

**78.** Produit pour administration intraveineuse après reconstitution et comprenant, sous la forme d'un solide finement divisé, un sel constitué essentiellement d'un sel monosodique ou monolithium d'un acide ayant la formule Cbz-(R)-Phe-(S)-Pro-(R)-Mpg-$B(OH)_2$, ce sel étant optionellement mélangé à un ou de plusieurs antioxidants, agents conservateurs ou autres additifs.

**79.** Produit pour administration intraveineuse comprenant, sous la forme d'une solution aqueuse isotonique, un sel constitué essentiellement d'un sel monosodique ou monolithium d'un acide ayant la formule Cbz-(R)-Phe-(S)-Pro-(R)-Mpg-$B(OH)_2$, ce produit comprenant en outre optionellement un ou de plusieurs antioxidants, agents conservateurs ou autres additifs.

**80.** Produit selon la revendication 78 ou la revendication 79 ayant en outre les caractéristiques exposées dans une ou une combinaison autorisée des revendications 61 à 67.

**81.** Le produit de l'une quelconque des revendications 78 à 80 dans lequel le sel comprend des espèces anhydride.

**82.** Méthode pour produire une formulation pharmaceutique parentérale, comprenant :

la mise en contact d'un acide boronique tel que défini dans l'une quelconque des revendications 1 à 33 avec une base pharmaceutiquement acceptable ayant un pKb de 7 ou plus; et
la formulation du produit résultant en la formulation.

**83.** Méthode selon la revendication 82 comprenant en outre l'obtention de l'acide organoboronique :

en formant une solution d'un ester de diol de l'acide boronique tel que défini dans l'une quelconque des revendications 1 à 33;
en combinant la solution avec de la diéthanolamine et en laissant ou en faisant en sorte que la diéthanolamine réagisse avec l'ester pour former un précipité insoluble;
en récupérant le précipité;
en convertissant le précipité en l'acide boronique libre.

**84.** Méthode selon la revendication 83 dans laquelle l'éther est un éther diéthylique et l'ester est un ester du pinacol.

**85.** Méthode selon l'une quelconque des revendications 82 à 84 dans laquelle la base est un composé organique contenant de l'azote ou est une base d'un métal.

**86.** Méthode selon la revendication 85 dans laquelle la base organique a un pKb de 7,5 ou plus et n'est pas la choline ou l'ammoniac.

**87.** Méthode selon l'une quelconque des revendications 82 à 84 dans laquelle la base et une base d'un métal alcalin ou d'un métal terre alcaline.

**88.** Méthode selon la revendication 87 dans laquelle la base est une base du sodium.

**89.** Méthode selon l'une quelconque des revendications 82 à 88 dans laquelle l'acide boronique est tel que défini dans l'une quelconque des revendications 27 à 32.

**90.** Méthode selon l'une quelconque des revendications 82 à 88 dans laquelle l'acide boronique est Cbz-(R)-Phe-(S)-Pro-(R)-Mpg-$B(OH)_2$.

**91.** Méthode selon l'une quelconque des revendications 82 à 90 dans laquelle ledit produit comprend l'espèce boronate anhydride.

**92.** Méthode selon n'importe laquelle des revendications 82 à 91 dans laquelle la formulation comprend ledit produit sous forme solide pour reconstitution en vue d'une administration intraveineuse.

**93.** Méthode selon n'importe laquelle des revendications 82 à 91 dans laquelle la formulation est une formulation intraveineuse liquide dans un véhicule à base aqueuse.

**94.** Utilisation d'un ester de diol d'un acide boronique tel que défini dans l'une quelconque des revendications 1 à 33 pour la réalisation d'une formulation pharmaceutique parentérale en faisant réagir l'acide boronique, produit par hydrolyse de l'ester de diol ou d'un ester de la diéthanolamine obtenu par transestérification dudit ester de diol, avec une base pharmaceutiquement acceptable telle que définie dans n'importe laquelle des revendications 83 à 88 et en formulant le produit résultant en une formulation pharmaceutique.

**95.** Utilisation de la revendication 94, l'ester de diol étant un pinacol, un pinanediol ou un ester de la diéthanolamine.

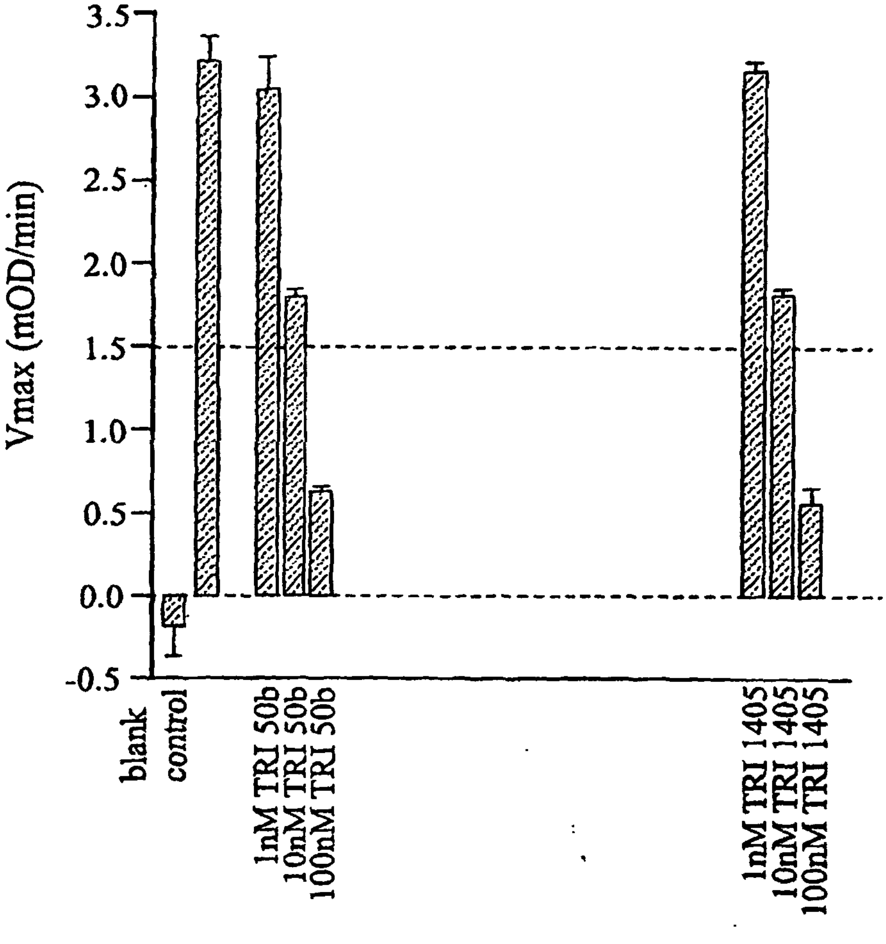
Vmax (mOD/min)
3.5
3.0
2.5
2.0
1.5
1.0
0.5
0.0
-0.5
blank
control
1nM TRI 50b
10nM TRI 50b
100nM TRI 50b
1nM TRI 1405
10nM TRI 1405
100nM TRI 1405

Fig 1

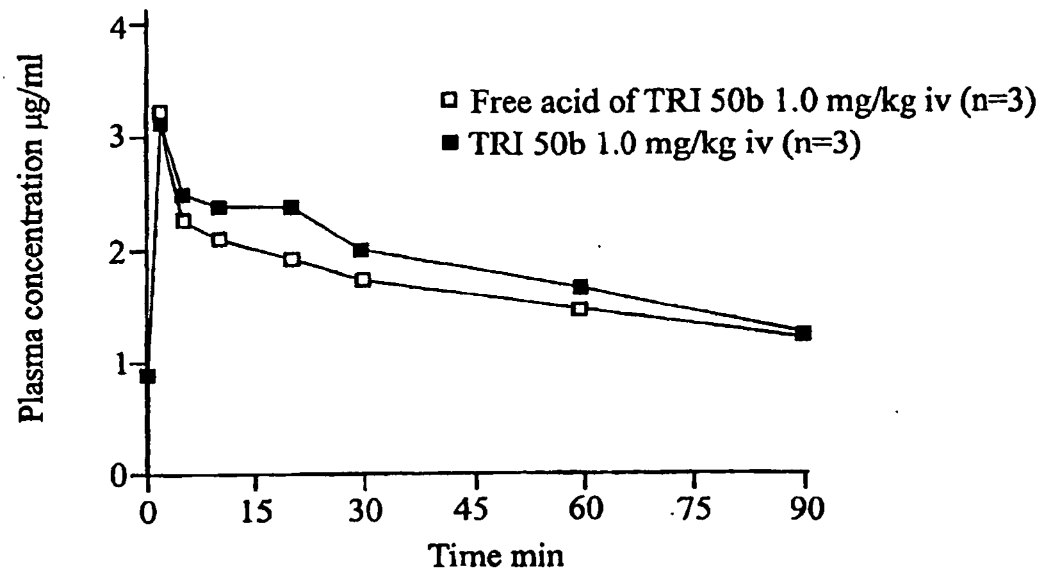
Plasma concentration µg/ml
4
3
2
1
0
Free acid of TRI 50b 1.0 mg/kg iv (n=3)
TRI 50b 1.0 mg/kg iv (n=3)
0
15
30
45
60
75
90
Time min

Fig 2